# EUROPEAN PATENT APPLICATION

(11) **EP 4 722 372 A2**
(43) Date of publication of application: **08.04.2026**
(21) Application number: 24759393.2
(22) Date of filing: 28.05.2024
(51) Int. Cl.: C12N 15/82, C12N 15/29, C12N 15/63, C12N 5/14, A01H 6/00, A01H 1/06

(54) **PLANTS WITH AGRONOMIC CHARACTERISTICS IMPROVED BY SUPPRESSION OF AIP10/ABAP1 REGULATORY NETWORK GENES**

(30) Priority: 30.05.2023 WO PCT/BR2023/050166; 31.05.2023 BR 102023010729; 29.08.2023 BR 102023017477
(71) Applicant: Hapiseeds Pesquisa e Desenvolvimento Ltda., 21941-614 Rio de Janeiro - RJ (BR); Universidade Federal do Rio de Janeiro, 21941-850 Rio de Janeiro - RJ (BR)
(72) Inventor: SILVA HEMERLY, Adriana, 22430-090 Rio de Janeiro - RJ (BR); DA FONSECA MONTESSORO, Patrícia, 21020-320 Rio de Janeiro - RJ (BR); FORERO BALLESTEROS, Helkin Giovani, 20550-090 Rio de Janeiro - RJ (BR); DE OLIVEIRA URQUIAGA, Maria Clara, 22451-030 Rio de Janeiro - RJ (BR); LOURES DA SILVA, Mirielson, 21920-150 Rio de Janeiro - RJ (BR); KÖHN CARNEIRO, Aline, 21930-125 Rio de Janeiro - RJ (BR); CARDOZO ROSMAN, Aline, 22470-040 Rio de Janeiro - RJ (BR); SILVA COELHO, Fernanda, 22.220-020 Rio de Janeiro - RJ (BR); SILVA DE OLIVEIRA, João Victor, 22640-908 Rio de Janeiro - RJ (BR); BARBOSA DE ALMEIDA, Janice, 06000 Nice (FR)
(74) Representative: Vossius & Partner Patentanwälte Rechtsanwälte mbB
(86) International application number: PCT/BR2024/050215
(87) International publication number: WO 2024/174016

(57) **Abstract**

The present invention refers to a genetic modification for the enhancement of plant traits useful in agriculture. Particular embodiments provide uses of plants containing said genetic modification, as well as methods of producing plants with said genetic modification and plant products obtainable therefrom.

## Description

### FIELD OF THE INVENTION

The invention is related, in general, to the field of biotechnology, especially genetic modifications useful for the improvement of plant characteristics useful in agriculture. The present patent application teaches plants with genetic modifications that result in improved plant traits of agronomic interest, including increased biomass, water stress tolerance, photosynthetic efficiency, carbon fixation, nutritional value, resistance to pest nematodes, and responsiveness to beneficial bacteria.

### BACKGROUND OF THE INVENTION

There is a growing need to enhance the productivity of agriculturally important crop plants. In addition to the rising demand for agricultural products for food, clothing, and energy to support a growing human population, climate-related impacts and increasing population-driven pressure to use land for purposes other than agricultural activities are likely to decrease the amount of land available for agriculture. These effects drive increased demand for higher productivity and efficiency in agricultural crop production.

Soil is an important framework of nutrients necessary for the maintenance of plant life. Brazilian soils are known to be poor in phosphorus, potassium, and nitrogen, nutrients of great importance for the development of plants (Almeida Júnior et al., 2020, BRAZILIAN JOURNAL OF AGRICULTURE - Revista de Agricultura 95: 251-260). To compensate for the scarcity of these nutrients, chemical fertilizers are routinely applied to the soil, causing damage to the environment and the microbiological community, thus intensifying the loss of arable land (Good and Beatty, 2011, PLoS Biol 9: e1001124; Ozede et al., 2018, Rhizosphere Microbiome Modulators: Contributions of Nitrogen Fixing Bacteria towards Sustainable Agriculture. Doi: 10.3390/ijerph15040574). According to a survey by CEPEA (Center for Advanced Studies in Applied Economics - Esalq/USP, 2022), for grain production in general, the average expenditure on the use of fertilizers for the 2022/2023 harvest doubled compared to the previous year. For the case of soybeans, for example, in March 2022, the increase was 103.4% compared to March 2021 (CEPEA, 2022, Agricultural Cost Report - Grain Costs.).

A more sustainable alternative for replacing chemical fertilizers is the use of bioinoculants (Kavadia et al., 2020, Atmosphere (Basel) 11: 1-18).

Bioinoculants can be defined as inputs composed of growth-promoting microorganisms that facilitate plant growth by making essential nutrients available to crops and/or providing tolerance against different abiotic and biotic factors (Maitra et al., 2021. Microorganisms Bioinoculants-Natural Biological Resources for Sustainable Plant Production. Doi: 10.3390/microorganisms10010051). Bioinoculants may be associated in the rhizosphere, on the surface of plant roots, or even be occupying intercellular spaces and vascular tissues (Carvalho et al., 2016). Among the most efficient associations described is the one established with nitrogen-fixing endophytic bacteria - diazotrophs (Baldani and Baldani, 2005, Annals of the Brazilian Academy of Sciences 77: 549-79; Santi et al., 2013, Annals of Botany 111: 743-767) - which can promote benefits such as: increased grain production, increased biomass production, and greater tolerance to abiotic stresses (Vargas et al., 2014, PLoS ONE 9: 1-37; Karimi et al., 2018, Environmental Sustainability 1: 3; Breda et al., 2020, Archives of Agronomy and Soil Science 66: 1948-1962) and biotics (Arencibia et al., 2006, Plant Signaling and Behavior 1: 265-273; Santa Brigida et al., 2016, PLoS ONE 11: 1-30; Mengistu, 2020, Endophytes: Colonization, Behavior, and Their Role in Defense Mechanism. International Journal of Microbiology. Doi: 10.1155/2020/6927219).

Brazil is one of the countries that leads the use of plant growth-promoting bacteria, with 90% of its entire soybean planted area fertilized with bioinoculants, thus avoiding the import of nitrogen-based fertilizers (EMBRAPA, 2019). Despite this, some plant species do not show so much efficiency in the use of the bioinoculant, since some studies indicate that the plant-bacteria interaction depends on several factors such as the plant genotype, the species of bacteria used and environmental factors (Carvalho et al., 2016, Plant Molecular Biology 90: 561-574; Clemente et al., 2017, Tropical Agricultural Research 47: 110-117; da Fonseca Breda et al., 2019, Archives of Microbiology 201: 547-558; dos Santos et al., 2020, Frontiers in Sustainable Food Systems 4: 1-15).

In this context, biotechnology can be a great ally, because by means of modern plant breeding techniques it is possible to modulate mechanisms that make plant species of economic interest more efficient in beneficial bacterial associations, leading to significant increases in agricultural productivity, reduction in water consumption and greater tolerance to abiotic and biotic stresses (Mohammad, Anwar Hossain Shabir, Hussain Wani Soumen Bhattacharjee, 2016, Drought Stress Tolerance in Plants, Vol 2. Drought Stress Tolerance in Plants, Vol 2. Doi: 10.1007/978-3-319-32423-4).

The soil in which plants sustain themselves, in addition to storing the water and nutrient necessary for their full development, can also harbor a multitude of living organisms that will inevitably interact with the plants in their surroundings, whether they are beneficial or not (Yang et al., 2020, Cropping systems in agriculture and their impact on soil health-A review. Global Ecology and Conservation, 23, p. e01118, 2020/09/01/2020.).

In view of the growing demand, the supply of plants with improved plant characteristics of agronomic interest, such as photosynthetic efficiency, carbon fixation, nutritional value, and responsiveness to beneficial bacteria, is vital to increase production per unit of land available for agriculture.

The provision of plant genetic modifications that result in plants with higher biomass, tolerance to water stress, photosynthetic efficiency, higher carbon fixation, higher nutritional value, and responsiveness to beneficial bacteria is desired for several reasons:

Food safety: As the global population continues to grow, there is a growing demand for food and the need to produce more food using less land, water, and other resources. Genetic modifications that result in plants with higher biomass and higher nutritional value can help increase food production and improve food safety.

Climate change: Climate change is having a significant impact on agriculture, and there is a need to develop crops that are more resilient to changes in temperature, water availability, and other environmental factors. Genetic modifications that result in plants with greater tolerance to water stress, greater photosynthetic efficiency, and greater carbon fixation can help mitigate the effects of climate change on agriculture.

Sustainable agriculture: There is a growing interest in sustainable farming practices that minimize the use of pesticides and fertilizers, reduce soil erosion, and improve soil health. Genetic modifications that result in plants with greater tolerance to pests and diseases and that are more responsive to beneficial bacteria can help reduce the need for pesticides and fertilizers, while plants with higher biomass and improved root systems can help reduce soil erosion and improve soil health.

Human health: There is a growing recognition that the quality of the food we eat can have a significant impact on human health. Genetic modifications that result in plants with higher nutritional value, such as higher levels of vitamins, minerals, and antioxidants, can help improve human health and reduce the incidence of diet-related diseases.

Animal feed: Plants with better nutritional value are desirable for animal feed production because they can provide a more balanced and nutritious diet for livestock, which can improve animal health, growth, and productivity. Cattle require a variety of nutrients to grow and maintain their health, including proteins, carbohydrates, fats, vitamins, and minerals. Genetically modified plants to contain higher levels of these nutrients can provide a more complete and balanced diet for livestock, reducing the need for synthetic supplements or additives.

Consequently, there is a constant demand for the identification of new genetic modifications that provide plants with improved plant characteristics of agronomic interest.

To satisfy this demand, the present invention describes new plants with genetic modifications that result in improved plant traits of agronomic interest, including increased biomass, increased tolerance to water stress, increased photosynthetic efficiency, increased carbon fixation, increased nutritional value, increased resistance to pest nematodes, and increased responsiveness to beneficial bacteria.

Patent US10647988B2 teaches that suppression and/or reduction of gene expression *AIP10* results in increased biomass, yield, and/or tolerance to water stress. Examples are *Arabidopsis thaliana* plants with *AIP10* gene expression suppressed (knockout) by T-DNA insertion, as well as *A. thaliana* plants with *AIP10* gene expression reduced (knockdown) by transformation with an RNAi construct.

Patent US11525143B2 exemplifies maize, soybean and cotton plants with reduced expression of the *AIP10* gene and consequent increase in biomass, yield and/or tolerance to water stress. Examples are maize plants edited with CRISPR and soybean and cotton plants transformed with RNAi. All of the plants exemplified have residual expression of the *AIP10* gene (detectable levels of mRNA). Patent US10647988B2 describes *AIP10* as a gene involved in cell cycle regulation. As is widely known in the technique, plants with the expression of an endogenous gene totally suppressed usually have unwanted deleterious effects. In particular, it is expected that the total suppression of genes involved in cell cycle regulation may have negative effects on the homeostasis of cell division rates. In fact, as taught in Masuda et al., 2008 (ABAP1 is a novel plant Armadillo BTB protein involved in DNA replication and transcription, The EMBO Journal (2008) 27, 2746-2756) the complete suppression (*knockout*) of ABAP1, which interacts with AIP10, is embryo-lethal. Thus, based on the teachings of patents US10647988B2 and US11525143B2, it is expected that the partial suppression of the expression of the gene *AIP10* will be the preferable alternative to obtain plants with improved agronomic characteristics.

The present invention teaches that, surprisingly, a genetic modification resulting in total or partial silencing of native genes of plants belonging to the AIP10/ABAP1 regulatory network results in plants with improved agronomic characteristics compared to plants without said genetic modification.

### SUMMARY OF THE INVENTION

The present invention teaches plants containing genetic modifications that result in improved agronomic traits, as well as plant genomes, methods of production of said plants, nucleic acid construction, uses of said plants, cultivation methods, and plant products produced therefrom.

The genes targeted by the genetic modifications according to the present invention are genes of the regulatory network of the gene *AIP10*/*ABAP1.* ABAP1 interacts with components of the pre-replicative complex of DNA and other proteins (Masuda et al., 2008, EMBO Journal 27: 2746-2756). Another cell cycle regulator identified by the inventors and also the object of the invention is AIP10, which interacts with ABAP1.

The present invention provides plants with a genetic modification that results in complete (*ko*) or partial (*kd*) suppression of native plant genes belonging to the AIP10/ABAP1 regulatory network.

In a first aspect, the present invention teaches that, surprisingly, partial or total suppression of the *AIP10* gene, as well as partial suppression of the *ABAP1* gene produce previously unknown enhanced agronomic traits, such as increased photosynthetic efficiency, increased carbon fixation, increased nutritional value, increased resistance to pest nematodes, and increased responsiveness to beneficial bacteria.

In a second aspect, the present invention teaches that, surprisingly, complete suppression of the *AIP10* gene (*aip10ko*) results in improved agronomic traits relative to an identical plant that has partial expression of the *AIP10* native gene, such as, for example, a plant containing reduced native gene expression (*aip10kd*) by RNAi or heterozygous for a native allele.

Improved agronomic traits resulting from a genetic modification in accordance with the present invention include increased biomass, increased tolerance to water stress, increased photosynthetic efficiency, increased carbon fixation, increased nutritional value, increased resistance to pest nematodes, and increased responsiveness to beneficial bacteria.

In certain embodiments described herein, the *AIP10* gene is a gene encoding a protein comprising a sequence with at least about 90%, 91%, 92%, 93%, 94%, 95%, 96%, 97%, 98%, 99%, 100% sequence identity with an amino acid sequence selected from the group consisting of SEQ ID NO: 2, 3, 4, 5, 9, 12, 13, 15, 17, 21, 22, 23, 25, 26, 28, 30, 32, 34, 36, 39, 40, 45, 46, 47, 48, 51, 52, 57, 58, 59, 60, 63, 64, 66, 69, 70, 72, 75, 76, 78, 81, 82, 84, 85, 86, 89, 90, 92, 94, 96, 98, 99, 101, 104, 105, 107 and 263. In certain embodiments described herein, the *AIP10* gene is a gene that has at least 80%, 81%, 82%, 83%, 84%, 85%, 86%, 87%, 88%, 89%, 90%, 91%, 92%, 93%, 94%, 95%, 96%, 97%, 98%, 99%, 100% sequence identity with a nucleotide sequence selected from the group consisting of SEQ ID NO: 1, 8, 10, 11, 14, 16, 18, 19, 20, 24, 27, 29, 31, 33, 35, 37, 38, 41, 42, 43, 44, 49, 50, 53, 54, 55, 56, 61, 62, 65, 67, 68, 71, 73, 74, 77, 79, 80, 83, 87, 88, 91, 93, 95, 97, 100, 102, 103 and 106.

In certain embodiments described herein, the *ABAP1* gene is a gene encoding a protein comprising a sequence with at least about 90%, 91%, 92%, 93%, 94%, 95%, 96%, 97%, 98%, 99%, 100% sequence identity with an amino acid sequence selected from the group consisting of SEQ ID NO: 7, 109, 111, 114, 115, 117, 121, 122, 123, 125, 127, 129, 132, 133, 136, 137, 140, 141, 142, 146, 147, 148, 155, 156, 157, 158, 159, 160, 161, 164, 165, 171, 172, 173, 174, 175, 180, 181, 182, 183, 188, 189, 190, and 191, 254, 198, 199, 200, 201, 202, 206, 207, 208, 210, 213, 214, 217, 218, 219, 220, 223, 224, 227, 228, 231, 232, 235, 236, 239, 240, 241, 242, 243, 246, 247, 248, 251 and 252. In certain embodiments described in this document, the *ABAP1* gene is a gene that has at least 80%, 81%, 82%, 83%, 84%, 85%, 86%, 87%, 88%, 89%, 90%, 91%, 92%, 93%, 94%, 95%, 96%, 97%, 98%, 99%, 100% sequence identity with a nucleotide sequence selected from the group consisting of SEQ ID NO: 6, 108, 110, 112, 113, 116, 118, 119, 120, 124, 126, 128, 130, 131, 134, 135, 138, 139, 143, 144, 145, 149, 150, 151, 253, 152, 153, 154, 162, 163, 166, 167, 168, 169, 170, 176, 177, 178, 179, 184, 185, 186, 187, 192, 193, 194, 195, 196, 197, 203, 204, 205, 209, 211, 212, 215, 216, 221, 222, 225, 226, 229, 230, 233, 234, 237, 238, 244, 245, 249 and 250.

The genetically modified plant species covered by this invention include any organisms of the Kingdom Plantae, preferably embryophytes, more preferably angiosperms. In preferred embodiments, the genetically modified plant is a crop plant.

Nucleic acid constructs (expression cassettes) are also provided for silencing the genes of the AIP10/ABAP1 regulatory network with the aim of introducing a genetic modification according to the present invention in a plant or plant genome.

The present invention further provides methods for producing genetically modified plants with a genetic modification that results in improved agronomic traits.

The methods for producing genetically modified plants include crossing a plant with a genetic modification according to the present invention with a second plant without said genetic modification to produce a progeny of plants with a genetic modification according to the present invention.

The methods of production of genetically modified plants further contemplate the introduction of a genetic modification in the genome of a plant that has native versions of the genes of the AIP10/ABAP1 regulatory network.

Also the objects of this invention are the uses of plants in accordance with this invention, as well as methods of cultivating them. Uses include purposes such as crossbreeding with a second plant, regeneration of a genetically modified plant, planting or growing a field of transgenic plants, production of plant products, or production of a plant product.

This invention also provides cells, plant parts, seeds and plant products produced from genetically modified plants in accordance with this invention. The plant products encompassed include plant biomass, oil, bran, animal feed, flour, flakes, husks, and processed seeds. A plant genome comprising a genetic modification that results in a complete suppression of the native *AIP10* gene is also contemplated.

A method of crop cultivation with an improved agronomic characteristic is also contemplated, comprising cultivating one or more plants according to the present invention.

Plant products produced from plants containing a genetic modification in accordance with this invention are covered. Plant products include seeds, grains, cereals, flakes, bran, flour, syrup, oil, juices, processed parts for food or animal feed, biomass, and fuels and optionally comprise a detectable amount of a DNA molecule containing a genetic modification in accordance with this invention.

A method of producing plant products from a plant containing a genetic modification according to this invention is also contemplated. Methods of production of plant products comprise the preparation of plant products from plants, seeds or parts of plants genetically modified according to the present invention for the production of products such as seeds, grains, cereals, flakes, bran, flour, syrup, oil, juices, processed parts for food or animal feed, biomass and fuels.

Other embodiments, features, and advantages of the invention will become apparent from the following detailed description, examples, and claims.

### BRIEF DESCRIPTION OF THE SEQUENCES

The nucleotide sequences of the native *AIP10* and *ABAP1* genes, as well as their target expression products for suppression according to the present invention, can be identified from a myriad of organisms. Although the nucleotide sequences mentioned are described herein, they should not be considered as limitations of the present invention. Thus, a sequence of *AIP10* or *ABAP1* can be identified and functionally annotated by sequence comparison. A person skilled in the art can readily identify a sequence of *AIP10* or *ABAP1* in a suitable database, such as GenBank, using publicly available sequence analysis programs and parameters. See the tables below:

**Table 1: Nucleotide or polypeptide sequences of AIP10 from different species that can be used in the present invention.**

| **AIP10** | | | | |
|---|---|---|---|---|
| | **Species** | | | |
| | **Scientific name** | **Common name** | **Gene quantity and ID** | **Amount and ID of proteins** |
| 1. | ***A. thaliana*** | **Arabidopsis** | 1 (AT1G80940) (SEQ ID NO: 1) | 4 isoforms (AT1G80940.1 (SEQ ID NO: 2), AT1G80940.2 (SEQ ID NO: 3), AT1G80940.3 (SEQ ID NO: 4), AT1G80940.4 (SEQ ID NO: 5)) |
| 2 | *Sorghum bicolor* | Sorghum | 1 (Sobic.004G328000) (SEQ ID NO: 8) | 1 (Sobic.004G328000) (SEQ ID NO: 9) |
| 3 | *Saccharum spontaneum* | Sugar cane | 2 genes [?] (Sspon.04G0001710- 2C (SEQ ID NO: 10) and Sspon.04G0001710- 3D) (SEQ ID NO: 11) | 2 proteins (Sspon.04G0001710-2C (SEQ ID NO: 12) and Sspon.04G0001710-3D) (SEQ ID NO: 13) |
| 4 | *Zea mays* | Maize | 1 gene (Zm00001d018364_P0 01) (SEQ ID NO: 14) | 1 protein (Zm00001d018364_P001) (SEQ ID NO: 15) |
| 5 | *Oryza sativa* | Rice | 1 gene (lLOC_Os02g55080) (SEQ ID NO: 16) | 1 protein (LOC_Os02g55080) (SEQ ID NO: 17) |
| 6 | *Triticum aestivum* | Wheat | 3 genes g TraesCS6A03G09119 00 (SEQ ID NO: 18), TraesCS6B03G10962 00 (SEQ ID NO: 19) and TraesCS6D03G07825 00 (SEQ ID NO: 20)) | 3 proteins (TraesCS6A03G0911900 (SEQ ID NO: 21), TraesCS6B03G 1096200 (SEQ ID NO: 22) and TraesCS6D03G0782500 (SEQ ID NO: 23)) |
| 7 | *Hordeum vulgare* | Barley | 1 gene (HORVU0Hr1G040430) (SEQ ID NO: 24) | 2 isoforms (HORVUOHr1G040430.1 (SEQ ID NO: 25)) - Isoform 1 and (HORVU0Hr1G040430.4 (SEQ ID NO: 26)) - Isoform 4 |
| 8 | *Musa acuminata* | Banana | 1 gene (Ma03_g04600) (SEQ ID NO: 27) | 1 protein (Ma03_g04600) (SEQ ID NO: 28) |
| 9 | *Coffea canephora* | Coffee | 1 gene (Cc04_g05300) (SEQ ID NO: 29) | 1 protein (Cc04_g05300) (SEQ ID NO: 30) |
| 10 | *Vitis vinifera* | Grape | 1 gene (GSVIVG0101610300 1) (SEQ ID NO: 31) | 1 protein (GSVIVG01016103001) (SEQ ID NO: 32) |
| 11 | *Solanum tuberosum* | Potato | 1 gene (PGSC0003DMG4000 14155) (SEQ ID NO: 33) | 1 protein (PGSC0003DMG4000141 55) (SEQ ID NO: 34) |
| 12 | *Solanum lycopersicum* | Tomato | 1 gene (Solyc03g117670) (SEQ ID NO: 35) | 1 protein (Solyc03g117670.4) (SEQ ID NO: 36) |
| 13 | *Manihot esculenta* | Cassava | 2 genes (Manes.04G100900 (SEQ ID NO: 37) and Manes.11G069200 (SEQ ID NO: 38)) | 2 proteins (Manes.04G100900 (SEQ ID NO: 39) and Manes.11G069200 (SEQ ID NO: 40)) |
| 14 | *Brassica napus* | Canola | 4 genes (A07p26960 (SEQ ID NO: 41), A07p44750 (SEQ ID NO: 42), C06p29630 (SEQ ID NO: 43) and C06p53570 (SEQ ID NO: 44)) | 4 proteins (A07p26960 (SEQ ID NO: 45), A07p44750 (SEQ ID NO: 46), C06p29630 (SEQ ID NO: 47) and C06p53570 (SEQ ID NO: 48)) |
| 15 | *Brassica oleracea* | | 2 genes (SEQ ID NO: 49) and (SEQ ID NO: 50) | 2 proteins (BolC6t38128H (SEQ ID NO: 51) and BolC6t40373H (SEQ ID NO: 52)) |
| 16 | *Brassica carinata* | Abyssinian mustard | 4 genes (BcaB02g07237 SEQ ID NO: 53), BcaNung04166 (SEQ ID NO: 54), BcaC08g47533 (SEQ ID NO: 55) and BcaB02g08711 (SEQ ID NO: 56)) | 4 proteins (BcaB02g07237 (SEQ ID NO: 57), BcaNung04166 (SEQ ID NO: 58), BcaC08g47533 (SEQ ID NO: 59) and BcaB02g08711 (SEQ ID NO: 60)) |
| 17 | *Brassica rapa* | Mustard | 2 genes (BraA07t30340Z (SEQ ID NO: 61) and raA07T31983Z (SEQ ID NO: 62)) | 2 proteins (BraA07t30340Z (SEQ ID NO: 63) and raA07T31983Z (SEQ ID NO: 64)) |
| 18 | *Phaseolus vulgaris* | Common beans | 1 gene (Phvul.008G253200 (SEQ ID NO: 65)) | 1 protein (Phvul.008G253200 (SEQ ID NO: 66)) |
| 19 | *Glycine max* | Soybean | 2 genes (Glyma.07G021400 (SEQ ID NO: 67) and Glyma.08G220400 (SEQ ID NO: 68)) | 2 proteins (Glyma.07G021400 (SEQ ID NO: 69) and Glyma.08G220400 (SEQ ID NO: 70)) |
| 20 | *Pisum sativum* | Pea | 1 gene (Psat7g262200 (SEQ ID NO: 71)) | 1 protein (Psat7g262200 (SEQ ID NO: 72)) |
| 21 | *Eucalyptus grandis* | Eucalyptus | 2 genes(Eucgr.F03985 (SEQ ID NO: 75) and Eucgr.J03130 (SEQ ID NO: 76)) | 2 proteins (Eucgr.F03985 (SEQ ID NO: 75) and Eucgr.J03130 (SEQ ID NO: 76)) |
| 22 | *Theobroma cacao* | Cocoa | 1 gene (Thecc.06G046600 (SEQ ID NO: 77)) | 1 protein (Thecc.06G046600 (SEQ ID NO: 78)) |
| 23 | *Gossypium hirsutum* | Cotton | 2 genes (Gohir.A11G075200 (SEQ ID NO: 79) and Gohir.D11G079900 (SEQ ID NO: 80)) | 2 proteins (Gohir.A11G075200 (SEQ ID NO: 81) and Gohir.D11G079900 (SEQ ID NO: 82)) |
| 24 | *Gossypium raimondii* | Cotton | 1 gene (Gorai.007G085500) (SEQ ID NO: 83) | 3 isoforms (Gorai.007G085500.1 (SEQ ID NO: 84), Gorai.007G085500.2 (SEQ ID NO: 85) and Gorai.007G085500.3 (SEQ ID NO: 86)) |
| 25 | *Lactuca sativa* | Lettuce | 2 genes (Lsat_1_v5_gn_1_30801 (SEQ ID NO: 89) and Lsat_1_v5_gn_4_85061 (SEQ ID NO: 90)) | 2 proteins (Lsat_1_v5_gn_1_30801 (SEQ ID NO: 89) and Lsat_1_v5_gn_4_85061 (SEQ ID NO: 90)) |
| 26 | *Carica papaya* | Papaya | 1 gene (ASGPBv0.4 (SEQ ID NO: 92)) | 1 protein (ASGPBv0.4 (SEQ ID NO: 92)) |
| 27 | *Ricinus communis* | Castor bean | 1 protein (30131.m006891 (SEQ ID NO: 93)) | 1 protein (30131.m006891 (SEQ ID NO: 94)) |
| 28 | *Citrus clementine* | Orange | 1 gene (Ciclev10026461m.g (SEQ ID NO: 95)) | 1 protein (Ciclev10026461m.g (SEQ ID NO: 96)) |
| 29 | *Citrus sinensis* | Sweet orange | 1 gene (orange1.1g027963m.g (SEQ ID NO: 97)) | 2 isoforms (isoform orange1.1g028560m (SEQ ID NO: 98) and isoform orange1.1g027963m (SEQ ID NO: 99)) |
| 30 | *Miscanthus sinensis* | | 1 gene (Misin07G522700 (SEQ ID NO: 100)) | 1 protein (Misin07G522700 (SEQ ID NO: 101)) |
| 31 | *Secale cereale* | Rye | 2 genes (GWHGASIY033046 (SEQ ID NO: 102) and GWHGASIY033783 (SEQ ID NO: 103)) | 2 proteins (GWHGASIY033046 (SEQ ID NO: 104) and GWHGASIY033783 (SEQ ID NO: 105)) |
| 32 | Beta vulgaris | Beetroot | 1 gene (EL10Ac8g19398 (SEQ ID NO: 106)) | 1 protein (EL10Ac8g19398 (SEQ ID NO: 107)) |

**Table 2: Nucleotide or polypeptide sequences of ABAP1 of different species that can be used in the present invention.**

| **ABAP1** | | | | |
|---|---|---|---|---|
| | **Species** | | | |
| | **Scientific name** | **Common name** | **Gene quantity and ID** | **Amount and ID of proteins** |
| 1. | ***A. thaliana*** | **Arabidopsis** | 1 (AT5G13060) (SEQ ID NO: 6) | 1 protein (AT5G13060) (SEQ ID NO: 7) |
| 2 | *Sorghum bicolor* | Sorghum | 1 (Sobic.009G129600) (SEQ ID NO: 108) | 1 (Sobic.009G129600) (SEQ ID NO: 109) |
| 3 | *Saccharum spontaneum* | Sugar cane | 1 gene (Sspon.07G0008510-2C) (SEQ ID NO: 110) | 1 protein (Sspon.07G0008510-2C) (SEQ ID NO: 111) |
| 4 | *Zea mays* | Maize | 2 genes (Zm00001eb287530 (SEQ ID NO: 112) and Zm00001eb349910 (SEQ ID NO: 113)) | 2 proteins (Zm00001eb287530 (SEQ ID NO: 114) and Zm00001eb349910 (SEQ ID NO: 115)) |
| 5 | *Oryza sativa* | Rice | 1 gene (OsR498G0510791000. 01 (SEQ ID NO: 116)) | 1 protein (OsR498G0510791000. 01 (SEQ ID NO: 117)) |
| 6 | *Triticum aestivum* | Wheat | 3 genes (TraesCS1A03G064550 0 (SEQ ID NO: 118), and TraesCS1B03G0731200 (SEQ ID NO: 120) and TraesCS1003G060250 0 (SEQ ID NO: 122)) | 3 proteins (TraesCS1A03G064550 0 (SEQ ID NO: 121), and TraesCS1B03G0731200 (SEQ ID NO: 122) and TraesCS1003G060250 0 (SEQ ID NO: 123)) |
| 7 | *Hordeum vulgare* | Barley | 1 gene (Horvu_ MOREX_1H01 G392500 (SEQ ID NO: 124)) | 1 protein (Horvu_ MOREX_1H01 G392500 (SEQ ID NO: 125)) |
| 8 | *Musa acuminata* | Banana | 1 gene (Ma09_g16490 (SEQ ID NO: 126)) | 1 protein (Ma09_g16490 (SEQ ID NO: 127)) |
| 9 | *Coffea canephora* | Coffee | 1 gene (Cc01_g19440 (SEQ ID NO: 128)) | 1 protein (Cc01_g19440 (SEQ ID NO: 129)) |
| 10 | *Vitis vinifera* | Grape | 2 genes (GSVIVG01011129001 (SEQ ID NO: 130) and GSVIVG01017925001 (SEQ ID NO: 131)) | 2 proteins (GSVIVG01011129001 (SEQ ID NO: 132) and GSVIVG01017925001 (SEQ ID NO: 133)) |
| 11 | *Solanum tuberosum* | Potato | 2 genes (PGSC0003DMG40001 2880 (SEQ ID NO: 134) and PGSC0003DMG401020 126 (SEQ ID NO: 136)) | 2 proteins (PGSC0003DMG40001 2880 (SEQ ID NO: 136) and PGSC0003DMG401020 126 (SEQ ID NO: 137)) |
| 12 | *Solanum lycopersicum* | Tomato | 2 genes (Solyc09G001948 (SEQ ID NO: 138), Solyc06T002747 (SEQ ID NO: 139)) | 3 proteins, two being isoforms (Solyc09G001948.1 (SEQ ID NO: 140), Solyc06T002747.1 (SEQ ID NO: 141) and Solyc06T002747.2 (SEQ ID NO: 142)) |
| 13 | *Manihot esculenta* | Cassava | 3 genes (Manes.03G181400 (SEQ ID NO: 143), Manes.05G076800 (SEQ ID NO: 144) and Manes.15G026400 (SEQ ID NO: 145)) | 3 proteins (Manes.03G181400 (SEQ ID NO: 146), Manes.05G076800 (SEQ ID NO: 147) and Manes.15G026400 (SEQ ID NO: 148)) |
| 14 | *Brassica napus* | Canola | 7 genes (A10p25140 (SEQ ID NO: 149), C09p64040 (SEQ ID NO: 150), C02p05870 (SEQ ID NO: 151), A02p01660 (SEQ ID NO: 253), C09p56950 (SEQ ID NO: 152), A03p09430 (SEQ ID NO: 153) and C03p11010 (SEQ ID NO: 154)) | 7 proteins (A10p25140 (SEQ ID NO: 155), C09p64040 (SEQ ID NO: 156), C02p05870 (SEQ ID NO: 157), A02p01660 (SEQ ID NO: 158), C09p56950 (SEQ ID NO: 159), A03p09430 (SEQ ID NO: 160) and C03p11010 (SEQ ID NO: 161)) |
| 15 | *Brassica oleracea* | | 2 genes (BolC2t06532H (SEQ ID NO: 162) and BolC9t58549H (SEQ ID NO: 163)) | 2 proteins (BolC2t06532H (SEQ ID NO: 164) and BolC9t58549H (SEQ ID NO: 165)) |
| 16 | *Brassica carinata* | Abyssinian mustard | 5 genes (BcaB01g05140 (SEQ ID NO: 166), BcaB08g36582 (SEQ ID NO: 167), BcaC04g18577 (SEQ ID NO: 168), BcaNung01177 (SEQ ID NO: 169), BcaNung01857 (SEQ ID NO: 170)) | 4 proteins (BcaB01g05140 (SEQ ID NO: 171), BcaB08g36582 (SEQ ID NO: 172), BcaC04g18577 (SEQ ID NO: 173), BcaNung01177 (SEQ ID NO: 174), BcaNung01857 (SEQ ID NO: 175)) |
| 17 | *Brassica rapa* | Mustard | 4 genes (BraA02t05145Z (SEQ ID NO: 176), BraA03t10261Z (SEQ ID NO: 177), BraA10t44106Z (SEQ ID NO: 178) and BraA10t44576Z (SEQ ID NO: 179)) | 4 proteins (BraA02t05145Z (SEQ ID NO: 180), BraA03t10261Z (SEQ ID NO: 181), BraA10t44106Z (SEQ ID NO: 182) and BraA10t44576Z (SEQ ID NO: 183)) |
| 18 | *Phaseolus vulgaris* | Common beans | 4 genes (Phvul.001G151300 (SEQ ID NO: 184), Phvul.002G047400 (SEQ ID NO: 185), Phvul.006G001300 (SEQ ID NO: 186) and Phvul.007G054500 (SEQ ID NO: 187)) | 4 proteins (Phvul.001G151300 (SEQ ID NO: 188), Phvul.002G047400 (SEQ ID NO: 189), Phvul.006G001300 (SEQ ID NO: 190) and Phvul.007G054500 (SEQ ID NO: 191)) |
| 19 | *Glycine max* | Soybean | 6 genes (Glyma.01G239200 (SEQ ID NO: 192), (Glyma.03G154000 (SEQ ID NO: 193), Glyma.10G250100 (SEQ ID NO: 194), Glyma.11G004300 (SEQ ID NO: 195), Glyma.19G156400 (SEQ ID NO: 196) and Glyma.20G143500 (SEQ ID NO: 197)) | 6 proteins (Glyma.01G239200 (SEQ ID NO: 254), (Glyma.03G154000 (SEQ ID NO: 198), Glyma.10G250100 (SEQ ID NO: 199), Glyma.11G004300 (SEQ ID NO: 200), Glyma.19G156400 (SEQ ID NO: 201) and Glyma.20G143500 (SEQ ID NO: 202)) |
| 20 | *Pisum sativum* | Pea | 3 genes (Psat6g208840 (SEQ ID NO: 203), Psat4g211360 (SEQ ID NO: 204) and Psat3g064160 (SEQ ID NO: 205)) | 3 proteins (Psat6g208840 (SEQ ID NO: 206), Psat4g211360 (SEQ ID NO: 207) and Psat3g064160 (SEQ ID NO: 208)) |
| 21 | *Eucalyptus grandis* | Eucalyptus | 1 gene (Eucgr.H03553 (SEQ ID NO: 209)) | 1 protein (Eucgr.H03553 (SEQ ID NO: 210)) |
| 22 | *Theobroma cacao* | Cocoa | 2 genes (Thecc.01G278800 (SEQ ID NO: 211) and Thecc.04G227100 (SEQ ID NO: 212)) | 2 proteins (Thecc.01G278800 (SEQ ID NO: 213) and Thecc.04G227100 (SEQ ID NO: 214)) |
| 23 | *Gossypium raimondii* | Cotton | 2 genes (Gorai.005G041500 -(SEQ ID NO: 215), and Gorai.006G237100 (SEQ ID NO: 216) | 4 proteins, 3 being isoforms (Gorai.005G041500 - Gorai.005G041500.1 protein (SEQ ID NO: 217), Gorai.006G237100/isofo rmGorai.006G237100.1 (SEQ ID NO: 218), Gorai.006G237100/isofo rmGorai.006G237100.5 (SEQ ID NO: 219), Gorai.006G237100/isofo rmGorai.006G237100.3) (SEQ ID NO: 220) |
| 24 | *Lactuca sativa* | Lettuce | 2 genes (Lsat_1_v5_gn_5_1881 80 (SEQ ID NO: 221), Lsat_1_v5_gn_9_3640) (SEQ ID NO: 222) | 2 proteins (Lsat_1_v5_gn_5_1881 80 (SEQ ID NO: 223), Lsat_1_v5_gn_9_3640) (SEQ ID NO: 224) |
| 25 | *Carica papaya* | Papaya | 2 proteins (Cpa.g.sc147.3 (SEQ ID NO: 225), Cpa.g.sc370.9 (SEQ ID NO: 226)) | 2 proteins (Cpa.g.sc147.3 (SEQ ID NO: 227), Cpa.g.sc370.9 (SEQ ID NO: 228)) |
| 26 | *Ricinus communis* | Castor bean | 2 proteins (29844.t000205 (SEQ ID NO: 229) and 29647.t000089(SEQ ID NO: 230)) | 2 proteins (29844.t000205 / isoform 29844.m003359 (SEQ ID NO: 231) and 29647.t000089/isoform 29647.m002081 (SEQ ID NO: 232)) |
| 27 | *Citrus clementine* | Orange | 2 genes (Ciclev10004440m.g (SEQ ID NO: 233) and Ciclev10019087m.g (SEQ ID NO: 234)) | 2 proteins (Ciclev10004440m.g (SEQ ID NO: 235) and Ciclev10019087m.g (SEQ ID NO: 236)) |
| 28 | *Citrus sinensis* | Sweet orange | 2 genes (orange1.1g005282m.g) (SEQ ID NO: 237) and orange1.1g004992m.g (SEQ ID NO: 238) | 5 proteins, being: orange1.1g005282m) (SEQ ID NO: 239) (orange1.1g005044m) isoform 1 (SEQ ID NO: 240), (orange1.1g004992m) isoform 2 (SEQ ID NO: 241), orange1.1g005144m) isoform 3 (SEQ ID NO: 242), orange1.1g005088m) isoform 4 (SEQ ID NO: 243) |
| 39 | *Miscanthus sinensis* | | 2 genes (Misin17G131600 (SEQ ID NO: 244) and Misin16G132500 (SEQ ID NO: 245) | 3 proteins (isoforms Misin17G131600.1 (SEQ ID NO: 246) and Misin17G131600.2 (SEQ ID NO: 247)) and Misin16G132500 (SEQ ID NO: 248)) |
| 30 | *Secale cereale* | Rye | - | - |
| 31 | *Beta vulgaris* | Beetroot | 2 gene (EL10Ac7g17234 (SEQ ID NO: 249), EL10As3g23353 (SEQ ID NO: 250)) | 2 proteins EL10Ac7g17234 (SEQ ID NO: 251), EL10As3g23353 (SEQ ID NO: 252)) |

SEQ ID NO: 263 is the amino acid sequence of the region conserved in AIP10 proteins, consensus among the sequences of the species identified in Table 1, called the "AIP10 domain".

### BRIEF DESCRIPTION OF THE FIGURES

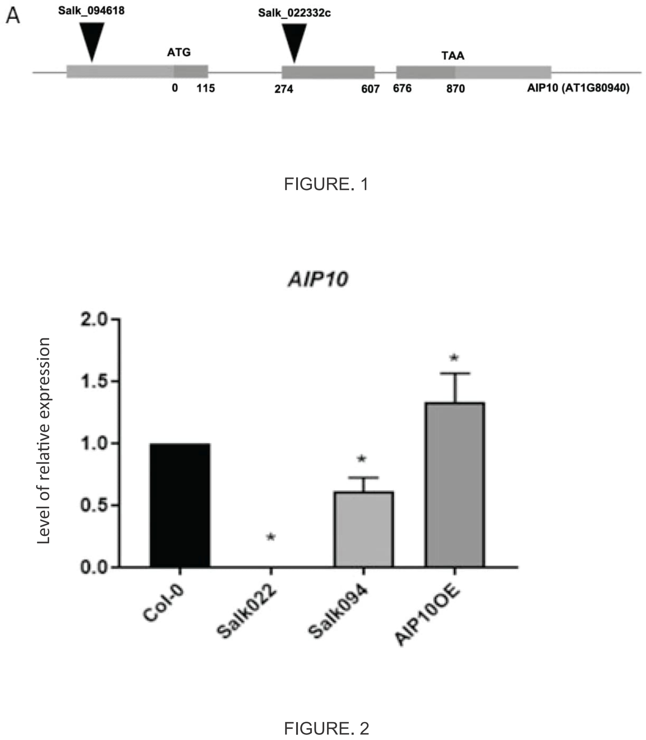

Figure 1 shows a scheme localizing T-DNA insertion in *A. thaliana* mutants with partial suppression and complete suppression of *AIP10* gene expression. Two types of T-DNA insertion mutants were selected, seeking complete gene silencing (*knockout*) or partial silencing (*knockdown*)*,* for functional studies of the effects on plant metabolism.
Figure 2 shows the relative expression of *AIP10* mRNA in Salk_022, Salk_094 mutants, and in plants expressing *AIP10* under control of the constitutive promoter 35S (AIP10^{OE}) analyzed by qRT-PCR and normalized by the mRNA levels of the gene *UBI14* and *GAPDH.* The data were compared with those obtained with Col-0 (WT) plants. The bars indicate mean ± standard deviation and *significantly different from Col-0 (WT) with p<0.05. Test *one-way* ANOVA - Dunnett. The mutant Salk_022, which showed total silencing of gene expression *aip10* (mutant *aip10ko),* and the mutant Salk_094, which showed partial silencing of gene expression *aip10* (mutant *aip10kd)* were selected.

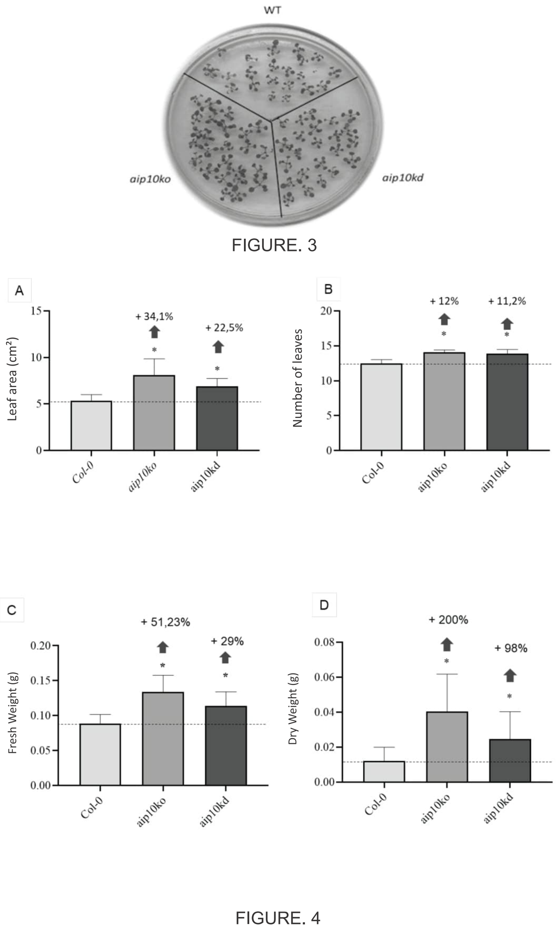

Figure 3 shows the germination of seeds Col-0 (WT), *aip10kd* and *aip10ko.* Total silencing of its gene expression of the *AIP10* gene of *A. thaliana* (mutant *aip10ko)* a presents more positive effects at the beginning of vegetative development, which are seen less expressively in plants with only the reduction of the expression of the *AIP10* gene (mutant *aip10kd).*
Figure 4 shows the analysis of vegetative development characteristics of Col-0 (WT), *aip10kd* and *aip10ko* plants, in which *aip10ko* and *aip10kd* plants have greater (A) leaf area, (B) number of leaves, (C) fresh weight, and (D) dry weight. Total gene expression silencing of the gene *AIP10* of *A. thaliana* (mutant *aip10ko)* apresents more positive effects on the vegetative growth thereof, which are seen less expressively in plants with only the reduction of the expression of the AIP10 gene (mutant *aip10kd).* The data were compared with those obtained with Col-0 (WT) plants. The bars indicate mean ± standard deviation and * significantly different from Col-0 (WT) with p<0.05. Test *one-way* ANOVA - Dunnett. *The percentages in which the data quantified in each mutant (aip10ko and aip10kd) is greater than that quantified in wild plants are indicated in the Figure, under the different conditions tested.*

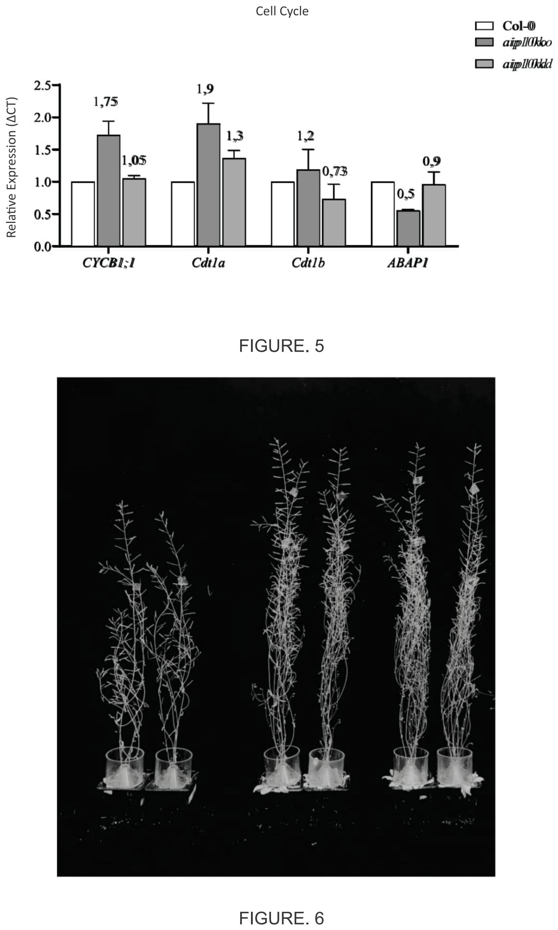

Figure 5 shows the differential expression of cell cycle-related genes in Col-0 (WT) plants, *aip10kd* and *aip10ko. aip10ko* plants perform better in all analyzed vegetative growth traits, compared to plants with reduced levels of *aip10 (aip10kd).* Corroborating these data, the expression of positive cell division marker genes (CycB1;1 and Cdt1a) was induced, and of the negative regulator ABAP1 was reduced, both more significantly in the mutant *aip10ko* compared to the mutant *aip10kd.* The gene expression analysis was analyzed by qRT-PCR and the data were normalized with *UBI14* and *GAPDH* as reference genes. The data presented represent mean values obtained from independent amplification reactions (n=3) and biological replicates (n=3). The bars indicate mean ± standard deviation. *The quantified expression values in each mutant (aip10ko and aip10kd) are shown in the Figure, in the different genes tested.*
Figure 6 shows a representative image comparing Col-0 (WT), *aip10kd* and *aip10ko* plants grown *in vivo* under a 16h/8h photoperiod with 57 days after germination (DAG). The total silencing of its gene expression of the *AIP10* gene from *A. thaliana* (mutant *aip10ko)* a presents positive effects on the reproductive growth thereof, which are seen less expressively in plants with only the reduction of expression of the *AIP10* gene (mutant *aip10kd).*

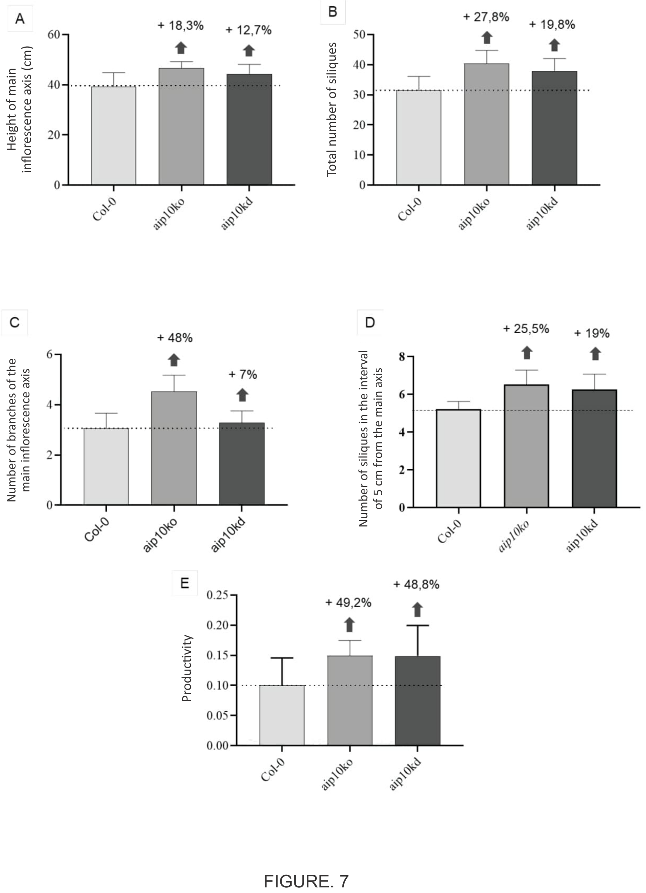

Figure 7 shows the analysis of reproductive development traits of Col-0 (WT), Salk094 (*aip10kd*) and *aip10ko* plants, indicating that plants with partial or total suppression of the *AIP10* gene have greater (A) height of the main inflorescence axis, (B) total number of siliques per plant, (C) number of branches of the main inflorescence axis, (D) number of siliques in the 5 cm interval, (E) on the main axis and (F) total number of seeds per plant (productivity). Surprisingly, the total silencing of the *AIP10* gene, involved in the control of cell divisions, does not lead to a penalty in its development, nor to a phenotype of lethality of the embryo. On the contrary, *aip10ko* plants show better performance in all analyzed characteristics of reproductive development (productivity), compared to plants with reduced levels of aip10 (*aip10kd*). The bars represent means ± standard deviation and * significantly different from Col-0 (WT) with p<0.05. Test *one-way* ANOVA - Dunnett. *The percentages in which the data quantified in each mutant (aip10ko and aip10kd) is greater than that quantified in wild plants are indicated in the Figure, under the different conditions tested.*

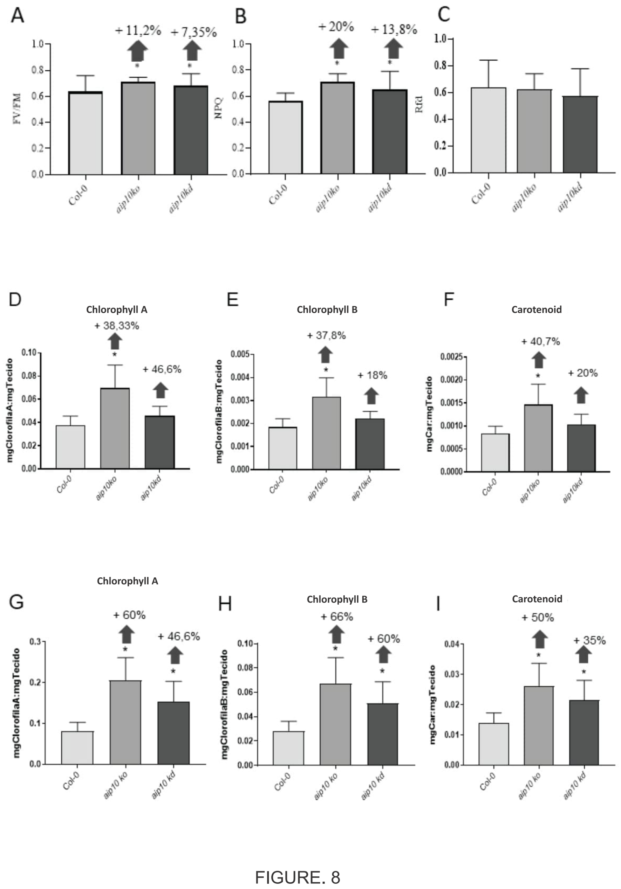

Figure 8 shows the analysis of chlorophyll content and photosynthesis parameters of Col-0 (WT), *aip10kd* and *aip10ko* plants, indicating that *aip10ko* and *aip10kd* plants display better photosynthetic efficiency parameters at 11 DAG, including higher (A) photochemical efficiency of PSII under dark adaptation (Fv/Fm), (B) non-photochemical quenching (NPQ), and (C) plant vitality (Rfd) (n=15), (D) chlorophyll A content, (E) chlorophyll B content, and (F) carotenoids in plants at 11 DAG, (G) chlorophyll A content, (H) chlorophyll B content, and (I) carotenoids in plants at 32 DAG (n=10). Total silencing of *AIP10* differentially affected photosynthetic traits in 11 and 35 DAG plants, showing a higher content of chlorophyll A, B and carotenoids when compared to Col-0 (WT), even higher than what was observed in plants with reduced levels of *aip10 (aip10kd).* The bars represent means ± standard deviation and * significantly different from Col-0 (WT) with p<0.05. Test *one-way* ANOVA - Dunnett. *The percentages at which the quantified data in each mutant (aip 10ko and aip10kd) is greater than that quantified in wild plants are indicated in the Figure under the different conditions tested.*

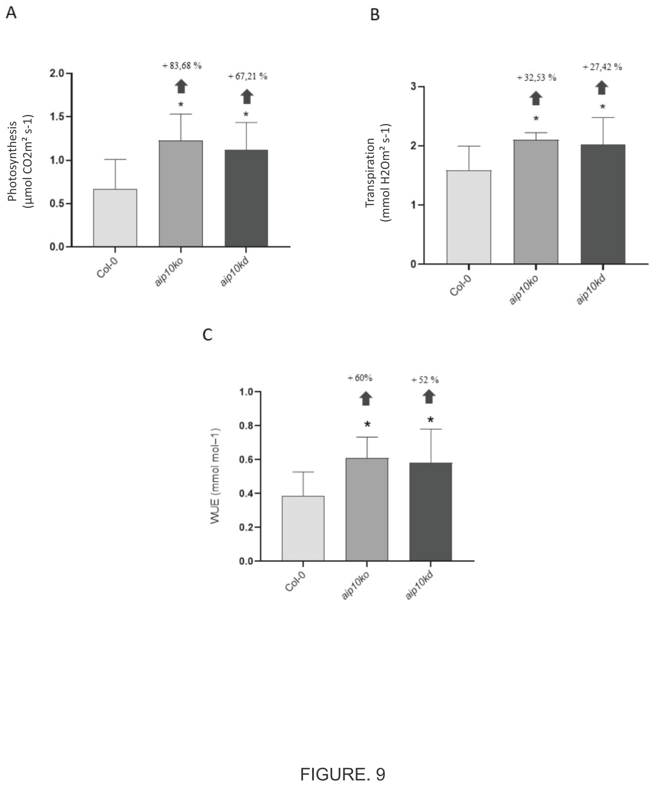

Figure 9 shows the analysis of carbon fixation through the photosynthetic content of mutants *aip10* by means of parameters of photosynthetic efficiency of Col-0 (WT) plants, *aip10*ko and *aip10*kd with 25 DAG, indicating that *aip10ko* and *aip10kd* plants have higher (A) net CO assimilation rates₂; (B) Transpiration rate and (C) More efficient use of water. Measurements were made with LICOR-6400 XT at 22°C in CO₂ ambient at 400 µmol photons m⁻²s⁻¹ between 8:00 a.m. and 10:00 a.m. Mutants *aip10ko* have a better photosynthetic efficiency using the absorbed light energy more efficiently. Total silencing of *AIP10* affected photosynthetic performance in 25 DAG plants, showing higher rates of photosynthetic carbon assimilation per unit of leaf area, than in Col-0 (WT) plants and higher than those observed in plants with reduced levels of *aip10 (aip10kd).* The bars represent means ± standard deviation and * significantly different from Col-0 (WT) with p<0.05. Test *one-way* ANOVA - Dunnett (n=10). *The percentages at which the quantified data in each mutant (aip10ko and aip10kd) is greater than that quantified in wild plants are indicated in the Figure under the different conditions tested.*

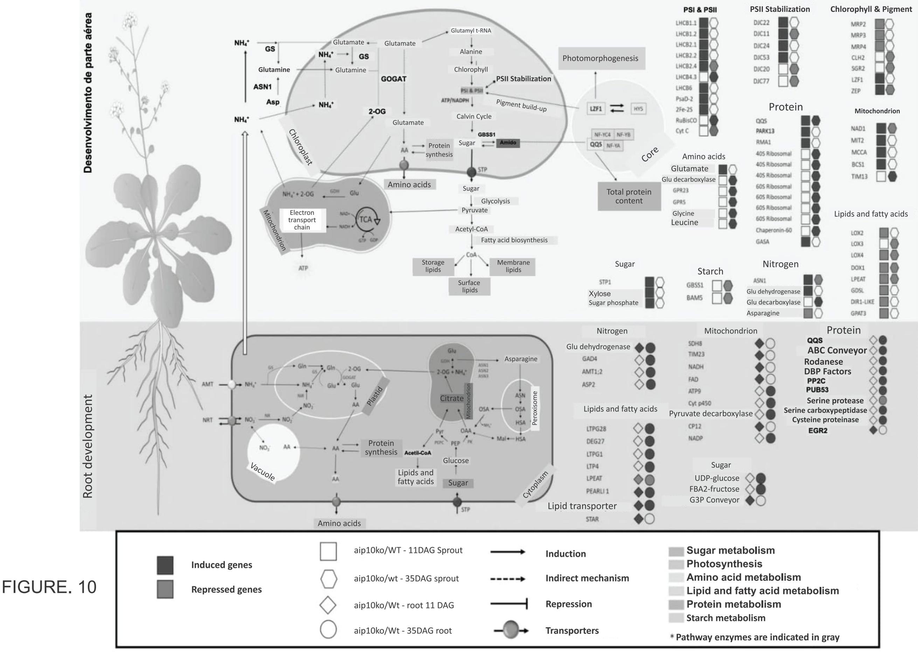

Figure 10 shows a scheme of the main genes differentially expressed in mutant plants *aip10ko* in aerial part and roots. Genes involved in CO₂ absorption and carbon metabolism, synthesis, and degradation of photosynthetic pigments. Analysis of the metabolism of energy, nitrogen, protein, amino acids, starch and sucrose in leaves and roots. The color red refers to genes that are upregulated and the color blue refers to genes that are downregulated. Squares are for differentially expressed genes (DEGs) found in the 11DAG shoot transcriptome and hexagons for 35 DAG shoots. The rhombus is for DEGs found in the transcriptome from root 11 DAG and the circle for root 35DAG. The icons present in the figure were highlighted above. The main products of each route were highlighted with different colors as indicated in the figure. Silencing *AIP10* triggers transcriptional reprogramming of different genes involved in pathways that have a major impact on development and response to the environment.

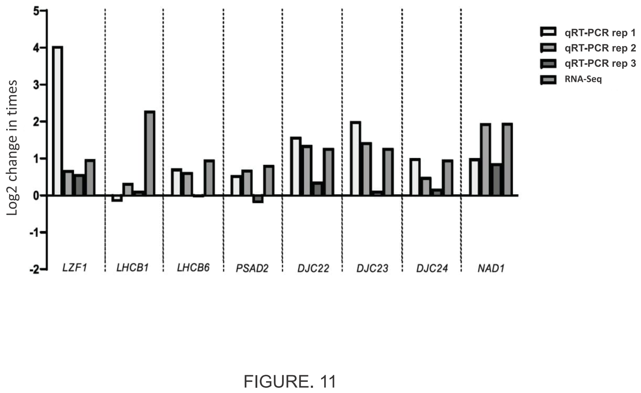

Figure 11 shows the analysis of the differential expression of 8 photosynthetic genes of *aip10ko* plants in relation to Col-0 (WT) plants by qRT-PCR in the transcriptome of leaves with 11DAG, which indicated that in plants *aip10ko* the photosynthesis genes are more expressed than in the Col-0 (WT) plant. Data were normalized with *UBI14* and *GAPDH* as reference genes. The data represent mean values obtained from independent amplification reactions (n = 3) and biological replicates (n = 3). Mutants *aip10ko* had more highly expressed light-harvesting complex genes PSI and PSII and those linked to light absorption/protection, as well as more prominent photosynthetic performance.

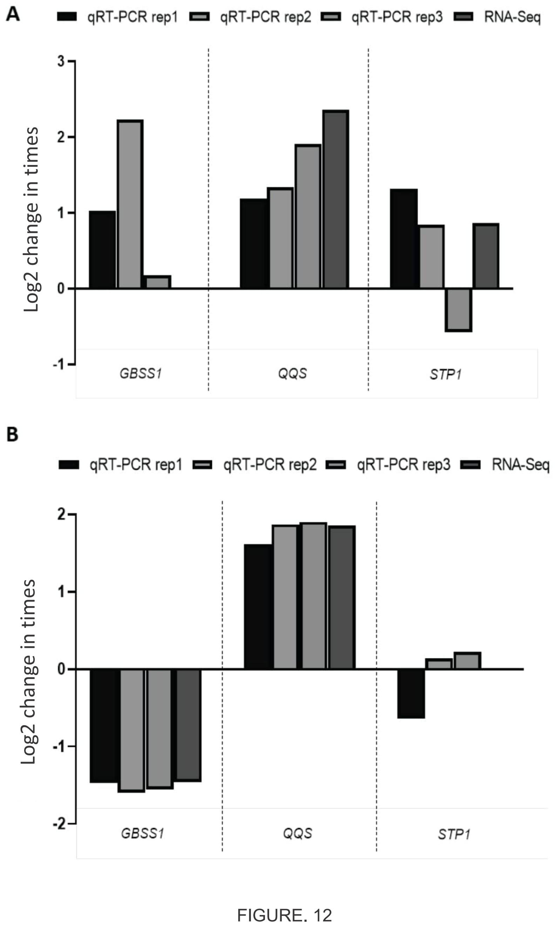

Figure 12 shows an analysis of 3 carbon metabolism genes by qRT-PCR differentially expressed in the transcriptome of leaves of *aip10ko* plants in relation to Col-0 (WT) plants with (A) 11 DAG and (B) 35 DAG indicating that *aip10ko* plants have more highly expressed carbon metabolism genes in relation to Col-0 (WT) plants. Data were normalized with *UBI14* and *GAPDH* as reference genes. The data represent mean values obtained from independent amplification reactions (n=3) and biological replicates (n=3). To meet their metabolic needs, in addition to high carbon absorption, *aip10ko* plants have an efficient carbon fixation, maintained in both young and mature plants.

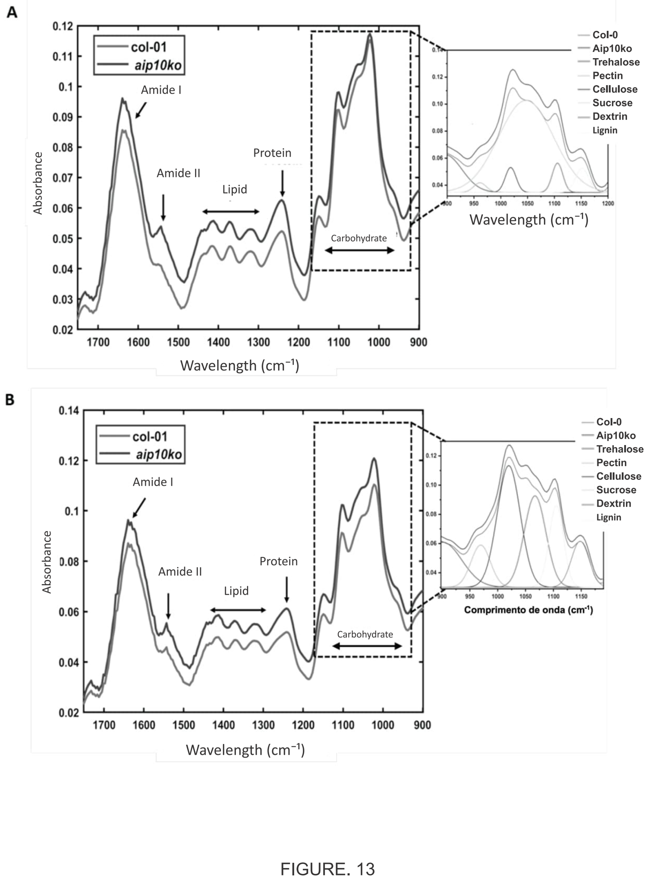

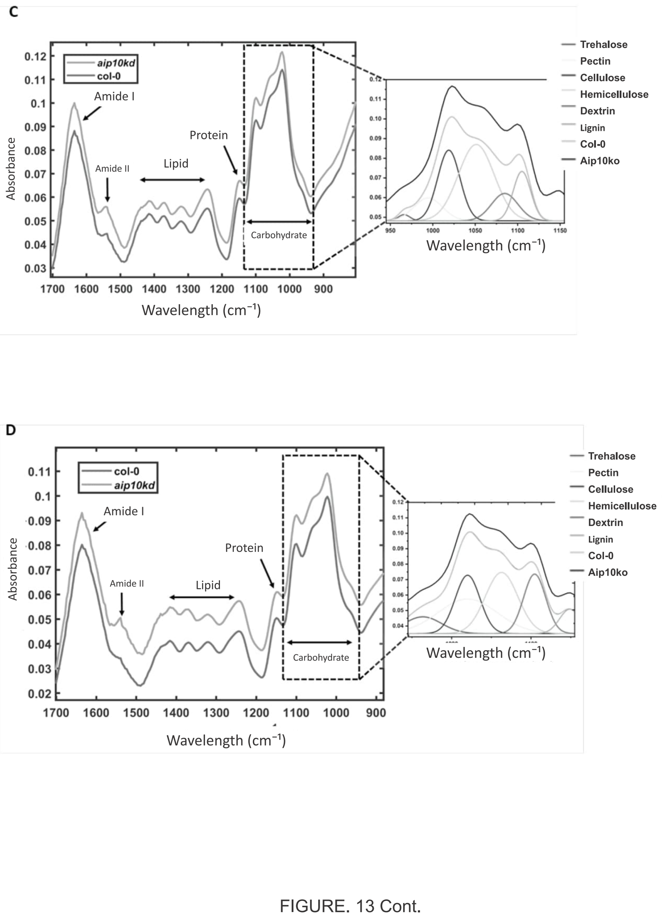

Figure 13 shows the comparison of the metabolic profile of Col-0 (WT) plants and *aip10ko*) plants, in which *aip10ko* plants showed higher average spectra obtained by the ATR-FTIR technique in the region of 900-1700 cm⁻¹ in leaves with (A) 20DAG and (B) 35DAG, compared to Col-0 (WT). *aip10kd* plants also showed higher average spectra obtained by the ATR-FTIR technique in the region of 900-1700 cm⁻¹ in leaves with (C) 20DAG and (D) 35DAG, in relation to Col-0 (WT). The analyses were carried out on 2 different leaves of 4 individuals of each genotype. To meet their metabolic needs, in addition to high carbon absorption, *aip10ko* plants have efficient carbon fixation, maintained in both young and mature plants, and higher than those observed in plants with reduced levels of *aip10 (aip10kd).*

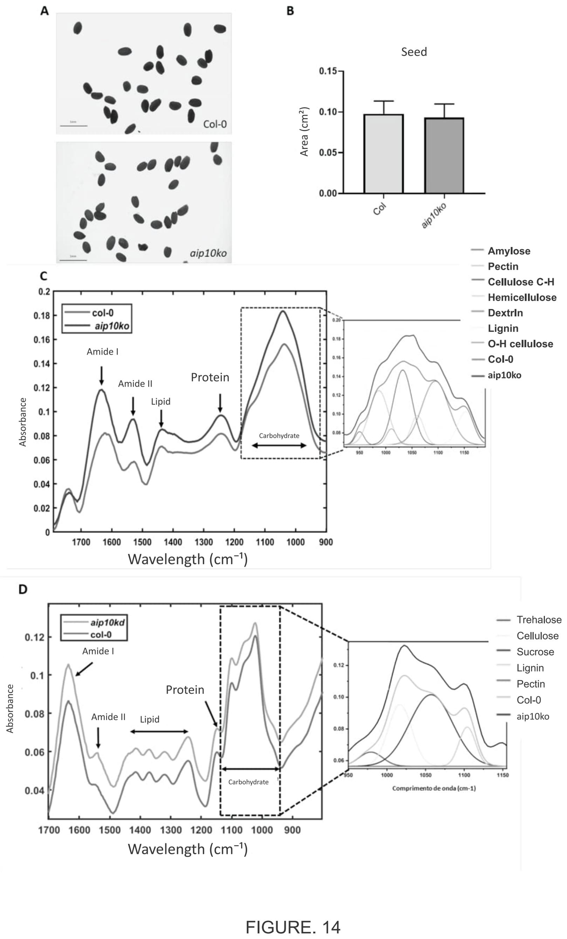

Figure 14 shows an analysis of the metabolic content in Col-0 (WT), *aip10ko* and *aip10kd* seeds. (A) *aip10ko* seeds were evaluated under microscopy and showed no difference in surface area compared to (B) Col-0(WT). The bars represent means ± SD (standard deviation) and * significantly different from Col-0 (WT) with p<0.05. Student's t-test. *(C) aip10ko* seeds showed higher average spectra obtained by the ATR-FTIR technique in the region of 900-1700 cm⁻¹ compared to Col-0 (WT), as well as (D) *aip10kd* seeds also showed higher average spectra. The analyses were carried out on a macerate of 100 seeds of each genotype. *aip10ko* seeds showed higher nutritive value (with more proteins, lipids and carbohydrates). These metabolic changes are also fundamental for the better vigor of mutants linked to the mobilization of metabolites during germination until seedling establishment.

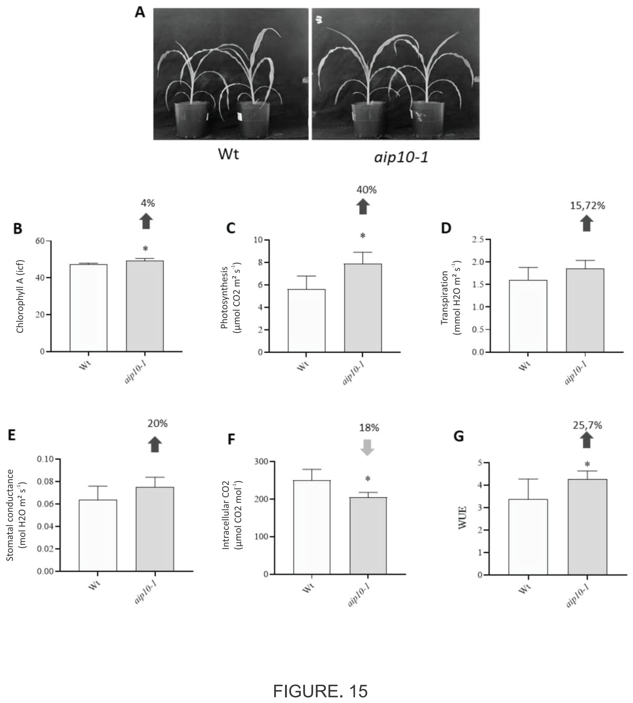

Figure 15 shows the analysis of chlorophyll content and photosynthesis parameters of mutant maize plants such as the knockout of the *AIP10* gene through CRISPR gene editing. The edited maize lines (*aip10-1* and *aip10-2*) are considered total knockout because they changed the reading phase of the AIP10 protein, through the deletion of nucleotides in the coding region. The maize plants edited for *aip10 (aip10-1),* as well as in mutants *aip10ko* in *A. thaliana,* presented: (A) greater development in relation to WT with 25DAG; (B) higher Chlorophyll A content, chlorophyll readings were performed by the ClorofiLOG ^{®} portable chlorophyll meter equipment; (C) higher net CO assimilation rates₂; (D) higher transpiration rate; (E) higher stomatal conductance; (F) higher intracellular CO concentration₂; (G) higher water efficiency (WUE) (n= 8), compared to Col-0 plants (WT). The measurements were performed with WT_CRISPR and *aip10_CRISPR* plants with 25DAG with the LICOR-6400 XT instrument at 25°C in CO₂ ambient at 400µmol and 500µmol of photons m⁻²s⁻¹. The box plot represent means ± standard deviation and * significantly different from WT with p<0.05. Student's t-test. *The percentages in which the data quantified in the aip10-1 mutant is greater than that quantified in wild plants are indicated in the Figure, under the different conditions tested.*

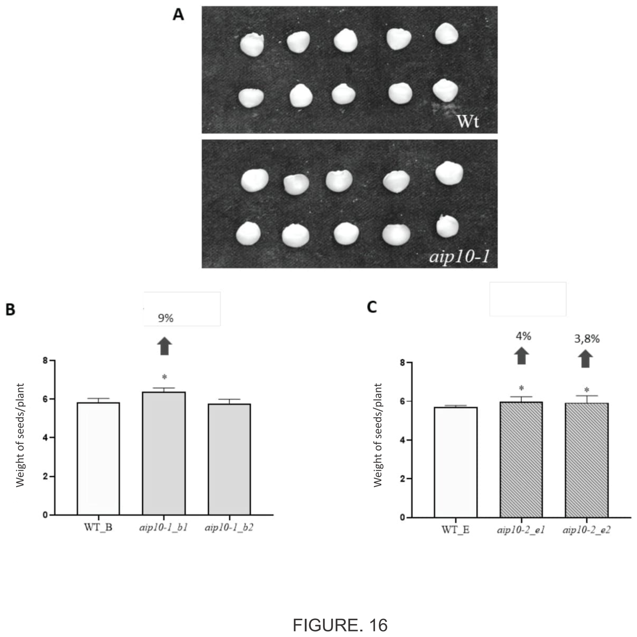

Figure 16 shows the seed analysis of two CRISPR strains *aip10.* (A) Seeds of wild plants and *aip10-1 (event B)* did not show differences in the surface area of the seeds. (B) However, seeds from *aip10-1 (event B)* plants showed a higher seed weight when evaluated in a "pool" of 20 seeds (in 12 replications). (C) Plant seeds *aip10-2 (event* E) showed a higher seed weight when evaluated in a pool of 20 seeds (in 12 replications). The bar graph represents means ± standard deviation and * significantly different from WT with p<0.05. Student's t-test. Maize plants edited for *aip10 (aip10-1 and aip10-2)* showed higher seed weight in the two distinct gene editing events, shown in the graphs. *The percentages in which the data quantified in the edited mutant aip10 is greater than that quantified in wild plants are indicated in the Figure, under the different conditions tested.*

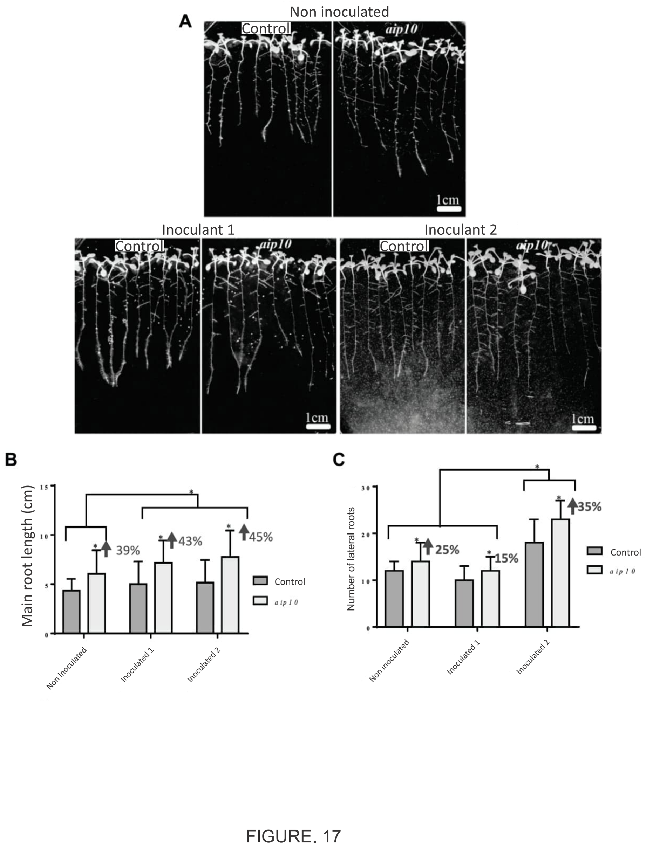

Figure 17 shows the effects of the use of bioinoculants containing diazotrophic bacteria on the *in vitro* growth of genetically modified plants regarding the *AIP10* gene. (A) *A. thaliana aip10ko* plants inoculated with diazotrophic bacteria have more shoots and root system compared to Col-0 plants (WT) Bioinoculants increase (B) the length of the taproot and (C) the number of lateral roots. Plants 10 DAG, 3 DAI. The bars indicate mean ± standard deviation. The data were analyzed *by two-way* ANOVA and the means were compared by Tukey's test at 5% probability (p<0.05). *, indicates statistical difference between the averages. DAG: days after germination; DAI: days after inoculation. Plants *aip10ko* (10 DAG) show a better growth response to inoculation with bioinoculants containing diazotrophic bacteria, compared to inoculated plants of the Col-0 (WT) type. *The percentages in which the quantified value in the mutant is greater than that quantified in wild plants are indicated in the Figure, under the different conditions tested (not inoculated or inoculated).* Bioinoculant 1: *A. baldaniorum* Sp245; Bioinoculant 2: *H. seropedicae* HRC54.

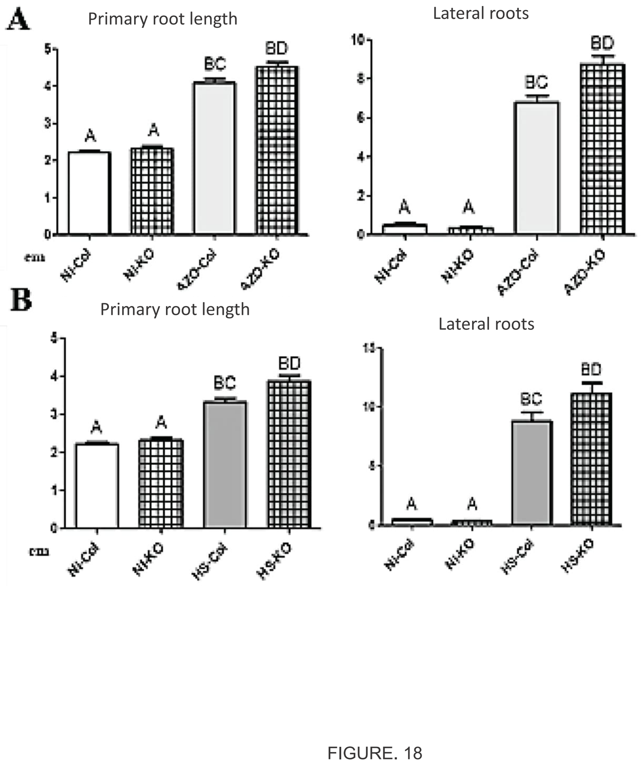

Figure 18 shows the effects on root architecture in the *in vitro* growth of *aip10ko* plants with the use of different bioinoculants, containing the diazotrophic bacteria *A. baldaniorum* or *H. seropedicae. A. thaliana aip10ko* plants inoculated with two different species of beneficial diazotrophic bacteria have a more developed root system, compared to Col-0 (WT) plants. Bioinoculants increase (A) root length and (B) the number of lateral roots. NI-Col: Non-inoculated Col-0 (WT) control; NI-KO: mutant *aip10ko* not inoculated; AZO-Col: Col-0 (WT) plant inoculated with *A. baldaniorum;* AZO-KO: mutant plant inoculated with *A. baldaniorum;* HS-Col: Col-0 (WT) plant inoculated with H. *seropedicae*; HS-KO: mutant plant inoculated with *H. seropedicae.* The bars indicate mean ± standard deviation. Data were analyzed by *one-way* ANOVA and means were compared by Bonferroni's test at 5% probability (p<0.05). *aip10ko* plants show a better growth response to inoculation with different bioinoculants containing distinct species of diazotrophic bacteria, compared to inoculated plants Col-0 (WT).

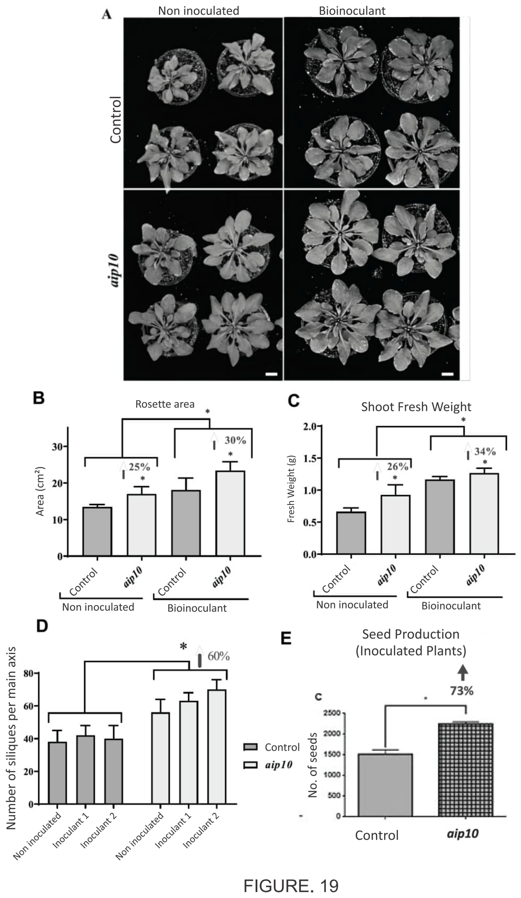

Figure 19 shows the effects of the use of bioinoculants with diazotrophic bacteria on the growth of *aip10ko* in the soil. *A. thaliana aip10ko* plants have (A-B) more developed rosettes (rosette area in cm²) and (C) shoot biomass (rosette fresh weight), compared to Col-0 (WT) plants. This increased growth in *aip10ko* plants is more pronounced when they are inoculated with bioinoculants containing diazotrophic bacteria. The final yield of the plant in relation to (D) the number of siliques and (E) seeds is higher in inoculated *aip10ko* plants than in inoculated control plants. The bars indicate mean ± standard deviation. The data were analyzed by *one-way* ANOVA and the means were compared by the Bonferroni test at 5% probability (p<0.05). Plants *aip10ko* grown in soil show better growth response to inoculation with bioinoculants containing diazotrophic bacteria, compared to inoculated plants Col-0 (WT). *The percentages in which the quantified value in the mutant is greater than that quantified in wild plants are indicated in the Figure, under the different conditions tested.* Bioinoculant 1: *A. baldaniorum* Sp245; Bioinoculant 2: *H. seropedicae* HRC54.

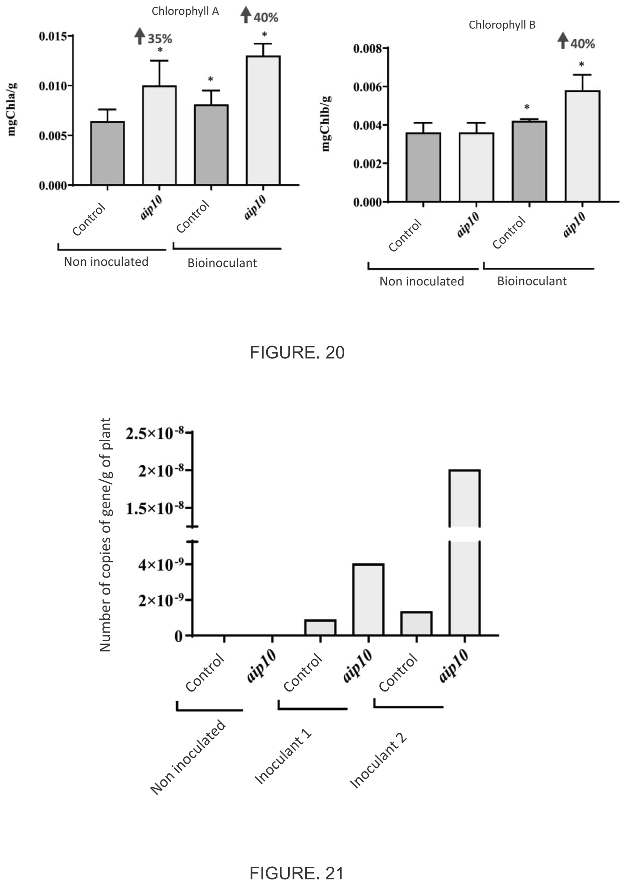

Figure 20 shows that *aip10ko* plants have higher chlorophyll content when they are associated with bioinoculants composed of diazotrophic bacteria. Plants in *A. thaliana aip10ko* have higher levels of chlorophyll a (mg Chla/g leaf) and chlorophyll b (mg Chlb/g leaf) when these plants are inoculated with the bioinoculant containing diazotrophic bacteria. Increased levels of chlorophyll have been linked to an increased level of photosynthesis. Floor plans 35 DAG, 28 DAI. DAG: days after germination; DAI: days after inoculation. Data were analyzed by *two-way* ANOVA and means were compared by Bonferroni's test at 5% probability (p<0.05). Plants of *A. thaliana aip10ko* grown in soil, associated with the use of bioinoculant containing diazotrophic bacteria, have increased levels of chlorophyll compared to plants Col-0 (WT). *The percentages in which the quantified value in the mutant is greater than that quantified in wild plants are indicated in the Figure, under the different conditions tested.* Bioinoculant 1: *H. seropedicae* HRC54.
Figure 21 shows the colonization rate in absolute single-copy values of specific genes for bioinoculants, in *A. thaliana.* The colonization rate was measured from the amplification of single-copy genes specific for bioinoculant 1 and bioinoculant 2, in mutant plants for the *AIP10* gene. All plants were grown in earth-vermiculite substrate in photoperiod 12 hours light/12 hours dark and collected 37 days after inoculation and 40 days after germination. Bioinoculant 1: *A. baldaniorum* SP245; Bioinoculant 2: *H. seropedicae* HRC54.
Figure 21 shows the colonization rate in absolute single-copy values of specific genes for bioinoculants, in *A. thaliana.* The colonization rate was measured from the amplification of single-copy genes specific for bioinoculant 1 and bioinoculant 2, in mutant plants for the *AIP10* gene. All plants were grown in earth-vermiculite substrate in photoperiod 12 hours light/12 hours dark and collected 37 days after inoculation and 40 days after germination. Bioinoculant 1: *A. baldaniorum* Sp245; Bioinoculant 2: *H. seropedicae* HRC54.

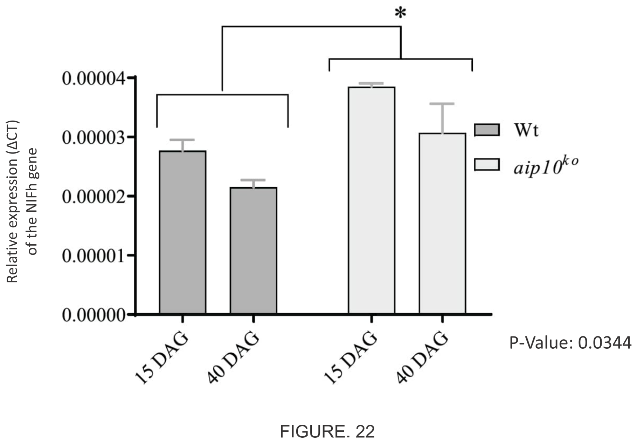

Figure 22 shows the mRNA levels of the *NifH* gene in *of A. thaliana* Col-0 (WT) plants and mutants for the *AIP10 gene.* The graph shows the expression of mRNA levels of the bacterial *NifH* from H. *seropedicae* HRC54, in control plants and mutants for the *AIP10* gene in *A. thaliana.* The results suggest that the native microbiota present in *aip10ko* plants may already have a higher biological nitrogen fixation activity due to the greater responsiveness of this mutant to beneficial bacteria. The bars indicate mean ± standard deviation. Statistical analysis with *two-way* ANOVA, Multiple Comparisons. p<0.05%. The *aip10* plants (15 DAG) showed a higher expression of the bacterial *NifH* gene, compared to the control plant. *The percentages in which the quantified value in the mutant is greater than that quantified in wild plants are indicated in the Figure, under the different conditions tested.*

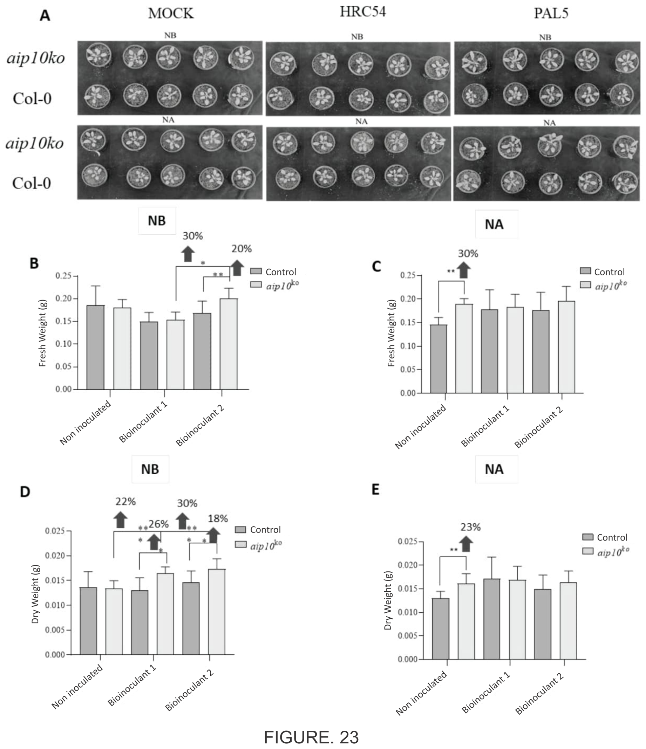

Figure 23 shows the effects of the use of bioinoculants containing diazotrophic bacteria on soil growth of *aip10ko* plants at low and high doses of chemical nitrogen fertilization. (A) *A. thaliana aip10ko* plants inoculated with bioinoculants show a more developed rosette aerial, compared to Col-0 (WT) plants in both high and low nitrogen fertilization doses. (B) Bioinoculant 2 promotes fresh weight gain in mutant *aip10ko* plants with low nitrogen availability. (C) In addition, bioinoculants 1 and 2 do not promote fresh weight gain in mutant *aip10ko* plants at high nitrogen availability. (D) Bioinoculants 1 and 2 promote dry weight increase in mutant *aip10ko* plants with low nitrogen availability. (E) Bioinoculants 1 and 2 do not promote dry weight increase in mutant *aip10ko* plants at high nitrogen availability. It was also observed that at high nitrogen dose, the uninoculated *aip10ko* mutant shows a more pronounced increase in dry weight (biomass) than the non-inoculated WT control plants. The groups were divided into plants under NB (low nitrogen availability) and NA (high nitrogen availability) conditions and inoculated with two distinct bioinoculants, called bioinoculant 1 and bioinoculant 2. In summary, the results show that (i) the beneficial effects of bioinoculants containing diazotrophic bacteria, on the accumulation of fresh and dry biomass, are more pronounced in the *aip10ko* plants, compared to the control Col-0 (WT) plants, and that (ii) these benefits are observed in the inoculation with a low dose of nitrogen chemical fertilization. These data indicate that the use of bioinoculants can largely replace the use of nitrogenous chemical fertilizers in mutant plants *aip10ko.* The bar chart represents means ± standard deviation and * significantly different from Control with p<0.05. Student's t-test within each treatment. *The percentages in which the quantified value in the mutant is greater than that quantified in wild plants are indicated in the Figure, under the different conditions tested.*

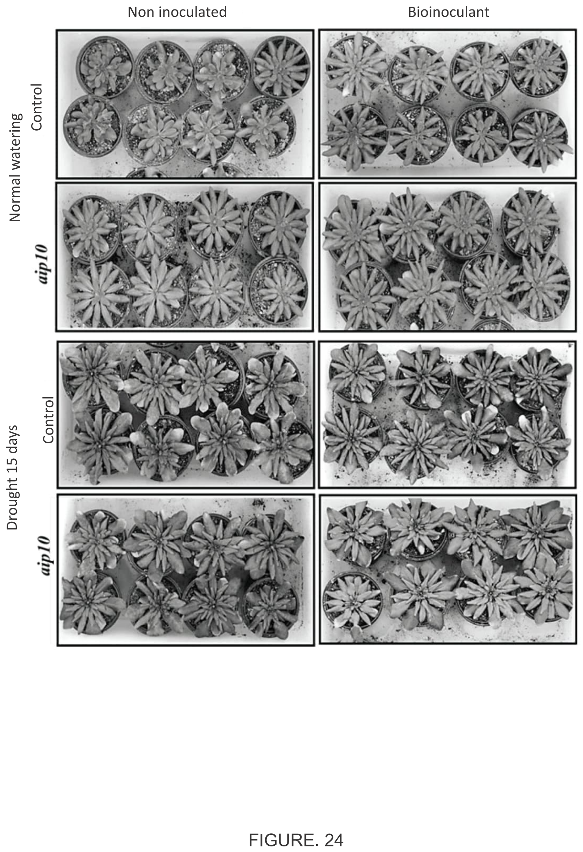

Figure 24 shows that *aip10ko* plants inoculated with bioinoculant containing beneficial diazotrophic bacteria have higher tolerance to water stress. Representative images of wild and *aip10ko* mutant plants of *A. thaliana* grown under normal conditions and under water stress conditions for 15 days. These plants were separated into two groups: non-inoculated (left) and inoculated with bioinoculant containing beneficial diazotrophic bacteria (right). During water stress, the non-inoculated plants showed strong wilt, chlorosis and anthocyanin synthesis in the leaves. On the other hand, *aip10ko* plants inoculated with the bioinoculant showed less wilting, chlorosis, and anthocyanin synthesis in the leaves, when compared to non-inoculated plants. *aip10ko* plants show greater tolerance to water stress when they are inoculated with bioinoculants containing diazotrophic bacteria, compared to non-inoculated or wild-type inoculated plants. Bioinoculant: *H. seropedicae* HRC54.

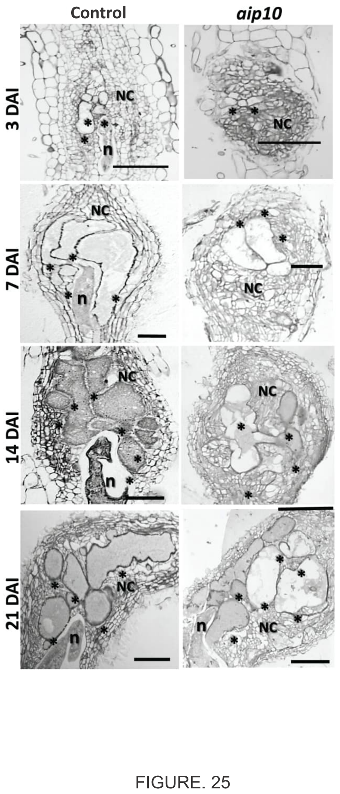

Figure 25 shows the histological analysis of the formation of nematode galls *Meloidogyne incognita* in infected WT control plants and *aip10ko* mutants. Brightfield microscope images of sections of galls stained with toluidine blue at 3, 7, 14, and 21 DAI. Asterisks, giant cells; n, nematode, NC, neighboring cells, DAI, days after inoculation. Bars = 100 µm. These data show that *A. thaliana aip10ko* plants are less susceptible to infection by root-knot nematode (*M. incognita*)*.* The silencing of *AIP10* was able to alter the morphology of gall cells, whose formation is dependent on cell cycle modulation and DNA replication. Giant cells are made up of a mixture of cells full of cytoplasm and completely empty cells and are located on the periphery of the gall, unlike control plants, which have large, central giant cells full of cytoplasm. Neighboring cells show greater disorganization than control galls.

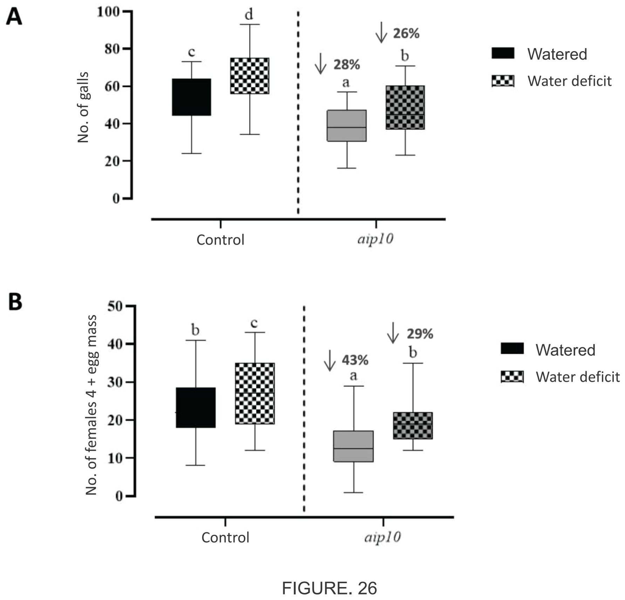

Figure 26 shows the root-knot nematode (*M. incognita*) infection test in WT control plants and in *aip10ko* mutants under normal conditions and under water deficit. (A) Number of galls per plant. (B) Number of females with egg mass per plant. It is possible to observe that *aip10ko* plants have fewer galls and fewer females with egg mass than control plants, both in normal situation and under water deficit. The data presented represent means ± standard deviation of three independent biological replications, in which a minimum of 30 seedlings of each line (WT and *aip10ko)* were evaluated for nematode infection in the soil. The data were analyzed by one-way ANOVA and the means were compared by the Scott-Knott test at 5% probability. The results show that plants of *A. thaliana aip10ko,* produced fewer galls and had fewer females with egg mass, both under normal conditions, that is, with the watered plant, as well as under water deficit. The data corroborate that the total silencing of *AIP10* makes plants less susceptible to infection by root-knot nematode *(M. incognita)* and show that this greater resistance in *aip10ko* mutants occurs even in combination with the water stress condition where nematodes become more infective.

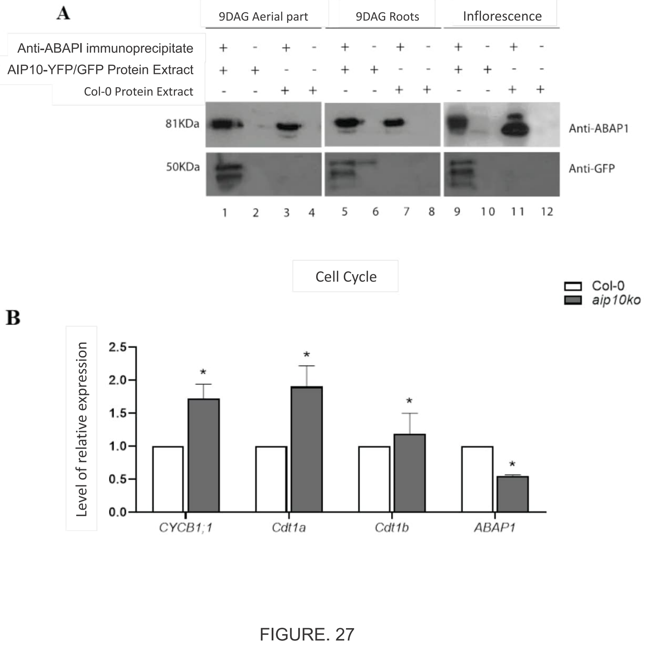

Figure 27 shows evidence that AIP10 and ABAP1 act in the same regulatory network in plants. Immunoprecipitation of protein extracts from *A. thaliana* control plants and plants expressing AIP10::YFP was performed. (A) Total protein extracts were immunoprecipitated with anti-ABAP1 and analyzed in protein blot gel with antibodies against YFP and ABAP1. The identification of 50 KDa bands in lines 1, 5 and 9 indicates that AIP10 interacts with ABAP1. (B) The expression pattern of the genes of the AIP10/ABAP1 regulatory network was evaluated in *aip10ko* mutant plants. Relative mRNA expression, by qRT-PCR, of cell cycle marker genes and ABAP1 target genes in Columbia and *aip10ko* plants with 30DAG. It was shown that *aip10ko* plants showed higher expression of the genes *Cdt1a, Cdt1b* and *CYCB1;1,* while there was a reduction in the expression of *ABAP1* compared to Col-0 (WT). The data were normalized using *UBI10* and *GAPDH* as reference genes. The values presented represent the means obtained from independent amplification reactions (n = 3) and biological replicates (n = 2). The bars indicate the standard deviation. Statistical analysis was performed using Student's t-test (p <0.05). Asterisks (*) indicate significant differences between the samples. *AIP10* and ABAP1 act on the same regulatory network in plants, where proteins interact and their expression levels are co-regulated during gene silencing. *aip10ko* mutant plants have reduced levels of ABAP1. In parallel, mutant plants have higher levels of expression of cell cycle regulatory genes, corroborating the phenotype of higher growth.

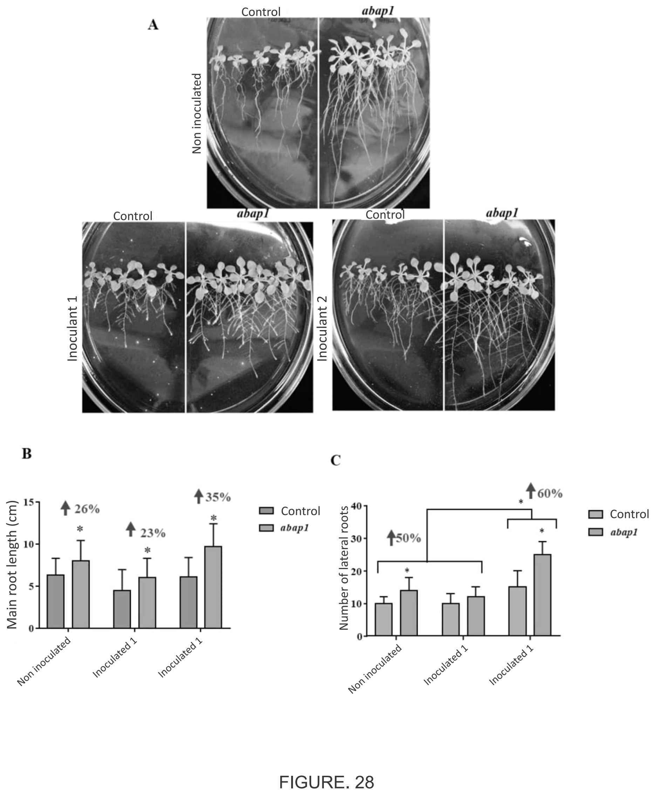

Figure 28 shows the effect of the use of bioinoculants containing diazotrophic bacteria on the growth of mutant with reduced expression of *ABAP1* (*abap1kd*)*.* (*A*) *abap1kd* plants inoculated with diazotrophic bacteria have more developed shoots and root system, compared to Col-0 (WT) plants. Bioinoculants increase (B) the length of the taproot, (C) the number of lateral roots, and (D) the rosette area. Plants 15 DAG, 7 DAI. DAG: days after germination; DAI: days after inoculation. The bars indicate mean ± standard deviation. Asterisk indicates the differences between genotypes and treatments analyzed with statistical test *two-way* ANOVA; p<0.05. DAG: days after germination; DAI: days after inoculation. *The percentages in which the quantified value in the mutant is greater than that quantified in wild plants are indicated in the Figure, under the different conditions tested.* abap1kdplants show increased biomass in response to the use of bioinoculants containing diazotrophic bacteria, compared to Col-0 (WT) plants. Bioinoculant 1: *A. baldaniorum* Sp245; Bioinoculant 2: *H. seropedicae* HRC54.

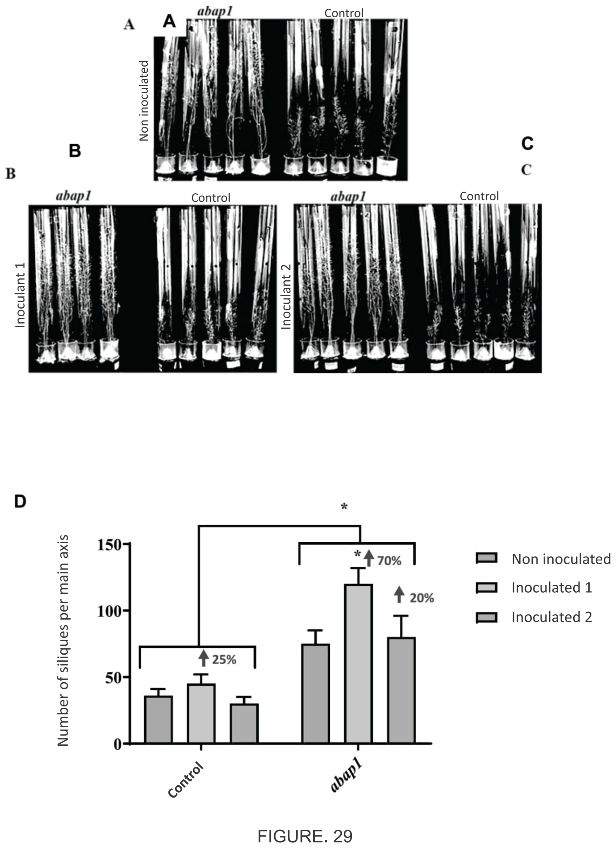

Figure 29 shows the effects of the use of bioinoculants containing diazotrophic bacteria on the reproductive development of *abap1kd* in the soil. (A) Mutant plants *abap1kd* have higher productivity under normal conditions compared to control plants, (D) in addition to having a higher number of siliques and inflorescence length. (B, C) Increased growth in *abap1kd* plants is most pronounced when plants are inoculated with bioinoculants containing diazotrophic bacteria. The bars indicate mean ± standard deviation. The data were analyzed by *one-way* ANOVA and the means were compared by the Bonferroni test at 5% probability (p<0.05). *The percentages in which the quantified value in the mutant is greater than that quantified in wild plants are indicated in the Figure, under the different conditions tested. abap1kd* plants have a higher number of siliques and inflorescence length when compared to Col-0 (WT) plants. The use of bioinoculants containing diazotrophic bacteria has an additive effect on the development of the reproductive phase of the *abap1kd* plant. Bioinoculant 1: *A. baldaniorum* Sp245; Bioinoculant 2: *H. seropedicae* HRC54.

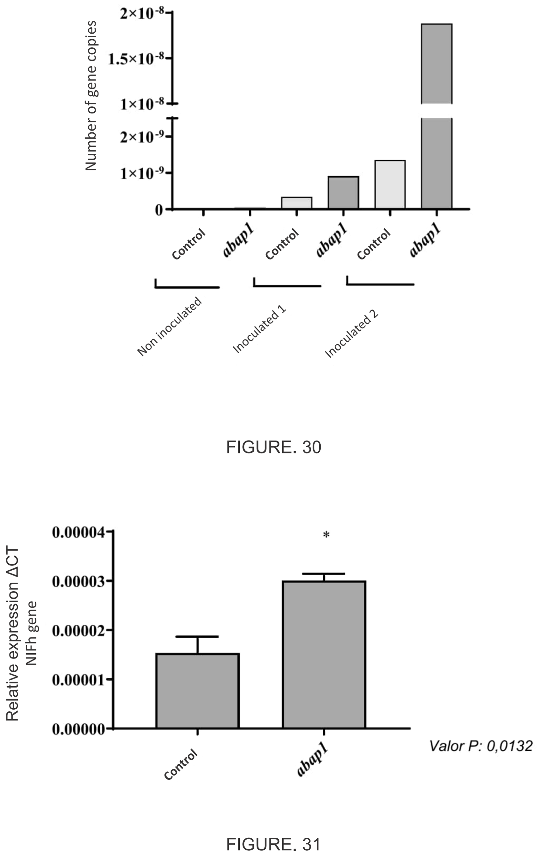

Figure 30 shows the colonization rate in absolute single-copy values of specific genes for bioinoculants. The colonization rate was measured from the amplification of specific single-copy genes for bioinoculant 1 and bioinoculant 2, in mutant plants for the *ABAP1 (abap1kd)* gene. All plants were grown in earth-vermiculite substrate in photoperiod 12 hours light/12 hours dark and collected 37 days after inoculation and 40 days after germination. *abap1kdplants* have a higher amplification of single-copy genes, specific to the bacteria that make up the bioinoculants used. Bioinoculant 1: *A. baldaniorum* Sp245; Bioinoculant 2: *H. seropedicae* HRC54.
Figure 31 shows the mRNA levels of the *NifH* gene in *A. thaliana* Col-0 (WT) plants and mutant for the *ABAP1 (abap1kd)* gene. The graph shows the expression of the mRNA levels of the bacterial *NifH* of *H. seropedicae* HRC54, in control plants and mutants *abap1kd* in *A. thaliana.* The results suggest that *abap1kd* plants may have a higher biological nitrogen fixation activity from the native microbiota present in plants. Bars indicate mean ± standard deviation. Plants with 15 DAG. Statistical analysis was performed using Student's t-test (p <0.05). Asterisk (*) indicates a significant difference between the samples. *abap1kd* plants(15 DAG) showed a higher expression of the bacterial *NifH* gene, compared to the control plant.

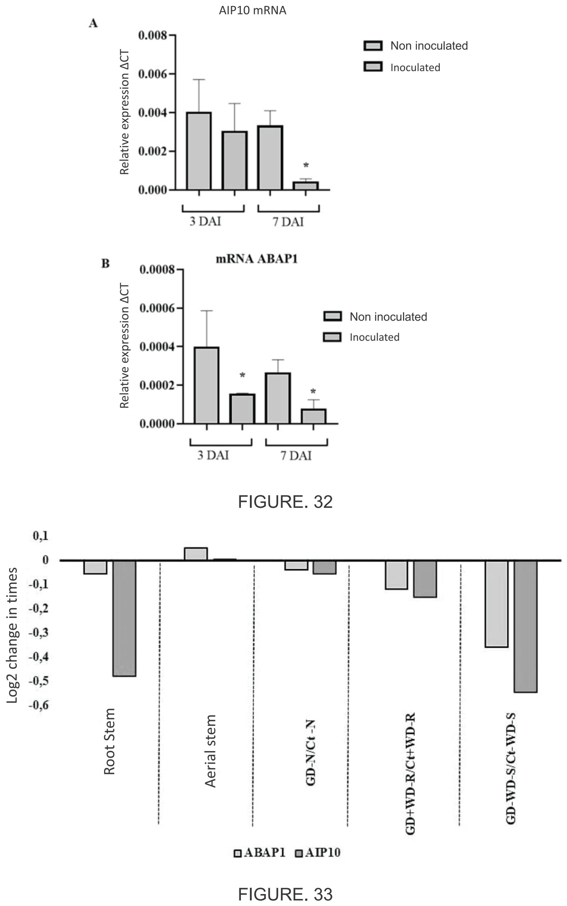

Figure 32 shows the mRNA levels of the *AIP10* and *ABAP1* genes in *A. thaliana* Col-0 (WT) plants associated with the bioinoculant. The graph shows the repression of the mRNA levels of the cell cycle repressor genes (A) *ABAP1* and (B) *AIP10* normalized relative to the non-inoculated control, at 3 and 7 days after inoculation with diazotrophic bacteria. The bars indicate mean ± standard deviation. Statistical analysis with *one-way* ANOVA with Bonferroni's Multiple Comparisons. p<0.05%. Plants of *A. thaliana* show repression of the *ABAP1* and *AIP10* genes when they are associated with bioinoculants containing diazotrophic bacteria. Because these are cell cycle repressor genes under normal conditions, these results indicate that the cell cycle is more active when the plant is associated with the bioinoculant. Bioinoculant: *H. seropedicae* HRC54.
Figure 33 shows the expression of genes homologous to *ABAP1* and *AIP10* identified in sugarcane plants, under different growth conditions, and in abiotic stress treatments. The graph shows the expression in log2 fold change, the values represent the repression of the identified genes, in relation to the control or the contrasting genotype. The mRNAs analyzed were: naturally colonized plants root (Stalk Root) and shoot (Stalk Shoot) of contrasting genotypes regarding biological nitrogen fixation; sugarcane plants grown in hydroponics and inoculated with *Gluconacetobacter diazotrophicus* (GD) at low nitrogen concentration (-N); and sugarcane plants inoculated with G. *diazotrophicus* (GD) watered (+WD) and subjected to drought (-WD), root (R), and shoot (S). Naturally associated sugarcane plants and plants inoculated with bioinoculants containing diazotrophic bacteria, in different substrates and growth conditions, show repression of the *ABAP1* and *AIP10* genes during association with the bioinoculant. Because these are cell cycle repressor genes under normal conditions, these results indicate that the cell cycle is more active when the plant is inoculated to such bacteria. The data show that this regulatory network works similarly across different plant species. Bioinoculant: G. *diazotrophicus* PAL5.

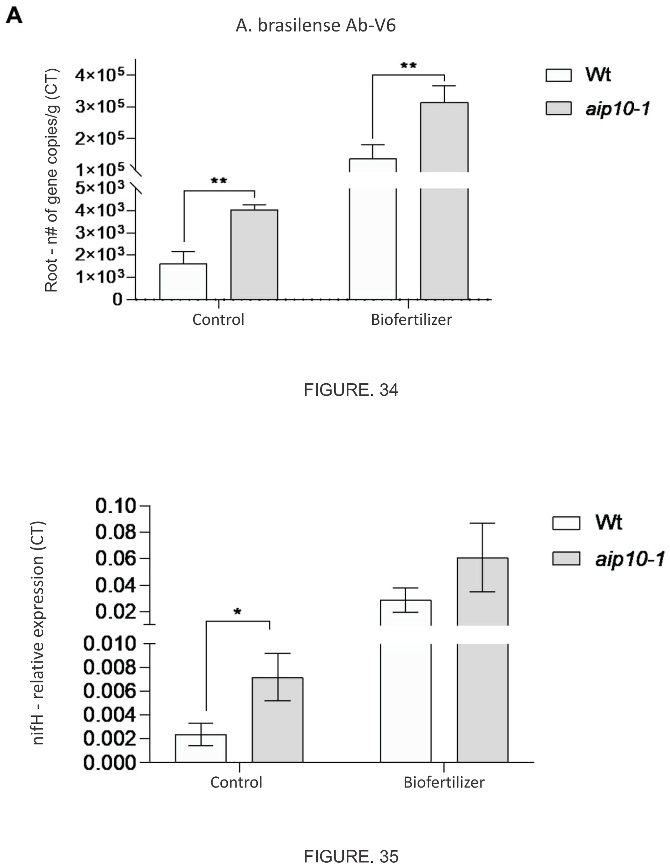

Figure 34 shows the quantification of colonization by beneficial bacteria in wild plants and mutant plants knockout in the *AIP10* gene in maize, when inoculated with bioinoculants. (A) The rate of root colonization was measured (absolute values) in DNA samples, from the amplification of bacteria-specific single-copy genes from the bioinoculant. (B) Relative quantification of a broad group of diazotrophic bacteria that was performed from RNA amplification 16S in RNA samples, by qRT-PCR. All plants were grown in soil-vermiculite substrate and collected 10 days after inoculation with Bioinoculant: *A.brasilense* Ab-V6. In yellow: WT control plants; in green: mutant plants *aip10-1.*
Figure 35 shows the mRNA levels of the *NifH* gene in WT maize plants and knockout mutants in the *AIP10* gene. The expression of the NifH gene of diazotrophic bacteria was quantified by qRT-PCR in plants collected 10 DAI. The graph shows a higher expression of bacterial *NifH* mRNA levels in uninoculated *aip10-1* mock mutant plants, and an increased expression of bacterial *NifH* in mutant plants inoculated with the bioinoculant, when compared to WT plants (control). The bars indicate mean ± standard deviation. The data were analyzed by two-way ANOVA and the means were compared by Tukey's test at 5% probability (p<0.05). Bioinoculant: *A.brasilense* Ab-V6. In yellow: WT control plants; in green: mutant plants *aip10-1.* A: biological replica.

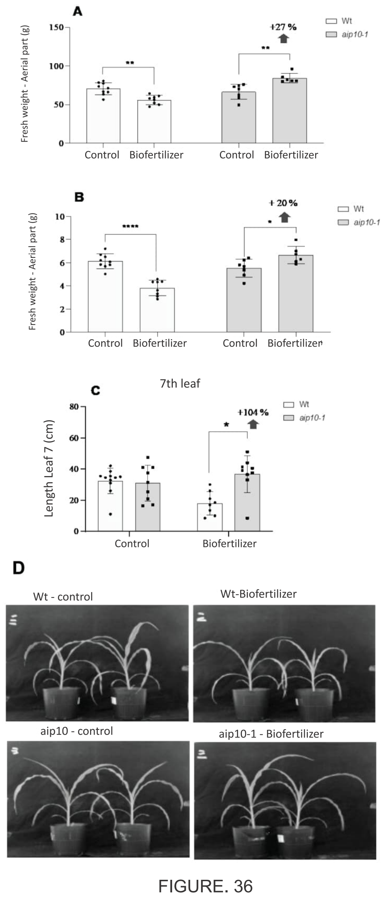

Figure 36 shows the effects of the use of bioinoculant with diazotrophic bacteria on the growth of shoots of mutant plants knockout in the *AIP10* gene in maize, obtained by gene editing (CRISPR). The graphs show phenotypic analyses performed with the edited mutant maize plants, of the *aip10-1,* line and wild plants, 20 days after germination (DAG) and 19 days after inoculation (DAI). Mutant plants with knockout in the *AIP10* gene in maize, obtained by gene editing (CRISPR), showed higher (A) shoot fresh weight; (B) shoot dry weight; (C) length of leaf 7, as a reference for development time, which can be observed through representative images of 20DAG plants and (D) 19DAI. The bars indicate mean ± standard deviation. The data were analyzed by *two-way* ANOVA and the means were compared by Tukey's test at 5% probability (p<0.05). Bioinoculant: *A.brasilense* Ab-V6. In yellow: WT control plants; in green: mutant plants *aip10-1. The percentages in which the value quantified in the mutant is greater than that quantified in wild plants are indicated in the Figure under the different conditions tested*

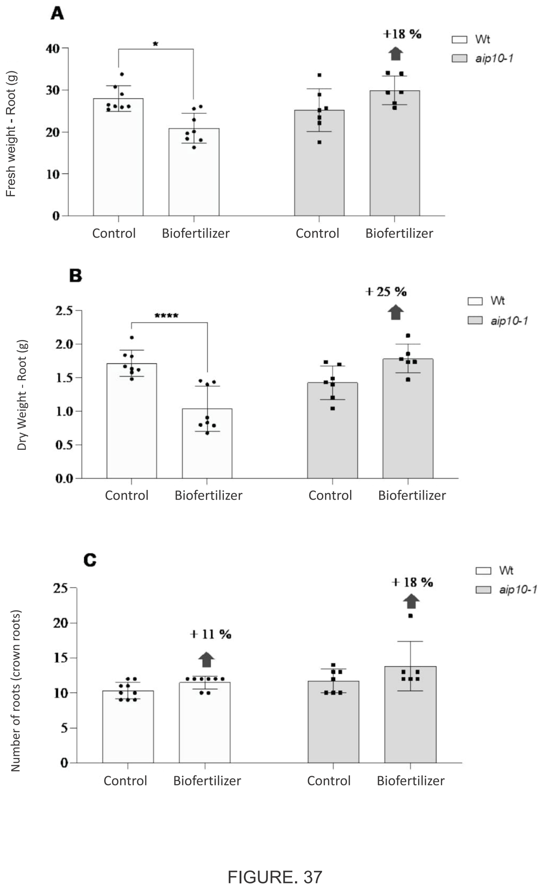

Figure 37 shows the effects of the use of bioinoculant with diazotrophic bacteria on the growth of roots of mutant knockout plants in the *AIP10* gene in maize, obtained by gene editing (CRISPR). The graphs show phenotypic analyses performed with the edited mutant maize plants, of the *aip10-1,* line and wild plants, 20 days after germination (DAG) and 19 days after inoculation (DAI). The bars indicate mean ± standard deviation. Mutant plants knocked out in the *AIP10* gene in maize, obtained by gene editing (CRISPR), showed higher (A) Fresh root weight; (B) Dry root weight; and (C) Number of roots (*crown*) as a reference to root architecture. The data were analyzed by *two-way* ANOVA and the means were compared by Tukey's test at 5% probability (p<0.05). Bioinoculant: *A.brasilense* Ab-V6. In yellow: WT control plants; in green: mutant plants *aip10-1. The percentages in which the quantified value in the mutant is greater than that quantified in wild plants are indicated in the Figure under the different conditions tested*

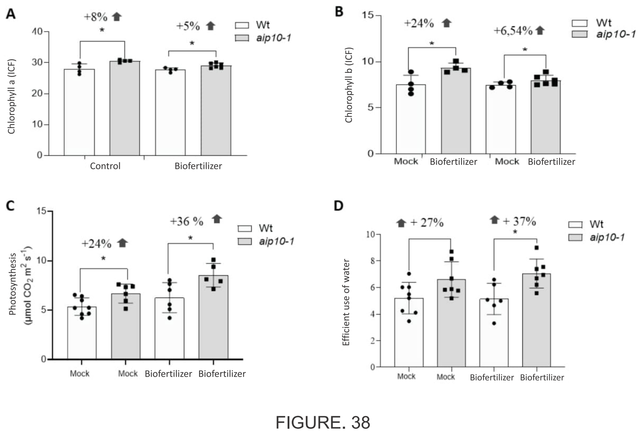

Figure 38 shows the effects of using bioinoculant with diazotrophic bacteria on the increase of chlorophyll A and B content, and on the photosynthetic capacity in mutant plants knockout in the *AIP10* gene in maize, obtained by gene editing (CRISPR). The graphs show physiological analyses performed with the edited mutant maize plants, of the *aip10-1* line, and wild plants, 45 days after germination (DAG) and 44 days after inoculation (DAI). Mutants knockout in the *AIP10* gene in maize inoculated with bioinoculant presented: (A) higher content of Chlorophyll A; (B) higher content of Chlorophyll B, chlorophyll readings were performed using the ClorofiLOG ^{®} portable chlorophyll meter; (C) higher net CO₂ assimilation rates; (D) higher water efficiency (WUE) (n= 8). The measurements were performed with WT_CRISPR and *aip10_CRISPR* plants with 25DAG with the LICOR-6400 XT instrument at 25°C in CO₂ ambient at 400µmol and 500µmol of photons m⁻²s⁻¹. The bar graphs represent means ± standard deviation and * significantly different from Wt with probability (p<0.05). Student's t-test. *The percentages in which the quantified value in the mutant is greater than that quantified in wild plants are indicated in the Figure, under the different conditions tested.*

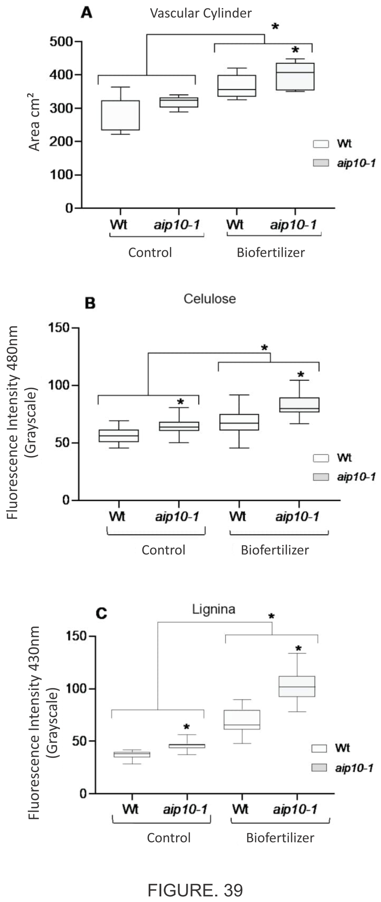

Figure 39 shows the morphological analysis and relative fluorescence of cellulose and lignin in root cross-sections of mutant plants knockout in the *AIP10* gene in maize. The graphs show phenotypic analyses performed with the edited mutant maize plants, of the *aip10-1*line, and wild plants, 10 days after germination (DAG) and 9 days after inoculation (DAI). (A) Measurements of the circular area of the vascular cylinder (stele) in transverse root sections stained with Safranin; (B) Quantification of the intensity of cellulose fluorescence at 480nm; (C) Quantification of the intensity of lignin fluorescence at 430nm. The bars indicate mean ± standard deviation. The data were analyzed by *two-way* ANOVA and the means were compared by Tukey's test at 5% probability (p<0.05). Bioinoculant: *A.brasilense* Ab-V6. In yellow: WT control plants; in green: mutant plants *aip10-1.*

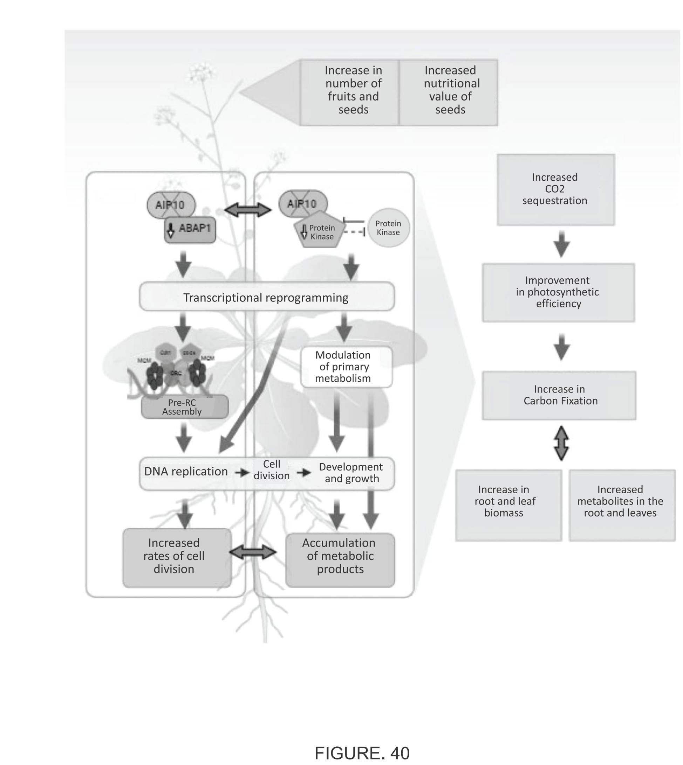

Figure 40 shows a model of mechanisms triggered by the absence of AIP10. AIP10 can act by modulating plant development through interaction with ABAP1 and kinases, key regulators of the cell cycle and primary metabolism, respectively. In the absence of AIP10, the transcriptional levels of ABAP1 decrease, preventing the inhibition of its targets, Cdt1a/b, which allows the formation of the pre-RC complex, licensing DNA replication and the progress of cells throughout the cell cycle. At the same time, the absence of AIP10 triggers a transcriptional reprogramming leading to cell division and modulating plant metabolism. These processes lead to an increase in photosynthetic efficiency and result in (i) greater carbon fixation, enhanced by the increase in the number of cells, and (ii) increased accumulation of metabolites. A combination of all these effects contributes not only to the real increase in root biomass and seed productivity, but also to the improvement of the nutritional value of the plants and to the increase in energy to sustain higher cell division rates.

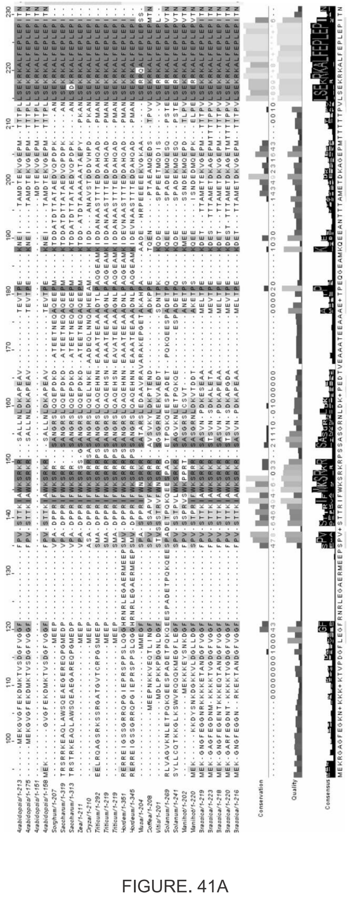

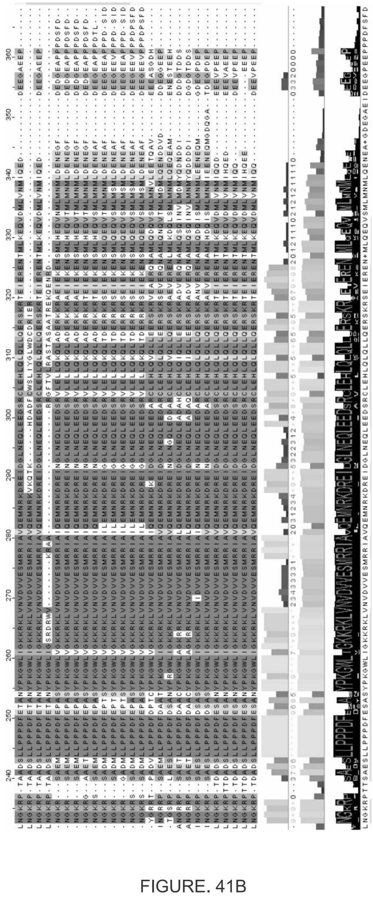

Figure 41 shows the alignment of amino acid sequences of AIP10 proteins from several plant species and the identification of a conserved sequence.

### DETAILED DESCRIPTION OF THE INVENTION

The problem in the technique of genetic improvement of agricultural plants can be defined as a need for new genes or genetic modifications that result in plants with improved agronomic characteristics without negative impact on other agronomic traits of interest.

As the person skilled in the art well knows, genetic modifications can improve some agronomic characteristics of a modified plant but worsen other agronomic characteristics due to the complexity of the plant's genome and the interactions between genes and their products.

For example, a genetic modification that increases a plant's tolerance to pests or diseases can improve its yield by reducing losses due to infection. However, this modification can also result in unintended consequences, such as reduced plant growth or decreased seed viability. Similarly, a modification that improves a plant's photosynthetic efficiency or water use efficiency can increase its biomass and yield under certain conditions, but it can also make the plant more susceptible to other stress factors, such as high temperatures.

In addition, plant traits are often interconnected and influenced by multiple genes and their products. A modification that alters the expression of one gene can affect the expression of other genes, leading to unintended consequences. For example, a modification that increases the biomass of a plant by altering the expression of a growth hormone gene can also affect the expression of genes involved in the stress response, resulting in lower stress tolerance.

Another factor that can contribute to trade-offs between different agronomic traits is the environment. The same genetic modification can have different effects on plant growth and productivity under different environmental conditions, such as temperature variations, light levels, or soil types.

In summary, genetic modifications can have both positive and negative effects on different agronomic traits of a plant, and the complexity of the plant genome and interactions between genes can make it difficult to predict the full range of effects. Environmental factors may also play a role in shaping the effects of genetic modifications on plant growth and yield.

It is necessary, therefore, not only to identify genetic modifications that result in desirable agronomic traits, but also to verify that such modification does not result in unacceptable trade-offs.

The present invention teaches genetic modifications that surprisingly result in plants with several improved agronomic traits without unacceptable trade-offs.

In the search for agronomically useful genetic modifications, the inventors of the present invention unexpectedly realized that plants that have partial or complete suppression of the *AIP10 gene* and partial suppression of the *ABAP1* gene exhibit enhanced plant traits of agronomic interest.

In a first embodiment, the present invention provides plants with a genetic modification that results in complete or partial suppression of the native *AIP10* gene.

In a second embodiment, the present invention provides plants with a genetic modification that results in partial suppression of the native *ABAP1* gene.

Plants containing a genetic modification according to the present invention exhibit enhanced plant traits of agronomic interest, including increased biomass, tolerance to water stress, photosynthetic efficiency, carbon fixation, nutritional value, resistance to pest nematodes, and responsiveness to beneficial bacteria.

A genetic modification according to the present invention is a modification that results in partial or complete suppression of the expression of the *AIP10* gene or partial suppression of the *ABAP1* native gene of the plant, which act on the same regulatory network. Thus, the modification can be, for example, a heterologous expression cassette that expresses an RNAi, miRNA, etc., or a modification in the sequence of the native gene that results in the production of reduced amounts of mRNA in relation to the native gene or expression of a less functional protein in relation to the native gene. For embodiments of the present invention involving the *AIP10* gene, a genetic modification according to the present invention includes, for example, (i) gene expression silencing cassette; (ii) a deletion of the native AIP10 gene; or (iii) a modification in the sequence of the native *AIP10* gene or its promoter, in which the modified sequence produces reduced or undetectable amounts of mRNA relative to the native *AIP10* gene or expresses a less functional protein relative to the native *AIP10* gene or a non-functional one. For embodiments of the present invention involving the *ABAP1* gene, a genetic modification according to the present invention includes, for example, (i) a gene expression silencing cassette; or (ii) a modification in the sequence of the native *ABAP1* gene or the promoter thereof, in which the modified sequence produces reduced or undetectable amounts of mRNA relative to the native *ABAP1* gene or expresses a less functional protein relative to the native ABAP1 gene.

Plants with improved plant characteristics of agronomic interest show improved growth, productivity, tolerance, and adaptability to grow under environmental stresses.

Adaptability is understood as the ability of different plant genotypes to take advantage of environmental stimuli in an advantageous way, that is, that plants are able to maintain good performance even when environmental conditions are unfavorable to the crop (Eberhart and Russel, 1966, Crop Science, Madison, v.6. p.36-40). Creating tools that can increase plant adaptability is extremely important for maintaining agricultural productivity in adverse situations.

The present invention allows for increased biomass, increased tolerance to water stress, increased photosynthetic efficiency, increased carbon fixation, increased nutritional value, increased resistance to pest nematodes, and increased responsiveness to beneficial bacteria.

To do this, the inventors altered the expression level of the *AIP10* and *ABAP1* genes, through silencing, repression, decreased expression, selective knockout of one of these genes or mutation.

### DEFINITIONS

Unless otherwise defined, all technical and scientific terms used herein have the same meaning as understood by a person skilled in the art to which the invention belongs. All technical terms used herein are terms commonly used in biochemistry, molecular biology and agriculture, and can be found, for example, in: Ausubel et al., eds. Current Protocols in Molecular Biology, John Wiley & Sons, Inc. N.Y. (1987-2008), including all supplements; Sambrook et al., Molecular Cloning: A Laboratory Manual, 2a edition, Cold Spring Harbor, N.Y. (1989); SHORT PROTOCOLS IN MOLECULAR BIOLOGY: A COMPENDIUM OF METHODS FROM CURRENT PROTOCOLS IN MOLECULAR BIOLOGY, 5a ed., vol. 1-2, ed. Ausubel et al., John Wiley & Sons, Inc. (2002); GENOME ANALYSIS: A LABORATORY MANUAL, vol. 1-2, ed. Green et a1., Cold Spring Harbor Laboratory Press, Cold Spring Harbor, N. Y., 1997. Methodologies involving plant biology techniques are described herein and are described in detail in works such as METHODS IN PLANT MOLECULAR BIOLOGY: A LABORATORY COURSE MANUAL, ed. Maliga et al., Cold Spring Harbor Laboratory Press, Cold Spring Harbor, N. Y., 1995. Several techniques using PCR are described, for example, in Innis et al. PCR PROTOCOLS: A GUIDE TO METHODS AND APPLICATIONS, Academic Press, San Diego, 1990 and in Dieffenbach and Dveksler, PCR PRIMER: A LABORATORY MANUAL, 2a ed., Cold Spring Harbor Laboratory Press, Cold Spring Harbor, N. Y., 2003.

The specification also provides definitions of terms to assist in the interpretation of what is described herein and the claims. Unless otherwise noted, all figures expressing quantities, percentages and proportions, and other numerical values used in the specification and claims, shall be understood to be modified in all cases by the term "about." Thus, unless otherwise indicated, the numerical parameters shown in the specification and claims are approximations that may vary, depending on the properties to be obtained.

It should be understood that this invention does not limit its application to the details given in the following description or illustrated in the drawings or examples. The invention is capable of other embodiments or of being practiced or carried out in various ways. In addition, it should be understood that the terminology employed herein is for the purposes of description and should not be considered as limiting.

For the purposes of this invention, the expression "completely inactive gene allele" means a gene allele containing a genetic modification in accordance with the present invention that results in complete suppression of mRNA production by said gene or production of a protein with completely eliminated activity.

For the purposes of this invention, the expression "partially inactive gene allele" means a gene allele containing a genetic modification under the present invention that results in partial suppression of mRNA production by said gene or production of a protein with partially eliminated activity.

As will be well understood by the person skilled in the art, a plant can have one or more alleles of each gene that makes up its genome. A "homozygous" plant or genome for a given allele is a plant or genome in which all alleles for a given gene are the same. A "heterozygous" plant or genome for a given allele is a plant or genome in which at least one of the alleles for a given gene is different from the other alleles of the same gene.

For the purposes of this invention, the expressions "beneficial bacteria", "plant growth promoting bacteria (BPCV)" and "bioinoculants" are used interchangeably and are defined as bacteria capable of enhancing the growth of plants with which they interact. Growth-promoting bacteria preferably include nitrogen-fixing, nutrient-supplying, phytohormone-producing, abiotic-stress-reducing, and/or pathogen- and pest-controlling bacteria. In a preferred embodiment, the beneficial bacterium is from a selected genus of the group consisting of *Acetobacter, Agrobacterium, Azospirillum, Bacillus, Bradyrhizobium, Burkholderia, Citrobacter, Enterobacter, Gluconacetobacter, Grimontella, Herbaspirillum, Klebsiella, Mesorhizobium, Mycobacterium, Nitrospirillum, Paenibacillus, Pantoea, Pseudomonas, Rahnella, Rhizobium, Serratia and Streptomyces.* In preferred embodiments, the beneficial bacterium is *Azospirillum brasilense, Azospirillum baldaniorum, Azospirillum lipoferum, Azotobacter chroococcum, Bacillus amyloliquefaciens, Bacillus megaterium, Bacillus phytofirmans, Bacillus polymyxa, Bacillus subtilis, Bradyrhizobium* spp, *Burkholderia* spp, *Burkholderia ambifaria, Citrobacter* spp, *Gluconacetobacter diazotrophicus, Grimontella* spp, *H. seropedicae, Klebsiella* spp, *Mesorhizobium ciceri, Mycobacterium phl, Nitrospirillum amazonense, Paenibacillus* sp, *Pantoea* spp, *Pseudomona putida, Pseudomonas aeruginosa, Pseudomonas alcaligenes, Pseudomonas fluorescens, Pseudomonas jessenii, Rahnella* spp, *Rhizobium* spp, and *Serratia* sp. In more preferred implementations, the CVPGs are of strains that include *A. brasilense* Ab-V6, *A. brasilense* Ab-V5, *A. baldaniorum* SP245, G. *diazotrophicus* PAL5, *H. seropedicae* ZAE, and *H. seropedicae* HRC54. Preferably, for the purposes of this invention, beneficial bacteria are diazotrophic (nitrogen-fixing) bacteria. Illustratively and not limited to, diazotrophic bacteria include those of the genus *Rhizobium, Frankia, Azospirillum, Azotobacter, Herbaspirillum, Burkholderia, Gluconacetobacter, Bacillus, Pseudomonas, Klebsiella.*

The term "enhanced agronomic traits" refers to plant traits of agronomic interest in plants used for cultivation that are increased or improved compared to wild or commercial variety plants that do not have a genetic modification according to the present invention. Traits include, but are not limited to, biomass production by the plant, tolerance to water stress, photosynthetic efficiency, carbon fixation, nutritional value, resistance to pest nematodes, and responsiveness to beneficial bacteria.

The term "encode" refers to the process by which a gene, through the mechanisms of transcription and translation, provides information to a cell from which a series of amino acids can be arranged into a specific sequence of amino acids to produce an active enzyme. Due to the degeneration of the genetic code, certain base changes in the DNA sequence do not alter the amino acid sequence in the protein. Therefore, it is understood that modifications in the DNA sequences encoding the proteins of the invention, which do not substantially affect their functional properties, are contemplated.

"Expression cassette" refers to a sequence of nucleotides that contains enough elements to result in transcription to RNA when inserted into a genome or cell, such as a plant cell. "Expression silencing cassette" refers to an expression cassette whose expression product results in silencing of a gene native to the cell, including, for example, RNAi expression cassettes, and micro RNA expression cassettes.

The term "stringent conditions" here refers to parameters with which the technique is familiar. Single-stranded polynucleotides hybridize when they become associated based on a variety of well-characterized physicochemical forces, such as hydrogen bonding, solvent exclusion, and base stacking. The stringency of a hybridization reflects the degree of identity of the sequence of the nucleic acids involved, so that the greater the stringency, the more similar the two polynucleotide strands are. Stringency is influenced by a variety of factors, including temperature, concentration and composition of salts, organic and inorganic additives, solvents, etc. present in both hybridization and washing solutions as well as incubations (and number). A person with ordinary knowledge of the technique can readily select such conditions by varying the temperature during the hybridization reaction and the washing process, the salt concentration during the hybridization reaction and the washing process, and so on.

For hybridization of complementary nucleic acids that have more than 100 complementary residues, in a Southern or Northern blot filter, the "stringent" hybridization conditions are exemplified by a temperature that is about 5°C to 20°C lower than the thermal melting point (Tm) for the specific sequence, at a defined ionic resistance and pH. Tm is the temperature under defined ionic resistance and pH, where 50% of the target sequence hybridizes to a perfectly matched probe. Nucleic acid molecules that hybridize under stringent conditions will typically hybridize to a probe based on either the entire cDNA or selected portions. More preferably, "stringent conditions" refer herein to the parameters with which the technique is familiar, such as hybridization at 3.3 x SSC, 1 x Denhardt's solution, 25mM sodium phosphate buffer (pH 7.0), 0.5% SDS, and 2mM EDTA, for 18 hours at 65° C, followed by four filter washes at 65⁰C for 20 minutes, in 2 x SSC and 0.1% SDS, and a final wash for up to 20 minutes in 0.5 x SSC and 0.1% SDS or 0.3 x SSC and 0.1% SDS for higher stringency, and 0.1 x SSC and 0.1% SDS for even higher stringency. Other conditions can be substituted as long as the degree of stringency is the same as that provided herein, using a fine wash with 0.5 x SSC. For identification of less closely related homologues there can be performed at a lower temperature, for example, 50%. In general, the stringency is increased by raising the washing temperature or decreasing the concentration of SSC.

In this description, the term "expression" refers to the production of the protein product encoded by a gene. *"Downregulation"* refers to the production of a gene product in genetically modified organisms that is below the production levels in normal or non-modified organisms. "Silencing" is the regulation of gene expression in a cell such that the expression of a certain gene is diminished but not completely eliminated. Finally, when a gene is knocked out, its expression is completely eliminated from the body. These changes in the level of expression can be achieved, for example and in a non-limiting way, through anti-sense RNA, RNAi, artificial microRNA, T-DNA insertion, transposons, among others. More recently, methods using TALENs, ZFNs, CRISPR/Cas9, etc., have been applied to alter only the target genes. For example, the CRISPR/Cas9 system has a very high target specificity and can recognize a target DNA, so it can be applied to a wide variety of genes and is passed on to descendants according to Mendel's genetic law, allowing generational fixation of *traits* (characters).

The term "photosynthetic efficiency" refers to the efficiency of the plant in converting light energy into chemical energy and/or plant biomass in the process of photosynthesis.

The term "carbon fixation" refers to biological carbon fixation, or carbon assimilation by plants. It is the process by which living organisms, such as plants, convert inorganic carbon, particularly carbon dioxide, into organic compounds. These organic compounds are then used to store energy and as structures for other biomolecules that make up plant biomass.

The term "biomass production" refers to the ability of plants to produce plant biomass in a given period of time. Plant biomass can be from any plant tissue or organ including, but not limited to, roots, stems, leaves, flowers, fruits, and seeds, which is usually measured by the dry weight of the plant.

The term "water stress tolerance" refers to drought tolerance and the ability by which a plant maintains its biomass production during arid or dry conditions or how quickly plants adapt to or recover from a period of drought.

The term "nutritional value" refers to the concentration of essential nutrients including carbohydrates, fats, proteins, minerals, and vitamins in plant biomass used as human or animal food, such as leaves, fruits, and seeds.

The term "gene" refers to a nucleotide sequence or genomic segment capable of being transcribed into RNA. A gene includes all the elements necessary for gene expression, including promoters, enhancers, introns, exons, and terminators.

The term "native *AIP10* gene" is used in this document to describe a plant gene that encodes a protein comprising an amino acid sequence with at least 90%, 91%, 92%, 93%, 94%, 95%, 96%, 97%, 98%, 99%, 100% sequence identity with an amino acid sequence selected from the group consisting of SEQ ID NO: 2, 3, 4, 5, 9, 12, 13, 15, 17, 21, 22, 23, 25, 26, 28, 30, 32, 34, 36, 39, 40, 45, 46, 47, 48, 51, 52, 57, 58, 59, 60, 63, 64, 66, 69, 70, 72, 75, 76, 78, 81, 82, 84, 85, 86, 89, 90, 92, 94, 96, 98, 99, 101, 104, 105, 107, and 263 and that occurs naturally in a plant species. In certain embodiments described herein, the *AIP10* gene is a gene that has at least 80%, 81%, 82%, 83%, 84%, 85%, 86%, 87%, 88%, 89%, 90%, 91%, 92%, 93%, 94%, 95%, 96%, 97%, 98%, 99%, 100% sequence identity with a nucleotide sequence selected from the group consisting of SEQ ID NO: 1, 8, 10, 11, 14, 16, 18, 19, 20, 24, 27, 29, 31, 33, 35, 37, 38, 41, 42, 43, 44, 49, 50, 53, 54, 55, 56, 61, 62, 65, 67, 68, 71, 73, 74, 77, 79, 80, 83, 87, 88, 91, 93, 95, 97, 100, 102, 103, and 106 and that occurs naturally in a plant species.

The term "native *ABAP1* gene" is used in this document to describe a plant gene that encodes a protein comprising an amino acid sequence with at least 90%, 91%, 92%, 93%, 94%, 95%, 96%, 97%, 98%, 99%, 100% sequence identity with an amino acid sequence selected from the group consisting of SEQ ID NO: 7, 109, 111, 114, 115, 117, 121, 122, 123, 125, 127, 129, 132, 133, 136, 137, 140, 141, 142, 146, 147, 148, 155, 156, 157, 158, 159, 160, 161, 164, 165, 171, 172, 173, 174, 175, 180, 181, 182, 183, 188, 189, 190, and 191, 254, 198, 199, 200, 201, 202, 206, 207, 208, 210, 213, 214, 217, 218, 219, 220, 223, 224, 227, 228, 231, 232, 235, 236, 239, 240, 241, 242, 243, 246, 247, 248, 251, 252 and that occurs naturally in a plant species. In certain embodiments described herein, the *ABAP1* gene is a gene that has at least 80%, 81%, 82%, 83%, 84%, 85%, 86%, 87%, 88%, 89%, 90%, 91%, 92%, 93%, 94%, 95%, 96%, 97%, 98%, 99%, 100% sequence identity with a nucleotide sequence selected from the group consisting of SEQ ID NO: 6, 108, 110, 112, 113, 116, 118, 119, 120, 124, 126, 128, 130, 131, 134, 135, 138, 139, 143, 144, 145, 149, 150, 151, 253, 152, 153, 154, 162, 163, 166, 167, 168, 169, 170, 176, 177, 178, 179, 184, 185, 186, 187, 192, 193, 194, 195, 196, 197, 203, 204, 205, 209, 211, 212, 215, 216, 221, 222, 225, 226, 229, 230, 233, 234, 237, 238, 244, 245, 249 and 250 and that occurs naturally in a plant species.

In this description, a "genetic modification" refers to any change in a sequence of a gene, including deletion, insertion, or replacement of nucleotides. The alteration can occur in any element of a gene, including coding and non-coding elements, such as promoter, enhancer, intron, exon, and terminator.

In this description, the term "introduction of a genetic modification" refers to gene editing, random or targeted mutagenesis, or genetic transformation to insert exogenous DNA into a plant, as well as introgression by sexual crossing that results in a plant containing the aforementioned genetic modification in its genome.

For the purposes of this invention, the expression "pest nematode" means a parasitic nematode of agricultural plants that causes losses to agricultural crops. Pest nematode genera include *Aphelenchoides, Ditylenchus, Globodera, Heterodera, Longidorus, Meloidogyne, Nacobbus, Pratylenchus, Radopholus, Rotylenchulus, Trichodorus, Tylenchulus* and *Xiphinema.* Examples of pest nematodes include root-knot nematode *(Meloidogyne* spp.), root-lesion nematode *(Pratylenchus brachyurus),* cyst nematode *(Heterodera glycines),* green stem nematode *(Aphelenchoides besseyi)* the root-lesion nematode *(Pratylenchus brachyurus)* and the reniform nematode *(Rotylenculus reniformis*). Examples of pest nematode species include *Meloidogyne javanica, Meloidogyne incognita,* and *Pratylenchus zeae.*

The term "crop plant" includes plants that are grown in an agricultural environment for subsistence purposes or marketing of plant products, such as seeds, grains, cereals, flakes, bran, flour, syrup, oil, juices, processed parts for food or animal feed, biomass and fuels. Crop plants include food crops, fodder crops, fiber crops, oil crops, ornamental crops, and industrial crops. Cultivated plants include monocotyledons and dicotyledons. Examples of monocotyledonous crop plants include grasses (Poaceae), including oats, rice, sugar cane, rye, barley, maize, miscantus, sorghum, and wheat, as well as banana and palm trees. Examples of dicotyledonous crop plants include cotton, lettuce, potato, sugar beet, cocoa, coffee, canola, citrus, eucalyptus, vegetables, cassava, castor bean, eucalyptus, pea, beans, papaya, castor bean, mustard, poplar, soybean, tomato, and grape.

The term "responsiveness to beneficial bacteria" refers to the ability of plants to take advantage of forming an association with a beneficial bacterium. A plant with responsiveness to superior beneficial bacteria has an agronomic trait of superior interest when inoculated with beneficial bacteria relative to a plant with inferior responsiveness to beneficial bacteria. In the present invention, it was surprisingly found that plants silenced in certain target genes, associated with the action of beneficial bacteria, applied in the form of a bioinoculant, show improved results regarding biomass increase, including organs such as leaves, stem, and root, which consequently show a significant increase in the production of fruits and seeds. These genetically modified plants, associated with the bioinoculant, also show better performance in situations of water deficit (tolerance to water stress) and a reduced need for the use of nitrogen chemical fertilizers.

The term "partial gene suppression" means that the production of a gene product in a genetically modified organism is below the production levels in normal or non-modified organisms, but not completely eliminated, or that, even if the production of a gene product is not altered, its activity is reduced but not completely eliminated. The term "complete gene suppression" means that the expression of a gene product in a genetically modified organism is completely eliminated or that, even if the production of a gene product is not completely eliminated, its activity is completely eliminated. Preferably, complete gene suppression is the result of a modification in the nucleotide sequence of the plant's native gene resulting in a nucleotide sequence that does not produce detectable mRNA or that expresses a non-functional protein. Detection of mRNA can be performed using known assays of the technique, such as, but not limited to, northern blotting, reverse transcription polymerase chain reaction (RT-PCR), hybridization *in situ,* RNA sequencing (RNA-seq), microarray analysis, fluorescent hybridization *in situ* (FISH), digital drop PCR (ddPCR), next generation sequencing (NGS), hybridization *in situ* single molecule fluorescent (smFISH), rolling circle amplification (RCA), hybridization chain reaction (HCR). Modifications in the nucleotide sequence of the plant's native gene resulting in expression of a non-functional protein include, for example, a change in the reading frame in the coding region or an amino acid deletion that results in a non-functional protein, which may be by means of the interruption in protein production or a change in conformation causing it to no longer function as it should or not function at all.

Techniques for suppressing the expression of a native plant gene are well known from the state of the art and include random and targeted mutagenesis techniques. There are several techniques for introducing mutations that can be used to completely or partially suppress the expression of a gene native to a plant, including: chemical mutagenesis, radiation mutagenesis, transposon mutagenesis, insertion of T-DNA by transformation with a bacterium capable of transferring DNA to plants. In addition, any genome editing technique can be utilized, including transcription activator-like effector nucleases (TALENs), zinc finger nucleases (ZFNs), RNA-guided Fokl nucleases, homing endonucleases, CRISPR-Cas9, CRISPR-Cas12a(Cpf1). Any technique capable of completely suppressing the expression of the *AIP10* gene native to a plant can be used to produce a plant according to the present invention.

Chemical methods of mutagenesis include exposing DNA to a chemical mutagen, for example, ethyl methanesulfonate (EMS), methyl methanesulfonate (MMS), N-nitrosourea (EN U), N-methyl-N-nitro-N'-nitrosoguanidine, 4-N-oxide of nitroquinoline, diethyl sulfate, benzopyrene, cyclophosphamide, bleomycin, triethylmelamine, acrylamide monomer, nitrogen mustard, vincristine, diepoxyalkanes (for example, diepoxybutane), ICR-170, formaldehyde, procarbazine hydrochloride, ethylene oxide, dimethylnitrosamine, 7,12 dimethylbenz(a) anthracene, chlorambucil, hexamethylphosphoramide, bisulfane and the like.

Radiation-inducing mutation agents include ultraviolet radiation, y-radiation, X-rays, and fast neutron bombardment. US2015106970, incorporated herein by reference, teaches the use of gamma radiation in the introduction of mutations into the genome of plants.

Large-scale production of mutant plants containing total or partial suppression of genes by insertion of T-DNA and transposons is taught, for example, in WO1999012411A2, incorporated herein by reference. Bacteria known to transfer segments of DNA into plant cells include *Agrobacterium tumefaciens, Agrobacterium rhizogenes, Rhizobium* spp., *Pseudomonas* spp., and *Sinorhizobium* spp. For example, *Agrobacterium* -mediated transformation is described, for instance, in documents US 8,404,930 (corn), US 2009/0142837 (maize), WO2011095460, US 2009/0138985 (soybean), US 2008/0280361 (soybean), WO2000071733 (cotton), and US 2008/0256667 (cotton). Examples of plant transformation with other bacteria can be found in WO2007137075A2, incorporated herein by reference.

In this description, the term *"AIP10* and/or *ABAP1* polynucleotide sequences" denotes any nucleic acid, gene, DNA, RNA, mRNA, or cDNA molecule that encodes an AIP10 and ABAP1 polypeptide, or their splicing variants, whose down-regulation, silencing, or knockout alters, improves, or optimizes the association of the genetically modified plant with beneficial bacteria, compared to wild plants. DNA or RNA can be double-stranded or single-stranded. Single-stranded DNA can be the coding strand, also known as the sense strand, or it can be the non-coding strand, also called the anti-sense strand. Illustrative of this category are polynucleotide molecules comprising the sequences described in the "brief description of sequences" section.

The term *"alternative splicing"* in the context of the invention refers to: inclusion of intron, exclusion of exon, addition or deletion of terminal sequences in the variant when compared to the original sequence, as well as the possibility of "intron retention". Intron retention is an intermediate stage in the processing of RNA transcripts, whereby before the production of fully processed mRNA, the intron (naturally removed in the original sequence) is retained in the variant.

The term "heterologous nucleic acid" refers to a nucleic acid, DNA or RNA, that has been introduced into a cell (or the cell's ancestor) through the efforts of humans. Such an exogenous nucleic acid may be a copy of a sequence that is naturally found in the cell or its complementary molecule into which it was introduced, or fragments thereof.

The term "homologous sequences" or "functional homologues" refers to polynucleotide or polypeptide sequences that are similar due to ancestry and conservation of common sequences and have identical or similar function at the catalytic, cellular, or organismal levels.

The sequences of the present invention can be identified from a myriad of organisms. Although the nucleotide sequences mentioned above are described herein, they should not be considered as limitations of the present invention. Thus, a sequence of *aip10* or *abap1* can be identified and functionally annotated by sequence comparison. A person skilled in the art can readily identify a sequence of *AIP10* or *ABAP1* in a suitable database, such as GenBank, using publicly available sequence analysis programs and parameters. Alternatively, selection of cDNA libraries or genomic libraries employing suitable hybridization probes or primers based on DNA or protein sequences described herein should lead to the identification of functionally related *AIP10* or *ABAP1* sequences (functional homologue). It is also appreciated in the field that sequences with reduced levels of identity can also be isolated with the aid of degenerate oligonucleotides and PCR-based methodology.

A plant with complete or partial suppression of the *AIP10* gene, or partial suppression of the *ABAP1* gene, according to the present invention can be produced by applying large-scale random mutagenesis techniques and subsequent selection of mutants in the gene and isolation of the specific mutation by backcrossing.

Plant genome editing using zinc finger nucleases (ZFNs), homing endonucleases, transcription activator-like effector nucleases (TALENs), and CRISPR-Cas9 is taught, for example in the document WO2016116032A1, incorporated herein by reference.

Techniques for editing plant genomes using the CRISPR/Cas9 system are taught, for example, in documents WO2013176772A2 and US8945839B2, incorporated herein by reference.

Plant genome editing using the CRISPR-Cas12a (Cpf1) system is taught, for example, in WO2017218185A1, incorporated herein by reference.

This document also provides methods for producing plants with improved agronomic characteristics through crossbreeding. Plants with improved agronomic characteristics can be produced through the introgression of a genetic modification according to the present invention in other varieties. Thus, a genetic modification according to the present invention can be combined with other agronomic traits of interest, such as transgenic events containing herbicide tolerance genes, insecticidal protein genes, and genes that confer yield or stress tolerance traits, and the like.

A method of producing plants with enhanced agronomic traits by means of crossbreeding comprises the crossing of a plant comprising a genetic modification according to the present invention with a second plant with expression to produce a progeny of plants with enhanced agronomic trait. Preferably, backcrossing or self-fertilization of the progeny is carried out to produce a new generation of homozygous plants for a genetic modification according to the present invention.

Screening of plants for partial or complete suppression of the *AIP10* gene or partial suppression of the *ABAP1* gene can be performed by any known detectable mRNA analysis technique, using known detection assays of the technique, such as, but not limited to, *northern blotting,* reverse transcription polymerase chain reaction (qRT-PCR), *in situ* hybridization, RNA sequencing (RNA-seq), *microarray* analysis, *in situ* fluorescent hybridization (FISH), digital droplet PCR (ddPCR), next-generation sequencing (NGS), *in situ* single-molecule fluorescent hybridization (smFISH), rolling circle amplification (RCA), hybridization chain reaction (HCR).

Screening of plants for suppression of a gene at the functional protein level can also be performed by any known technique of DNA sequencing in the gene region or whole genomic DNA sequencing; or by protein detection analyses using specific antibodies, such as western blot, elisa, immunolocalization.

Processed plant products produced from plants in accordance with this invention are also covered in this application. In certain embodiments, the processed product is selected from the group consisting of plant parts, plant biomass, oil, flour, sugar, animal feed, bran, flakes, husks, processed seeds, and seeds. In certain embodiments, the processed product is not regenerable in a plant. The plant product may comprise commodities or other consumer goods products derived from a plant or a part of a plant, in which the consumer goods product or other products can be traced through trade by detecting nucleotide or RNA segments specific to a genetic modification according to the present invention.

As described in the Examples, by means of a broad study of different types of partial and complete suppression of a gene native to the AIP10/ABAP1 pathway in plants, it was surprisingly verified that plants with improved agronomic characteristics can be obtained.

The amino acid sequences of the AIP10 and ABAP1 proteins and coding genes *AIP10* and *ABAP1* that have a substantial percentage of identity with the sequences described in this document can be identified using several computer-based algorithms known in the technique. For example, sequences can be used to search databases using sequence alignment algorithms such as BLAST (Altschul SF, Gish W, Miller W, Myers EW, Lipman DJ. Basic local alignment search tool. J Mol Biol. 1990 Oct 5; 215(3):403-10. Doi: 10.1016/S0022-2836(05)80360-2. PMID: 2231712) to identify nucleotide and/or amino acid sequences that are closely related. The amino acid sequence identities of proteins related to AIP10 and ABAP1 or nucleotide sequences of genes *AIP10* and *ABAP1* can be analyzed using a Clustal W alignment with standard parameters: Weight matrix: blosum, gap opening penalty: 10,0. Gap extension penalty: 0.05, hydrophilic leaks: On, hydrophilic residues: GPSNDQERK, specific residue leakage penalties: On (Thompson, et al (1994) Nucleic Acids Research, 22: 4673-4680). The percentage of amino acid identity is further calculated as the product of 100% multiplied by (amino acid identities/length of the protein in question). Other alignment algorithms are also available in the technique and provide results similar to those obtained using a Clustal W alignment. In addition, AIP10 and ABAP1 proteins can be used to produce antibodies that bind specifically to related proteins and can be used to search for and find other proteins that are closely related.

In addition, the nucleotide sequences of the *AIP10* and *ABAP1* genes disclosed herein, or fragments thereof, may be used as probes and primers for screening to identify other members of the class using hybridization and isothermal amplification or thermal cycling methods. For example, oligonucleotides derived from sequences of any of SEQ ID NO can be used: 1, 8, 10, 11, 14, 16, 18, 19, 20, 24, 27, 29, 31, 33, 35, 37, 38, 41, 42, 43, 44, 49, 50, 53, 54, 55, 56, 61, 62, 65, 67, 68, 71, 73, 74, 77, 79, 80, 83, 87, 88, 91, 93, 95, 97, 100, 102, 103 and 106 (*AIP10*) and any of SEQ ID NO: 6, 108, 110, 112, 113, 116, 118, 119, 120, 124, 126, 128, 130, 131, 134, 135, 138, 139, 143, 144, 145, 149, 150, 151, 253, 152, 153, 154, 162, 163, 166, 167, 168, 169, 170, 176, 177, 178, 179, 184, 185, 186, 187, 192, 193, 194, 195, 196, 197, 203, 204, 205, 209, 211, 212, 215, 216, 221, 222, 225, 226, 229, 230, 233, 234, 237, 238, 244, 245, 249 and 250 (*ABAP1*) to determine the presence or absence of a genetic modification according to the present invention in a sample of deoxyribonucleic acid derived from a plant product, such as a commercial product. Given the sensitivity of certain nucleic acid detection methods utilizing oligonucleotides, it is anticipated that oligonucleotides derived from sequences as presented in any of the SEQ ID NO: 1, 8, 10, 11, 14, 16, 18, 19, 20, 24, 27, 29, 31, 33, 35, 37, 38, 41, 42, 43, 44, 49, 50, 53, 54, 55, 56, 61, 62, 65, 67, 68, 71, 73, 74, 77, 79, 80, 83, 87, 88, 91, 93, 95, 97, 100, 102, 103 and 106 (*AIP10*) and any of the SEQ ID NO: 6, 108, 110, 112, 113, 116, 118, 119, 120, 124, 126, 128, 130, 131, 134, 135, 138, 139, 143, 144, 145, 149, 150, 151, 253, 152, 153, 154, 162, 163, 166, 167, 168, 169, 170, 176, 177, 178, 179, 184, 185, 186, 187, 192, 193, 194, 195, 196, 197, 203, 204, 205, 209, 211, 212, 215, 216, 221, 222, 225, 226, 229, 230, 233, 234, 237, 238, 244, 245, 249, and 250 (*ABAP1*) can be used to detect a nucleic acid from a genetic modification according to the present invention.

Additionally, the category of suitable *AIP10 and ABAP1* sequences includes a nucleic acid molecule as taught in this document with one or more bases deleted, substituted, inserted, or added, the variant of which encodes a polypeptide that, when totally or partially suppressed, results in the alteration, improvement, or optimization of an agronomic characteristic of the plant compared to wild plants. The "base sequences with one or more bases deleted, replaced, inserted, or added" referred to herein are widely known to those skilled in the art for retaining physiological activity even when the amino acid sequence of a protein usually having this physiological activity has one or more amino acids replaced, deleted, inserted, or added. For example, the poly A tail or the untranslated regions of the 5' or 3' ends can be deleted, and the bases can be deleted as long as the amino acids are deleted. The bases can also be replaced as long as it does not result in displacement of the structure. Bases can also be "added" as long as amino acids are added. However, it is essential that any such modification does not result in the loss of physiological activity. A modified DNA in this context can be obtained by modifying the DNA base sequences of the invention so that amino acids at specific sites are replaced, deleted, inserted, or added by site-specific mutagenesis, for example, Zoller & Smith, 1982, Nucleic Acid Res. 10: 6487-6500. Therefore, the term "variant" is a sequence of nucleotides or amino acids that deviates from the pattern or given sequence of nucleotides or amino acids of a particular gene or protein. The variant may have "conservative" changes, wherein a substituted amino acid has similar structural or chemical properties, for example, replacement of leucine by isoleucine. A variant because it has "non-conservative" alterations, for example, replacement of a glycine by a tryptophan. Minor analogous variations may also include deletions or insertions of amino acids, or both. Guidance whose amino acid residues can be replaced, inserted or deleted can be found using well-known computer programs of the technique such as Vector NTI Suite software (InforMax, MD).

Another way to obtain a DNA sequence of *AIP10* or *ABAP1* is to synthesize them *ab initio* from the appropriate bases, for example, by using the appropriate cDNA sequence as a template.

### Nucleic Acid Constructs

The present invention includes recombinant constructs comprising a polynucleotide sequence of the *AIP10* or *ABAP1* gene or a fragment of at least 10, 20, 50, or 100 nucleotides thereof, in antisense orientation. Such sequences are operationally linked to a heterologous promoter. Constructs typically comprise a vector, such as a plasmid, a cosmid, a phage, a virus (for example, a plant virus), an artificial bacterial chromosome (BAC), an artificial yeast chromosome (YAC), or similar, into which a sequence of nucleic acids has been inserted, in a reverse orientation (reverse), in the case of insertion of antisense sequences, or not, when there is, for example, insertion of T-DNA with interruption of the *AIP10* or *ABAP1* genes, or of their 5'UTR or 3' UTR portions, and complete (knockout, ko) or partial (knockdown, kd) suppression of the genes may occur. Several suitable vectors are known and are commercially available.

Recombinant nucleic acid constructs can be made using standard techniques. Typically, plant transformation vectors include one or more cloned coding sequences (genomic or cDNA) under the transcriptional control of 5' and 3' regulatory sequences and a selectable marker. Such plant transformation vectors also typically contain a promoter, a transcription initiation starting site, an RNA processing signal (such as union signal sequences), a transcription termination site or a polyadenylation signal. *Enhancers* and targeting sequences may also be present.

The invention provides nucleic acid molecules that lead to an improved agronomic trait of interest of the genetically modified plant when compared to wild plants. An important aspect of the present invention is the use of nucleic acid constructs, in which nucleotide sequences encoding *AIP10* or *ABAP1* modified, with deleted portions, or in antisense orientation, or non-coding sequences of said genes, such as modified 5'UTR or 3'UTR portions or with deletions, are operably linked to one or more promoters, which control the expression of the coding sequence in a constitutive manner or in certain types of cells, organs, or tissues in order to alter, improve, or optimize the association of a modified plant with beneficial bacteria compared to a non-modified plant.

Suitable constitutive plant promoters, which may be useful for expressing the sequences or their fragments of *AIP10* or *ABAP1* include, but are not limited to, the 35S promoter from cauliflower mosaic virus (CaMV), polyubiquitin promoters, which confer high-level constitutive expression in most plant tissues (see, for example, WO 2007/00611, US Patent 5,510,474; Odell et al., Mature, 1985, 313: 810-812); the promoter of nopalin synthase (An et al. 1988, Plant Physiol. 88: 547-552); the FMV promoter of the scrophularia mosaic virus (US Patent 5,378,619) and the octopine synthase promoter (Fromm et al., Plant Cell 1: 977-984).

The promoter can also be selected so that expression occurs at a determined time point in plant development, or at a time point determined by external influences, or in a specific or preferred tissue mode. For example, it can have specific expression in roots.

According to one aspect of the invention, mutations can be induced in specific and relevant regions. There are four main classes of nucleases that can be employed in new gene editing technologies, namely: meganucleases, zinc-finger nucleases (ZFNs); transcription activator-like effector nucleases (TALENs); and Clustered Regularly Interspaced Short Palindromic Repeats (CRISPR)-associated (Cas) (GAJ, T.; SIRK, S. J.; SHUI, S. L.; LIU, J. Genome-editing technologies: principles and applications. Cold Spring Harb Perspectives in Biology, v. 8, n. 12, p. a023754, Dec. 2016). Among the techniques that have made genome editing more accessible and revolutionary, the CRISPR/Cas system stands out. This is due to the simplicity, low cost, and high efficiency of the technique compared to other editing tools. The CRISPR system is based on inducing mutations in DNA through double-strand cuts, in an extremely specific and targeted way. These cuts are promoted by endonucleases of the Cas family and directed by the so-called guide RNAs (gRNA). They will be repaired by one of the two main repair pathways that the cell presents when the double strand is damaged: homologous recombination (HR) or non-homologous end joining (NHEJ) (Molinari, Hugo & Rios, Leticia & Volpi e Silva, Nathalia & Souza Prado, Guilherme & Hernandes-Lopes, José. (2020). CRISPR technology in plant genome editing: biotechnology applied to agriculture). According to another aspect of the invention, it is also possible to use an antisense sequence of *AIP10* or *ABAP1* and incorporate it into a nucleic acid construct suitable for plant transformation. Therefore, nucleic acid constructs are provided comprising an antisense sequence of *AIP10 or ABAP1* under the control of an operative transcriptional initiation region in a plant, so that the construct can generate RNA in a host plant cell. Preferably, the transcriptional initiation region is part of a root promoter or root preferred. Such nucleic acid construct can be used to modify the gene expression *AIP10* or *ABAP1* in plants, as described above.

According to another aspect of the invention, it is also possible to insert genes of interest into a plant using bacteria of the genus *Agrobacterium.* These bacteria are phytopathogens with the natural ability to transfer DNA (T-DNA) to some species of dicotyledons, inducing the formation of a tumor known as crown gall or hairy root syndrome. Genes of interest can be introduced into the plant through binding to the disarmed region of the T-DNA in substitution of the original T-DNA region (ANDRADE, SRM. Plant Transformation. Planaltina, DF, 2003).

Expression vectors may also contain a selection marker by means of which transformed cells can be identified in the culture. The marker can be associated with the nucleic acid molecule, that is, the gene operably linked to a promoter. As used herein, the term "marker" refers to a gene that encodes a trait or a phenotype that allows for the selection of, or the screening of, a plant or cell containing the marker. In plants, for example, the marker gene will encode resistance to antibiotics or herbicides. This allows the selection of transformed cells from among cells that are not transformed or transfected.

Examples of suitable selectable markers include adenosine deaminase, dihydrofolate reductase, hygromycin-B-phosphotransferase, thymidine kinase, xanthine-guanine phosphoribosyltransferase, glyphosate resistance and glufosinate resistance, and amino-glycoside 3-0-phosphotransferase (resistance to kanamycin, neomycin, or G418). These markers may include resistance to G418, hygromycin, bleomycin, kanamycin, and gentamicin. The construct may also contain the Bar selectable marker gene, which confers resistance to phosphinotricin analogues such as glufosinate ammonium. Thompson et al., EMBO J. 6: 2519-23 (1987). Other suitable selectable markers are also known.

Visible markers, such as green fluorescent protein (GFP), can be used. Methods to identify or select transformed plants based on the control of cell division have also been described. See John and Van Mellaert, WO 2000/052168, and Fabijansk et al., WO 2001/059086.

Replication sequences, of bacterial or viral origin, may also be included to allow the vector to be cloned into a bacterial or phage host. Preferably, a prokaryotic origin of a wider range of replication is used. A selectable marker for bacteria can be included to allow the selection of bacterial cells containing the desired construct. Suitable prokaryotic selectable markers also include resistance to antibiotics such as kanamycin or tetracycline.

Other DNA sequences that encode additional functions may also be present in the vector, as known from the technique. For example, when *Agrobacterium* is the host, T-DNA sequences can be included to facilitate subsequent transfer and incorporation into the plant's chromosomes.

### Plants for Genetic Engineering

The present invention comprises a method to improve an agronomic trait of interest in plants. Such a method occurs through silencing, repression, decreasing expression (*"downregulation"*) or knockout of the genes of the invention.

In this description, the term "plant" denotes any plant material of interest, including, but not limited to, differentiated or undifferentiated plant cells, protoplasts, whole plants, plant tissues, or plant organs, or any component of a plant such as leaf, stem, root, tuber, fruit, rhizome, or the like.

Plants that can be engineered according to the invention include, but are not limited to, crop plants, such as, for example, monocotyledons such as grasses (Poaceae), including maize, rice, wheat, sugarcane, sorghum, miscanthus, rye, oats, barley, as well as banana, palm trees, and dicotyledons, such as canola, soybean, bean, cassava, cotton, tomato, potato, papaya, cocoa, grapevine, castor bean, eucalyptus, poplar, citrus, vegetables.

In the present description, "genetically modified plant" refers to a plant in which a sequence of nucleic acids has been incorporated by insertion or modification, including, but not limited to, genes that are not normally present in a host plant genome, sequences of nucleic acids not normally transcribed into RNA or translated into a protein, or any other genes or sequences of nucleic acids that are intended to be introduced into the wild-type plant, such as genes that may normally be present in the wild-type plant, but which are intended to be genetically engineered or have their expression altered. The category of "genetically modified plant" includes either a primary transformant or a plant that includes a transformant in its lineage, for example, through standard introgression or any other breeding procedure.

A "hybrid plant" refers to a plant or part of same resulting from a cross between two parent plants, in which a parent plant is a genetically modified plant of the invention. Such crossing can occur naturally, for example, by sexual reproduction, or artificially, for example, a nuclear fusion *in vitro.* The methods for plant multiplication are well known and within the level of an ordinary knowledge of the technique of plant biology.

In contrast, a plant that is not genetically modified is a control plant and is referred to as an "unmodified" or "control" or "wild" plant. An unmodified plant may be a plant whose genome is not modified by the introduction of a construct comprising the polynucleotide sequences or any fragment thereof from the present invention. It may also be a plant generated from cells or tissues grown without prior modification by the introduction of a construct comprising the polynucleotide sequences of the invention, or it may comprise homozygous recessive progeny (i.e., it has no copy of the transgene) resulting from self-fertilization of a transgenic plant.

It is contemplated that, in some cases, the genome of a modified plant will be augmented through the stable introduction of a transgene. In other cases, however, the endogenous gene will be edited, without the introduction of DNA sequences into the plant's genome. A preferred gene with respect to the present invention is a DNA sequence of *AIP10* or *ABAP1* of plants of agronomic interest, edited by means of tools such as CRISPR-Cas9, which allows the introduction of mutations in regions of interest to obtain a desired phenotype. It is also important to note that plants subjected to this type of gene editing would not be considered transgenic plants, which would simplify regulatory issues.

### Methods for Genetic Engineering

Constructions according to the invention can be introduced into any cell of the plant, using a suitable technique. Both angiosperm or gymnosperm plant cells of monocotyledons and dicotyledons can be genetically engineered in several ways known from the technique. For example, see Klein et al., 1993, Biotechnology 4: 583-590; Bechtold et al., 1993, C. R. Acad. Sci. Paris 316: 194-1199; Koncz and Schell, 1986, Mol. Gen. Genet. 204: 383-396; Paszkowski et al., 1984, 30 EMBO J. 3: 2717-2722; Sagi et al., 1994, Plant Cell Rep. 13: 262-266.

*Agrobacterium* species such as *A. tumefaciens* and A. *rhizogenes* can be used, for example, according to Nagel et al., 1990, Microbiol Lett 67: 325. In summary, *Agrobacterium* can be used with a plant expression vector, for example, electroporation, after which *Agrobacterium* is introduced into plant cells through, for example, the well-known method of leaf discs.

Additional methods for achieving this include, but are not limited to, transformation by *Rhizobium, Sinorhizobium,* or *Mesorhizobium* (Broothaerts et al., 2005, Nature 433: 629-633), electroporation, particle gun bombardment, calcium phosphate precipitation, and glycol fusion, transfers in germinating pollen grains, direct transformation (Lorz et al., 1985, Mol. Genet. 199: 179-182), and other well-known methods of the technique. If a selection marker, such as resistance to kanamycin, is employed, it becomes easier to determine which cells are successfully transformed.

The *Agrobacterium* transformation methods discussed above are known to be useful for transformed dicotyledons. Additionally, de La Peña et al., 1987, Nature 325: 274-276; Rhodes et al., 1988, Science 240: 204-207; and Shimamoto et al., 1989, Nature 328: 274-276, all of which are incorporated by way of reference, have monocot cereals transformed using *Agrobacterium.* See Bechtold and Pelletier, 1998, Methods Mol. Biol. 82: 259-266, showing the use of vacuum infiltration for *Agrobacterium*-mediated transformation.

The presence of a protein, polypeptide, or nucleic acid molecule in a particular cell can be measured to determine whether, for example, a cell has been successfully transformed or transfected. The ability to perform such an assay is well known and need not be reiterated here.

### Quantification of improvement in agronomic traits

Transgenic plants of the invention are characterized by the improvement of an agronomic trait of interest.

Such a goal in the genetically modified plant is preferably achieved by altering the expression level of the *AIP10* and/or *ABAP1* genes, through silencing, repression, decreased expression ("*downregulation*") or knockout of these genes compared to a wild plant or Col-0 (WT) or WT.

Studies conducted by the inventors proved that the use of genetically modified plants to alter the expression level of these genes was able to improve agronomic traits of interest.

Plants with partial or complete suppression of the *AIP10* gene and partial suppression of the *ABAP1* gene showed agronomic traits of interest superior to wild plants. In addition, complete suppression of the *AIP10* gene was shown to be superior to partial suppression.

### EXAMPLES

Considering the foregoing, a person skilled in the art will recognize that the embodiments described below are merely illustrative of the invention, which may be carried out in various forms. Accordingly, specific structural and functional details disclosed in this document should not be construed as limiting.

### EXAMPLE 1

### ACQUISITION AND MOLECULAR ANALYSIS OF A. THALIANA MUTANTS WITH COMPLETE (KO) AND PARTIAL (KD) SUPPRESSION OF AIP10

Plants of *A. thaliana* with T-DNA insertion by *Agrobacterium tumefaciens* were generated. Plants with inserts in the *AIP10* gene were selected and analyzed for the molecular nature of the insert.

Seeds of the Col-0 wild type ecotype and T-DNA insertion mutants in exon and in the 5'UTR region of *A. thaliana* of the *AIP10* gene were obtained from the Salk collection (Salk Institute Genomic Analysis Laboratory). The *aip10ko* and *aip10kd* mutants were selected in 50 mg/I of kanamycin in germination medium and the T-DNA insertions were confirmed by PCR with specific primers for T-DNA insertion in the *AtAlP10* gene (AtAIP10-RP + AtAIP10-LP primers) and the LB1.3 primer (T-DNA-specific primer). Homozygous mutants for *aip10 (*Salk_022-*aip10ko and* Salk_094-*aip10kd*) were used for phenotypic research. The seeds were sterilized with chlorine gas (97 ml of sodium hypochlorite 4.45% active chlorine and 3 ml of hydrochloric acid) for 2 hours. Subsequently, the seeds were germinated in Petri dishes containing 1/2 strength MS medium (Murashige and Skoog, 1962), 0.05% of 2-(N-morpholino) ethanesulfonic acid, 1% of sucrose and 1% of Agar (Sigma-Aldrich^{®}). The pH was adjusted to 5.7 using 1M solution of KOH, after being maintained for two days at 4 °C in the dark (stratification). Next, they were transferred to the cultivation room *in vitro* cultured at 22°C±2°C and under photoperiod of 12h light/12h dark. The plants were kept under these conditions for 15 days, which were collected for gene expression analysis.

Figure 1 shows a scheme localizing T-DNA insertion in *A. thaliana* mutants with partial suppression and complete suppression of *AIP10* gene expression. Two types of T-DNA insertion mutants were selected, seeking complete gene silencing (*knockout*) or partial silencing (*knockdown*), for functional studies of the effects on plant metabolism.

The Salk_094 lineage has an insertion in the 5'UTR and the Salk_022 lineage has an insertion in the second exon of the gene coding sequence *AIP10.*

From the selection of the mutants carried out in this example, they were analyzed for the molecular nature of the insert and the level of relative expression of the mRNA of the gene *AIP10.*

Gene expression analyses by qRT-PCR were performed in three biological replicates from a pool of 10 seedlings grown *in vitro* with 11 DAG. Wild seedlings (Col-0) and strains Salk_022, Salk_094 and AIP10^{OE} were macerated in liquid nitrogen and subjected to total RNA extraction using the protocol of Logemann et al. (Logemann et al. 1987). For RNA precipitation, 1/10V of 3M ammonium acetate, pH 4.8 and 2V of isopropanol were added. The tubes were homogenized by inversion and incubated at -80°C for 30 minutes and -21°C for two hours. Subsequently, the samples were centrifuged for 20 minutes at 4° C and 12,000g. The supernatant was discarded, and the RNA was desalinated with 70% ethanol treated with DEPC water and resuspended in water treated with DEPC. The amount of RNA was measured using a Thermo Scientific NanoDrop 2000c spectrophotometer and the quality was verified by electrophoresis on 1% agarose gel with ethidium bromide staining. Approximately 5µg of total RNA were treated with DNAse I (Biolabs) according to the manufacturing protocol. The first strand of cDNA was synthesized using the "Taqman First Strand cDNA Synthesis kit", in reactions with a final volume of 25µL. For each 500ng of total RNA, 2.5µL of Taqman RT Buffer 10X, 5.5µL of 25mM MgCl2, 5µL of 10mM deoxyNTPs mixture, 1.0µL of 50 µM random primers, 0.5µL of RNAse Inhibitor, 0.625µL of MultiScribe TM Reverse Transcriptase (50U/µL) were added. The samples were incubated at 25°C for 10 minutes, followed by 48°C for 40 minutes and a final step at 90°C for 5 minutes. The samples were diluted twice with ultrapure water and frozen at -20°C. To analyze gene expression, qRT-PCR reactions were performed with the SYBR Green PCR Master Mix (Applied Biosystems). To each well, 2µL of the first strand diluted 2X, 5 µL of SYBR Green 124 PCR Master Mix, 1 µL of forward primer (10 µM) and 1 µL of reverse primer (10 µM) were added, along with 1 µL of sterile ultrapure water for a final volume of 10µL. Relative mRNA levels were determined by normalizing amplification cycles of constitutive genes such as *Ubiquitin 14* (*UBI14*) and *GAPDH* of *A. thaliana.*

Figure 2 shows the relative expression of the mRNA of *AIP10* in Salk_022, Salk_094 mutants, and in plants overexpressing *AIP10* under the control of the constitutive promoter 35S (AIP10^{OE}). Gene expression analysis was analyzed by qRT-PCR and the data were normalized with *UBI14* and *GAPDH* as reference genes. The data presented represent mean values obtained from independent amplification reactions (n = 3) and biological replicates (n = 3). The bars indicate mean ± standard deviation.

Insertion in the coding sequence leads to complete suppression (knockout) of *aip10* (*aip10ko*), while insertion in the 5'UTR leads to partial suppression (*knockdown*) of aip10 *(aip10kd).*

### EXAMPLE 2

### ANALYSIS OF THE VEGETATIVE DEVELOPMENT OF MUTANTS OF A. THALIANA AIP1KO AND AIP10KD

Vegetative development analyses of the mutants *aip10ko* and *aip10kd* were performed.

Seeds of *A. thaliana* wild-type (Columbia), and mutant lineages *aip10ko* and *aip10kd* were sown *in vitro* to obtain homogeneous plants (which germinated on the same day). The seeds were sterilized with chlorine gas (97 ml of sodium hypochlorite 4.45% active chlorine and 3 ml of hydrochloric acid) for 2 hours. Subsequently, the seeds were germinated in Petri dishes containing 1/2 strength MS medium (Murashige and Skoog, 1962), 0.05% of 2-(N-morpholino) ethanesulfonic acid, 1% of sucrose and 1% of Agar (Sigma-Aldrich^{®}). The pH was adjusted to 5.7 using 1M solution of KOH, after being maintained for two days at 4 °C in the dark (stratification). They were then transferred to the cultivation room *in vitro* cultured at 22°C±2°C and under photoperiod of 12h light/12h dark. The plants were kept under these conditions for 15 days and then transferred to pots containing a mixture of soil:vermiculite (3:1) and grown at 22°C±2°C under a 16h light/8h dark photoperiod and covered with plastic film for 3 days for acclimatization.

Figure 3 shows the germination of Col-0 (WT), Salk_094 (*aip10kd*) and Salk_022 (*aip10ko*) seeds.

Total silencing of gene expression of the *AIP10* gene from *A*. *thaliana* (mutant *aip10ko)* a presents more positive effects at the beginning of vegetative development, being seen less expressively in plants with only reduced expression of the *AIP10* gene (mutant *aip10kd*)*.*

Figure 4 shows the analysis of vegetative development characteristics of Col-0 (WT), Salk_094 (*aip10kd*) and Salk_022 (*aip10ko*) plants, in which *aip10ko* and *aip10kd* plants display (A) larger leaf area, (B) number of leaves, (C) fresh weight and (D) dry weight.

The total silencing of gene expression of the *AIP10* gene from *A. thaliana (*a*ip10ko*) presents more positive effects on its vegetative growth, which are seen less expressively in plants with only the reduction of the expression of the *AIP10* gene (*aip10kd).* The bars represent means ± SD (standard deviation) and * significantly different from Col-0 (WT) with p<0.05. One-wayAnova Test - Dunnett. *The percentages in which the data quantified in each mutant (aip10ko and aip10kd) is greater than that quantified in wild plants are indicated in the Figure, under the different conditions tested.*

The relative mRNA expression of cell cycle marker genes was performed using three biological replicates with a pool of 10 seedlings grown *in vitro* with 11 DAG Col-0 (WT) seedlings, *aip10ko* and *aip10kd* were macerated in liquid nitrogen and subjected to total RNA extraction using the protocol of Logemann et al. (Logemann et al. 1987). For RNA precipitation, 1/10V of 3M ammonium acetate, pH 4.8 and 2V of isopropanol were added. The tubes were homogenized by inversion and incubated at -80°C for 30 minutes and -21°C for two hours. Subsequently, the samples were centrifuged for 20 minutes at 4° C and 12,000g. The supernatant was discarded, and the RNA was desalinated with 70% ethanol treated with DEPC water and resuspended in water treated with DEPC. The amount of RNA was measured using a Thermo Scientific NanoDrop 2000c spectrophotometer and the quality was verified by electrophoresis on 1% agarose gel with ethidium bromide staining. Approximately 5µg of total RNA were treated with DNAse I (Biolabs) according to the manufacturing protocol. The first strand of cDNA was synthesized using the "Taqman First Strand cDNA Synthesis kit", in reactions with a final volume of 25µL. For each 500ng of total RNA, 2.5µL of Taqman RT Buffer 10X, 5.5µL of 25mM MgCl2, 5µL of 10mM deoxyNTPs mixture, 1.0µL of 50 µM random primers, 0.5µL of RNAse Inhibitor, 0.625µL of MultiScribe TM Reverse Transcriptase (50U/µL) were added. The samples were incubated at 25°C for 10 minutes, followed by 48°C for 40 minutes and a final step at 90°C for 5 minutes. The samples were diluted twice with ultrapure water and frozen at -20°C. To analyze gene expression, qRT-PCR reactions were performed with the SYBR Green PCR Master Mix (Applied Biosystems). To each well, 2µL of the first strand diluted 2X, 5 µL of SYBR Green 124 PCR Master Mix, 1 µL of forward primer (10 µM) and 1 µL of reverse primer (10 µM) were added, along with 1 µL of sterile ultrapure water for a final volume of 10µL. Relative mRNA levels were determined by normalizing amplification cycles of constitutive genes such as *Ubiquitin 14* (*UBI14*) and *GAPDH* of *A. thaliana.*

Figure 5 shows the differential expression of cell cycle-related genes in Col-0 (WT) plants, *aip10kd* and *aip10ko.* The gene expression analysis was analyzed by qRT-PCR and the data were normalized with *UBI14* and *GAPDH* as reference genes. The data presented represent mean values obtained from independent amplification reactions (n = 3) and biological replicates (n = 3). The bars indicate mean ± standard deviation. *The expression values quantified in each mutant (aip10ko and aip10kd) are shown in the Figure, in the different genes tested.*

Surprisingly, the total silencing of the *AIP10* gene, involved in the control of cell divisions, does not lead to a penalty in its development. On the contrary, *aip10ko* plants show better performance in all analyzed vegetative growth traits, compared to plants with reduced levels of *AIP10 (aip10kd).* Corroborating these data, the expression of positive cell division marker genes (CycB1;1 and Cdt1a) was induced, and of the negative regulator *ABAP1* was reduced, both more significantly in the mutant *aip10ko* compared to the mutant *aip10kd.*

### EXAMPLE 3

### ANALYSIS OF THE REPRODUCTIVE DEVELOPMENT OF MUTANTS OF A. THALIANA AIP10KO AND AIP10KD

Analyses of the reproductive development of the mutants *aip10ko* and *aip10kd* were performed.

Reproductive development analyses were carried out on plants whose seeds were placed directly in pots containing a mixture of soil and vermiculite (3:1) and grown in a greenhouse at 22°C±2°C, under a photoperiod of 16h light/8h dark and covered with plastic film. After the 7-day acclimatization period, supplemented irrigation was started with a dose of 0.06g/L of NPK 20-20-20 fertilizer. This supplementation was maintained throughout the entire development until the reproductive phase during which analyses of phenotypic parameters were performed, such as counting the height of the main inflorescence axis, number of branches and siliques of the main inflorescence axis and weighing the amount of seeds produced by each plant.

Figure 6 shows a representative image comparing Col-0 (WT), *aip10kd* and *aip10ko* plants grown *in vivo* under a 16h/8h photoperiod with 57 days after germination (DAG).

Total silencing of gene expression of the *AIP10* gene of *A. thaliana* (mutant *aip10ko*) a presents positive effects on its reproductive growth, which are seen less expressively in plants with only the reduction of gene expression of *AIP10* (mutant *aip10kd*)*.*

Figure 7 shows the analysis of reproductive development characteristics of Col-0 (WT) plants, *aip10kd* and *aip10ko,* indicating that plants with partial or total suppression of the *AIP10* gene have (A) greater height of the main inflorescence axis, (B) total number of siliques in plants, (C) number of branches of the main inflorescence axis, (D) number of siliques in the 5cm interval, (E) in the main axis, and (F) total number of seeds per plant (productivity). The bars represent means ± standard deviation and * significantly different from Col-0 with p<0.05. One-way Anova - Dunnett test. *The percentages in which the data quantified in each mutant (aip10ko and aip10kd) is greater than that quantified in wild plants are indicated in the Figure, under the different conditions tested.*

Surprisingly, the total silencing of the *AIP10* gene, involved in the control of cell divisions, does not lead to a penalty in its development, nor to a phenotype of embryo lethality. On the contrary, *aip10ko* plants show better performance in all analyzed characteristics of reproductive development (productivity), compared to plants with reduced levels of *AIP10.*

### EXAMPLE 4

### PHOTOSYNTHETIC ANALYSIS OF MUTANTS OF A. THALIANA AIP10KO AND AIP10KD

Analyses of photosynthetic pigments, chlorophyll A, B and carotenoids, analysis of photosynthetic parameters of the mutants *aip10ko* and *aip10kd* were performed.

The contents of chlorophyll *a*, *b* and carotenoids were quantified by the DMSO solvent extraction method from plants of 11 DAG and 35 DAG, grown directly in soil as described in example 3, and the reading was performed in a spectrophotometer. Seedlings and rosette leaves of wild plants (Col-0), *aip10kd* and *aip10ko* were weighed and immediately submerged in microtubes containing 1ml of DMSO and kept at rest in the dark for 3 days as per (Ni et al., 2009). Subsequently, each sample was analyzed in duplicate technique by spectrophotometry (NanodropTM 2000, Thermo Fisher Scientific). The absorbance readings were taken at different wavelengths: A665nm for chlorophyll, A649nm for chlorophyll b and A480nm for carotenoid measurement, as described by Wellburn (1994). The results were presented as a ratio of pigment mass (µg) to ground tissue mass (g). To measure the efficiency of photosystem II, a closed fluorescence imaging device (Fluorcam 800 MF, Photon Systems Instruments) was used, in which 15 plants of each genotype were analyzed to measure the Fv/Fm, NPQ and Rfd ratio. To measure the exchange of CO₂, a closed 6400-17 chamber was used for whole plants, using 25DAG rosettes through the LI-COR 6400XT instrument (LI-COR Biosciences, USA). To record the assimilation of CO₂ in response to photosynthesis light, the concentration of CO₂ was adjusted to 400 ppm, and the photosynthetic photon flux density (PPFD) to 350 µmol m⁻² s⁻¹ and measurements were taken between 8:00 and 10:00.

Figure 8 shows the analysis of chlorophyll content and photosynthesis parameters of Col-0 (WT), *aip10kd* and *aip10ko* plants, indicating that *aip10ko* and *aip10kd* plants display better photosynthetic efficiency parameters at 11 DAG, including higher (A) photochemical efficiency of PSII under dark adaptation (Fv/Fm), (B) non-photochemical quenching (NPQ), and (C) plant vitality (Rfd) (n=15), (D) chlorophyll A content, (E) chlorophyll B content, and (F) carotenoids in plants at 11 DAG, (G) chlorophyll A content, (H) chlorophyll B content, and (I) carotenoids in plants at 32 DAG (n=10). Total silencing of *AIP10* differentially affected photosynthetic traits in 11 and 35 DAG plants, showing a higher content of chlorophyll A, B and carotenoids when compared to Col-0 (WT), even higher than what was observed in plants with reduced levels of *aip10 (aip10kd)*. The bars represent means ± standard deviation and * significantly different from Col-0 (WT) with p<0.05. Test *one-way* ANOVA - Dunnett. *The percentages at which the quantified data in each mutant (aip 10ko and aip10kd) is greater than that quantified in wild plants are indicated in the Figure under the different conditions tested.*

Total silencing of *AIP10* differentially affected photosynthetic traits in 11 and 35 DAG plants, showing a higher content of chlorophyll *a, b* and carotenoids when compared to Col-0 (WT). In addition, mutants *aip10ko* have higher photosystem activity, as well as higher energy dissipation in the form of heat when compared to Col-0 (WT).

Figure 9 shows the analysis of carbon fixation through the photosynthetic content of mutants *aip10* by means of parameters of photosynthetic efficiency of Col-0 (WT) plants, *aip10*ko and *aip10*kd with 25 DAG, indicating that *aip10ko* and *aip10kd* plants have higher (A) net CO assimilation rates₂; (B) Transpiration rate and (C) More efficient use of water. Measurements were made with LICOR-6400 XT at 22°C in CO₂ ambient at 400 µmol photons m⁻²s⁻¹ between 8:00 a.m. and 10:00 a.m. Mutants *aip10ko* have a better photosynthetic efficiency using the absorbed light energy more efficiently. Total silencing of *AIP10* affected photosynthetic performance in 25 DAG plants, showing higher rates of photosynthetic carbon assimilation per unit of leaf area, than in Col-0 (WT) plants and higher than those observed in plants with reduced levels of *aip10 (aip10kd).* The bars represent means ± standard deviation and * significantly different from Col-0 (WT) with p<0.05. Test *one-way* ANOVA - Dunnett (n=10). *The percentages at which the quantified data in each mutant (aip10ko and aip10kd) is greater than that quantified in wild plants are indicated in the Figure under the different conditions tested.*

Total or partial silencing of *AIP10* affected photosynthetic performance in 25 DAG plants, showing higher rates of photosynthetic carbon assimilation, per unit of leaf area, than in Col-0 (WT) plants. Mutants *aip10ko* contain more photosynthesis using the absorbed light energy much more efficiently.

Figure 11 shows the analysis of the differential expression of 8 photosynthetic genes of plants *aip10ko* in relation to Col-0 (WT) plants by qRT-PCR in the transcriptome of leaves with 11DAG, which indicated that in plants *aip10ko* the genes related to photosynthesis are more highly expressed than in the Col-0 (WT) plant. Data were normalized with *UBI14* and *GAPDH* as reference genes. The data represent mean values obtained from independent amplification reactions (n = 3) and biological replicates (n = 3). Mutants *aip10ko* had more highly expressed light-harvesting complex genes PSI and PSII and those linked to light absorption/protection, as well as more prominent photosynthetic performance.

Total silencing of *AIP10* differentially affected photosynthetic characteristics in 11 DAG and 35 DAG plants, showing a higher content of chlorophyll *a, b* and carotenoids when compared to Col-0 (WT). In addition, *aip10ko* mutants have higher photosystem II activity, as well as higher energy dissipation in the form of heat when compared to Col-0 (WT) as a form of protection from high CO₂ capture. In addition, the mutants show higher rates of photosynthetic carbon assimilation, per unit of leaf area, than in Col-0 (WT) plants, with an increase of approximately 84% and 68% in *aip10ko* and *aip10kd* plants respectively. In addition, this increase in photosynthesis consequently increases transpiration in mutants due to stomatal opening.

The results indicate that the performance in the growth and development of mutants is promoted by the increase of photosynthetic efficiency, under the influence of the efficiency of light capture and the higher capture of CO₂.

### EXAMPLE 5

### TRANSCRIPTOME ANALYSIS OF A. THALIANA AIP10KO

Analyses of the regulation of the expression of different classes of genes in the mutant *aip10ko* in relation to the Col-0 (WT) plant were performed.

To understand the genetic developments caused by the silencing of *AIP10,* a transcriptome (RNA-seq) of differential expression was generated between control plants (Col-0/WT) and *aip10ko* at two fundamental developmental times, 11 DAG corresponding to the vegetative phase of the development of *A. thaliana* and 35 DAG corresponding to the reproductive phase. To generate the libraries that were analyzed the total RNA of two repeats of 11 DAG (growth *in vitro*) and three repeats of rosettes (aerial part) and roots of 35 DAG (growth *in vivo*) were extracted using the protocol of Logemann et al. (Logemann et al. 1987). For RNA precipitation, 1/10V of 3M ammonium acetate, pH 4.8 and 2V of isopropanol were added. The tubes were homogenized by inversion and incubated at -80°C for 30 minutes and -21°C for two hours. Subsequently, the samples were centrifuged for 20 minutes at 4° C and 12,000g. The supernatant was discarded, and the RNA was desalinated with 70% ethanol treated with DEPC water and resuspended in water treated with DEPC. The amount of RNA was measured using a Thermo Scientific NanoDrop 2000c spectrophotometer and the quality was verified by electrophoresis on 1% agarose gel with ethidium bromide staining. Approximately 5µg of total RNA were treated with DNAse I (Biolabs) according to the manufacturing protocol. The first strand of cDNA was synthesized using SuperScript III (Invitrogen). The protocol allows for the synthesis of cDNA in a range of 10ng to 5µg of total RNA. 1µL of Oligo (dT)20 oligonucleotides (50mM) was added to a 0.5mL microtube; 10ng - 5µg of total RNA; 1µL of dNTP (10mM), and the volume was completed with sterile distilled H2O up to 13µL. The mixture was heated to 65°C for 5 minutes and incubated on ice for 1 minute. Then another 4µL of 5x First-Strand Buffer was added to the microtube; 1µL of DTT (0.1M); 1µL of RNAseOUT Recombinant RNAse Inhibitor (40U/µL) and 1µL of SuperScript III (200U/µL). The reaction was incubated at 50°C for 60 minutes and then inactivated at 70°C for 15 minutes. To remove the remaining RNA, 2U of RNAse H (USB, Affymetrix) were added to the microtube, and it was incubated at 37°C for 15 minutes. Finally, the samples were diluted 1 time with H2O MilliQ and sent to Fasteris Life Sciences S.A for RNA sequencing. 8 libraries (11DAG) and 12 libraries (35 DAG) were prepared according to the protocol available at http://www.fasteris.com and sequenced in Illumina HISeq2'OOO (11 DAG) and NovaSeq 6000 (35DAG) (single end). After sequencing, the raw readings were subjected to quality control (Q30>80%) and adapter adjustment using the Trimmomatic software. The readings were mapped (Bowtie) using the TAIR10 genome as a reference through the Bowtie software, a tool used to align sequencing reads to the reference sequences, and TopHat, a splicing identifier. To estimate the expression, normalization and differential expression between the samples, the Cufflinks software was used. From the significance cut-off with p-value <0,05, foram normalizados is reads de cada biblioteca através do método de FPKM (Fragments Per Killobase of loci per Million mapped reads), utilizando a fórmula de FPKM = (n° de fragmentos X 109) / (n° de fragmentos totais da biblioteca X tamanho do lócus). Após a normalização por FPKM, realizou-se a comparação dos genes diferencialmente expressos (DEGs) entre as bibliotecas, a partir da diferenga de expressão gerando valores de FoldChange (FC). Para gerar os padrões de expressão classificados em induzidos ou reprimidos, sendo > 0 or < 0 respectively, the Log base 2 of the FC values was calculated by comparing the FPKM of the library of interest against the FPKM control library. All bioinformatics analyses, as well as the sequencing were carried out by Fasteris Life Sciences S.A.

Figure 10 shows a schematic of the main genes differentially expressed in the shoots and roots of aip10koplants. Genes involved in CO₂ absorption and carbon metabolism, synthesis, and degradation of photosynthetic pigments. Analysis of the metabolism of energy, nitrogen, protein, amino acids, starch and sucrose in leaves and roots. The color red refers to genes that are upregulated, and the color blue refers to genes that are downregulated. Squares are for differentially expressed genes (DEGs) found in the transcriptome of 11-day-post-germination plant shoots (DAG) and hexagons for 35-DAG shoots. The rhombus is for DEGs found in the root transcriptome of plants with 11 DAG and the circle for the root of plants with 35 DAG. The main products of each route were highlighted with different colors as indicated in the figure.

Analysis of the differentially expressed genes of *aip10ko* plants and Col-0 (WT), showed that the categories of photosynthesis and carbon fixation are enriched, indicating that the silencing of *AIP10* leads to the induction of photosynthetic genes and carbon metabolism, as well as protein, amino acids, nitrogen, and cellular respiration, thus indicating a reprogramming of energy metabolism for the formation of new organs and tissues as observed in the phenotype of mutant plants.

The results indicate that the complete suppression of AIP10 triggers the transcriptional reprogramming of different genes involved in pathways that have a major impact on development and response to the environment.

### EXAMPLE 6

### ANALYSIS OF THE REGULATION OF GENES INVOLVED IN PHOTOSYNTHESIS

From the analysis performed in Example 5, genes involved in photosynthesis were selected for validation by qRT-PCR.

The relative expression of the mRNA of these differentially expressed genes related to photosynthesis was performed using three biological replicates with a pool of 10 seedlings grown *in vitro* with 11 DAG. Samples of Col-0 (WT) and *aip10ko* were macerated in liquid nitrogen and subjected to total RNA extraction using the protocol of Logemann et al. 1987). For RNA precipitation, 1/10V of 3M ammonium acetate, pH 4.8 and 2V of isopropanol were added. The tubes were homogenized by inversion and incubated at -80°C for 30 minutes and -21°C for two hours. Subsequently, the samples were centrifuged for 20 minutes at 4° C and 12,000g. The supernatant was discarded, and the RNA was desalinated with 70% ethanol treated with DEPC water and resuspended in water treated with DEPC. The amount of RNA was measured using a Thermo Scientific NanoDrop 2000c spectrophotometer and the quality was verified by electrophoresis on 1% agarose gel with ethidium bromide staining. Approximately 5µg of total RNA were treated with DNAse I (Biolabs) according to the manufacturing protocol. The first strand of cDNA was synthesized using the "Taqman First Strand cDNA Synthesis kit", in reactions with a final volume of 25µL. For each 500ng of total RNA, 2.5µL of Taqman RT Buffer 10X, 5.5µL of 25mM MgCl2, 5µL of 10mM deoxyNTPs mixture, 1.0µL of 50 µM random primers, 0.5µL of RNAse Inhibitor, 0.625µL of MultiScribe TM Reverse Transcriptase (50U/µL) were added. The samples were incubated at 25°C for 10 minutes, followed by 48°C for 40 minutes and a final step at 90°C for 5 minutes. The samples were diluted twice with ultrapure water and frozen at -20°C. To analyze gene expression, qRT-PCR reactions were performed with the SYBR Green PCR Master Mix (Applied Biosystems). To each well, 2 µL of the first strand diluted 2X, 5 µL of SYBR Green 124 PCR Master Mix, 1 µL of forward primer (10 µM) and 1 µL of reverse primer (10 µM) were added, along with 1 µL of sterile ultrapure water for a final volume of 10 µL. Relative mRNA levels were determined by normalizing amplification cycles of constitutive genes such as *Ubiquitin 14 (UB114)* and *GAPDH* of *A. thaliana.*

Figure 11 shows the analysis of the differential expression of 8 photosynthetic genes of *aip10ko* plants in relation to Col-0 (WT) plants by qRT-PCR in the transcriptome of leaves with 11DAG, which indicated that in *aip10ko* plants the genes related to photosynthesis are more highly expressed than in the Col-0 (WT) plant. Data were normalized with *UBI14* and *GAPDH* as reference genes. The data represent mean values obtained from independent amplification reactions (n = 3) and biological replicates (n = 3). Mutants *aip10ko* had more highly expressed light-harvesting complex genes PSI and PSII and those linked to light absorption/protection, as well as more prominent photosynthetic performance.

Some photosynthesis genes that were differentially expressed in the transcriptome of plants with 11 DAG *aip10ko* plants were validated by qRT-PCR. The analysis showed that such genes are induced indicating better photosynthetic performance, corroborating with the biochemical data obtained, that *aip10ko* mutants have greater photosystem activity, as well as greater energy dissipation in the form of heat when compared to Col-0 as a form of protection from high CO capture ₂.

*aip10* mutants have genes from the PSI and PSII light-harvesting complex and genes linked to light absorption/protection more highly expressed, in addition to more prominent photosynthetic performance.

### EXAMPLE 7

### CARBON METABOLISM ANALYSIS

From the analysis performed in Example 5, genes involved in carbon metabolism were selected for validation by qRT-PCR.

The relative mRNA expression of these differentially expressed genes related to metabolism was performed using three biological replicates with a pool of 10 seedlings grown *in vitro* with 11 DAG and three distinct rosettes grown *in vivo* with 35 DAG. Samples of Col-0 (WT) and *aip10ko* were macerated in liquid nitrogen and subjected to total RNA extraction using the protocol of Logemann et al. 1987). For RNA precipitation, 1/10V of 3M ammonium acetate, pH 4.8 and 2V of isopropanol were added. The tubes were homogenized by inversion and incubated at -80°C for 30 minutes and -21°C for two hours. Subsequently, the samples were centrifuged for 20 minutes at 4° C and 12,000g. The supernatant was discarded, and the RNA was desalinated with 70% ethanol treated with DEPC water and resuspended in water treated with DEPC. The amount of RNA was measured using a Thermo Scientific NanoDrop 2000c spectrophotometer and the quality was verified by electrophoresis on 1% agarose gel with ethidium bromide staining. Approximately 5µg of total RNA were treated with DNAse I (Biolabs) according to the manufacturing protocol. The first strand of cDNA was synthesized using the "Taqman First Strand cDNA Synthesis kit", in reactions with a final volume of 25µL. For each 500ng of total RNA, 2.5µL of Taqman RT Buffer 10X, 5.5µL of 25mM MgCl2, 5µL of 10mM deoxyNTPs mixture, 1.0µL of 50 µM random primers, 0.5µL of RNAse Inhibitor, 0.625µL of MultiScribe TM Reverse Transcriptase (50U/µL) were added. The samples were incubated at 25°C for 10 minutes, followed by 48°C for 40 minutes and a final step at 90°C for 5 minutes. The samples were diluted twice with ultrapure water and frozen at -20°C. To analyze gene expression, qRT-PCR reactions were performed with the SYBR Green PCR Master Mix (Applied Biosystems). To each well, 2 µL of the first strand diluted 2X, 5 µL of SYBR Green 124 PCR Master Mix, 1 µL of forward primer (10 µM) and 1 µL of reverse primer (10 µM) were added, along with 1 µL of sterile ultrapure water for a final volume of 10 µL. Relative mRNA levels were determined by normalizing amplification cycles of constitutive genes such as *Ubiquitin 14* (*UBI14*) and *GAPDH* of *A. thaliana.*

Figure 12 shows an analysis of 3 carbon metabolism genes by qRT-PCR differentially expressed in the transcriptome of leaves from *aip10ko* plants compared to Col-0 (WT) plants with (A) 11 DAG and (B) 35 DAG indicating that *aip10ko* plants have more highly expressed carbon metabolism genes compared to Col-0 (WT) plants. Data were normalized with *UBI14* and *GAPDH* as reference genes. The data represent mean values obtained from independent amplification reactions (n=3) and biological replicates (n=3). To meet their metabolic needs, in addition to high carbon absorption, *aip10ko* plants have an efficient carbon fixation, maintained in both young and mature plants.

Some carbon metabolism genes that were differentially expressed in the transcriptome of 11 DAG and 35 DAG of *aip10ko* plants were validated by RT-qPCR and are induced, indicating greater carbon fixation.

*aip10ko* mutants have metabolism genes that are more highly expressed in the early stages of development, remaining more prominent than Col-0 (WT) throughout development, thus indicating a more active metabolism with greater carbon fixation.

From the qRT-PCR validation analysis of the metabolism genes, the metabolic profiles of Col-0 (WT), *aip10kd* and *aip10ko* plants were evaluated in leaves at 20 DAG, 35 DAG.

For the analysis of the leaf metabolites of Col-0 (WT), *aip10kd* and *aip10ko* plants, the spectra were obtained using a Shimadzu IRPestige-21 total attenuated reflectance infrared spectrometer (ATR-FTIR) with a diamond crystal plate (IRIS module - PIKE Technologies). The leaves were divided into two distinct regions, and measurements were made to obtain a representative spectrum of the entire sample. The spectra were acquired in the region of 4000 to 500 cm⁻¹, and the instrumental conditions were 64 scans, resolution of 8 cm⁻¹, 298K and relative humidity below 15%. All samples were evaluated in three different biological replicates. The IRsolutions software (version 1.04) was used to process and suppress the analytical CO₂ signal band of the spectra. All data processing was performed in an internal computational routine implemented in Matlab 2021a^{®} (The MathWorks Inc., Natick, USA). The spectra were cut in the bio-fingerprint region (1600 to 900 cm-1). The second derivative was also applied and tested with Savitz-Golay smoothing using a second-order polynomial and a 7-point window, which removes not only simple additive displacements, but also first-order effects such as baseline displacement and spectral noise. All data were centered on the mean before the model was built. To identify carbohydrate metabolism, FTIR spectra were deconvoluted using Fourier auto-deconvolution in the region of 900 to 1200 cm-1. The content of proteins, lipids (triglycerides) and carbohydrates in *aip10ko* and relation to wild plants was also analyzed in both young leaves at 20 DAG and mature leaves at 35 DAG using the ATR-FTIR technique.

Figure 13 shows the comparison of the metabolic profile of Col-0 (WT) plants and *aip10ko* plants, in which *aip10ko* plants showed higher average spectra obtained by the ATR-FTIR technique in the region of 900-1700 cm⁻¹ in leaves with (A) 20DAG and (B) 35DAG compared to Col-0 (WT). *aip10kd* plants also showed higher average spectra obtained by the ATR-FTIR technique in the region of 900-1700 cm⁻¹ in leaves with 20DAG (C) and 35DAG (D) compared to Col-0 (WT). The analyses were carried out on 2 different leaves of 4 individuals of each genotype. It was found that in addition to high carbon absorption, *aip10ko* plants have an efficient carbon fixation, maintained in both young and mature plants to meet their metabolic needs.

Analysis of the metabolic profile: it was possible to observe that there is a greater abundance of proteins, lipids (triglycerides) and carbohydrates in *aip10 (aip10kd* and *aip10ko),* in both young leaves at 20 DAG and mature leaves at 35 DAG. *aip10ko* plants also show a higher metabolic content when plants with reduced levels of *aip10 (aip10kd)* are observed.

To meet their metabolic needs, in addition to high carbon absorption, *aip10* plants have an efficient carbon fixation, maintained in both young and mature plants.

### EXAMPLE 8

### ANALYSIS OF THE NUTRITIONAL PROFILE OF MUTANT SEEDS OF A. THALIANA AIP10KO

From the analysis described in Example 7, analyses of the nutritional content of *aip10ko* and *aip10kd* seeds compared to seeds of wild-type Col-0 (WT) plants were performed.

For the analysis of the metabolites in seeds, the same parameters and data treatment were used as in Example 7, however, a pool of 100 macerated seeds without hydration was used, enough to cover the entire surface of the ATR accessory crystal.

Figure 14 shows an analysis of the metabolic content in Col-0 seeds, *aip10ko* and *aip10kd:* (A) *aip10ko* seeds were evaluated under microscopy and showed no difference in (B) surface area in relation to Col-0 (WT). The bars represent means ± standard deviation and * significantly different from Col-0 with p<0.05. Student's t-test. *aip10ko* seeds showed higher (C) average spectra obtained by the ATR-FTIR technique in the region of 900-1700 cm⁻¹ compared to Col-0 (WT), as well as (D) *aip10kd* seeds also showed higher average spectra. The analyses were carried out on a macerate of 100 seeds of each genotype.

Analysis of seed size showed that there is no difference, however, when examining the metabolic profile, a higher content of proteins, lipids (triglycerides), and carbohydrates was observed in *aip10ko* as well as in the leaves, indicating a higher nutritional value. The improved vigor of mutants is also linked to the mobilization of the large stock of starch, proteins, and lipids in the seeds.

It was found that *aip10ko* seeds have higher nutritional value (with more proteins, lipids and carbohydrates). These metabolic changes are also fundamental for the better vigor of mutants linked to the mobilization of metabolites during germination until seedling establishment.

### EXAMPLE 9

### PHOTOSYNTHETIC ANALYSIS OF AIP10 MAIZE PLANTS EDITED BY CRISPR

From the analyses performed in the Examples above, CRISPR-Cas9-edited plants were evaluated to investigate the biotechnological potential of silencing *AIP10,* which was observed in *A. thaliana,* in a crop of agricultural importance.

Knockout maize seeds for the *AIP10* gene were obtained by gene editing by CRISPR-Cas9 in the B104 maize genotype using a dual gRNA approach. Two gRNAs were engineered to target the exon1 and exon2 of *AIP10* maize, respectively. The primers have been designed to match the double sgRNA sequences:
Fw (SEQ ID NO: 255): 
ATATATGGTCTCTCTGGCGACCTGGGGGGATCAGCCGGAGTTTTA
GAGCTAGAAATAGCAAGTTAAAATAAGG;
Rv (SEQ ID NO: 256):

TTATTGGTCTCTAAACTCCGCTTCTCGGATAGGTTATGCTTCTTGG
TGCCGC.

PCR was performed on the pCBC-MT1T2 plasmid, resulting in a fragment containing the desired target sites and the correct sites for binding on the pBUN411-Sp destination vector using Golden Gate cloning. The expression vector was transformed into the DH5α strain of *Escherichia coli* and the EHA101 strain of *Agrobacterium tumefaciens.* Embryogenic calli of maize of the B104 genotype were subjected to genetic transformation mediated by *A. tumefaciens.* Rooted shoots were confirmed by PCR analysis and transferred to the soil, acclimatized in the growth chamber for several weeks and then transferred to the greenhouse. Genome editing was verified by extracting genomic DNA from seedling leaves (Direct-zol^{™} RNA MiniPrep Plus from Zymo), followed by conventional PCR using specific primers for target 1 (Aip10-F2 primers (SEQ ID NO: 257): GGCCCAGAGCCACAAGATAA; Aip10-R2 (SEQ ID NO: 258) GCTTGTGGACCGAAACGAAG) and for target 2 (primers Aip10-F5 (SEQ ID NO: 259): CATCCTCGTCACTCGCTAAC; Aip10-R5 (SEQ ID NO: 260): ACATGAACGCGTACTCTTTATTATC), which specifically amplify genomic regions that flank the gRNAs. Genomic PCRs were performed using standard protocols, such as Gotaq DNA Polymarese, using the following reaction conditions: Denaturation at 98⁰C 30sec, Annealing at 60°C 30sec, extension at 72°C for 1min, 35 cycles. The PCR fragments Target1 and Target2 were purified and sequenced (Eurofins Genomics). Genomic DNA PCR was performed to identify Cas9 (primers: Cas9_FW2 (SEQ ID NO: 261): CGAGATGGCGAAGGTTGACG, Cas9_RV4 (SEQ ID NO: 262): AATGTCCGCTGCTTCCTCAG) using the following reaction conditions: Denaturation at 98⁰C 30 sec, Annealing at 68°C 30 sec, extension at 72°C for 40 seconds, 30 cycles. Plants with edited genome were crossed with B104 and self-pollinated. Heterozygous plants without Cas9 were selected at T1 (by genomic PCR and PAT assay) and were self-pollinated. On T2, wild plants and homozygous plants without the presence of exogenous DNA (parts of the vector containing Cas9) were selected. These were self-pollinated to generate T3 WT and homozygous seeds for tests with beneficial bacteria. The mutant maize lineages *aip10-1* and *aip10-2* showed a premature termination codon in exon 1, indicating a knockout of gene expression, by preventing the synthesis of the AIP10 protein.

From the analysis performed in Example 4, photosynthetic parameters were evaluated in maize plants edited for *aip10 (aip10-1 and aip10-2),* edited by CRISPR.

Photosynthetic analyses were performed with the mutant maize line *aip10-1,* which used 10 individuals of each genotype (WT-unedited or *aip10-1-* edited in the gene *AIP10).* Maize seeds B104 WT and *aip10-1* were previously incubated in sterile water for 1 hour without sterilization. Subsequently, the seeds were sown directly in tubes containing soil supplemented with the company's NPK in a 1cm furrow and all seeds were equally covered with substrate. Subsequently they were transferred to 3L pots containing soil supplemented with the company's NPK and cultivated at 25°C±2°C and under a photoperiod of 16h light/8h dark. The plants were maintained under these conditions throughout development and evaluated at 25 DAG for photosynthetic parameters. Photosynthetic pigment analyses were performed using the Falker ClorofiLOG^{®} model CFL1030 portable equipment in the middle of leaf 6. This analysis allowed us to evaluate the chlorophyll content a. To measure the exchange of CO₂ the LI-COR 6400XT instrument (LI-COR Biosciences, USA) was used. To record the assimilation of CO₂ in response to photosynthesis light, the concentration of CO₂ was adjusted to 400 ppm, and the photosynthetic photon flux density (PPFD) to 350 µmol m⁻² s⁻¹. The measurements were taken between 8:00 a.m. and 11:00 a.m., with leaf temperature maintained between 25 and 26°C.

Figure 15 shows the analysis of chlorophyll content and photosynthesis parameters of mutant maize plants such as the knockout of the *AIP10* gene through CRISPR gene editing. The edited maize lines *(aip10-1* and *aip10-2)* are considered total knockout because they changed the reading phase of the AIP10 protein, through the deletion of nucleotides in the coding region. The maize plants edited for *aip10 (aip10-1),* as well as in mutants *aip10ko* in *A. thaliana,* presented: (A) greater development in relation to Wt with 25DAG; (B) higher chlorophyll content a, chlorophyll readings were performed using the ClorofiLOG ^{®} portable chlorophyll meter; (C) higher net CO assimilation rates₂; (D) higher transpiration rate; (E) higher stomatal conductance; (F) higher intracellular CO concentration₂; (G) higher water efficiency (WUE) (n= 8), compared to wild plants. The measurements were performed with WT_CRISPR and *aip10_CRISPR* plants with 25DAG with the LICOR-6400 XT instrument at 25°C in CO₂ ambient at 400µmol and 500µmol of photons m⁻²s⁻¹. The bar graphs represent means ± standard deviation and * significantly different from WT with p<0.05. Student's t-test. *The percentages in which the data quantified in the aip10-1 mutant is greater than that quantified in wild plants are indicated in the Figure, under the different conditions tested.*

The total knockout of AIP10 differentially affected the photosynthetic characteristics of maize plants edited for *aip10* (*aip10-1*), showing a higher chlorophyll a content compared to WT, in addition to better photosynthetic performance, with higher rates of photosynthetic carbon assimilation (an increase of approximately 40%) than in WT plants. In addition, this increase in photosynthetic efficiency consequently increased transpiration in the *aip10-1* mutants due to stomatal opening. In addition, the internal concentration of CO2 was 18% lower in the edited *aip10-1* plants, which suggests that the CO2 captured is more fixed. Additionally, maize plants edited for *aip10* showed 25.7% better water use efficiency.

The results indicate that, similar to what was observed in *A. thaliana aip10ko* plants, the development performance of maize plants edited for *aip10* is promoted by the increase in photosynthetic efficiency, under the influence of light capture efficiency and higher CO2 capture.

Maize plants edited for *aip10,* obtained by gene editing (CRISPR), contain more chlorophyll a and higher photosynthetic efficiency, using the absorbed light energy much more efficiently, as observed in *aip10ko* plants of *A. thaliana.*

### EXAMPLE 10

### WEIGHT ANALYSIS OF SEEDS OF AIP10 MAIZE PLANTS EDITED BY CRISPR

Analyses of the seed weight of two maize lineages edited for *aip10* by CRISPR *(aip10-1* and *aip10-2),* compared to seeds from control WT plants.

To evaluate the potential effect of editing on grain yield, we selected a pool of 20 seeds in 12 replicates of two distinct lineages *(aip10-1* and *aip10-2)* from two independent editing events, and the weight was evaluated on a precision scale.

Figure 16 shows the seed analysis of two CRISPR strains *aip10.* Seeds of wild plants and *aip10-1 (event B)* did not show differences in the surface area of seeds (A). However, seeds of *aip10-1 (event B)* showed a higher seed weight when evaluated in a pool of 20 seeds (in 12 replications) (B). *aip10-2 (event E)* plant seeds showed a higher seed weight when evaluated in a pool of 20 seeds (in 12 replicates) C). The bar graph represents means ± standard deviation and * significantly different from WT with p<0.05. Student's t-test. Maize plants edited for *aip10 (aip10-1 and aip10-2,* events B and E respectively) showed higher seed weight in the two distinct gene editing events, shown in the graphs. *The percentages in which the data quantified in the edited mutant aip10 is greater than that quantified in wild plants are indicated in the Figure, under the different conditions tested.* The bar graph represents means ± SD (standard deviation) and * significantly different from WT with p<0.05. Student's t-test.

From the analysis of seed weight, it was observed that in both gene editing events *(aip10-1* and *aip10-2),* maize seeds edited for *aip10* have higher weight. The seeds of the *aip10-1* strain were about 10% heavier than the seeds of the WT plants, while the seeds of the strain *aip10-2* were about 4% heavier than the seeds of the WT plants. This change suggests a better maize vigor *aip10* (i.e. *aip10-1* and *aip10-2),* as observed in *A. thaliana aip10ko,* being linked to the mobilization of the large stock of starch, proteins, and lipids in the seeds.

*aip10* maize seeds edited by CRISPR have higher weight. This change suggests a higher vigor of the *aip10* edited maize, linked to the mobilization of metabolites during germination until seedling establishment.

### EXAMPLE 11

### ASSAYS OF A. THALIANA PLANTS SILENCED IN THE AIP10 GENE, INOCULATED WITH BENEFICIAL BACTERIA

Analyses of the association between plants and plant growth-promoting bacteria were carried out evaluating the response of bioinoculants in the *aip10ko* mutant in relation to the Col-0 (WT) plant.

The growth-promoting bacteria tested (*Gluconacetobacter diazotrophicus,* strain PAL5, *Herbaspirillum seropediceae* HRC54 (BR 11335) and *Azospirillum baldaniorum* SP245 and Ab6, were grown with 0.5 mL of pre-inoculum in the media: (a) LGI-P, adding 50µg/mL of streptomycin, pH 5.8 for G. *diazotrophicus* PAL5, JNFB, pH 6.0 for *H*. *seropediceae* HRC54 and NFB for *A. baldaniorum.* 0.05ml of each pre-inoculum was added in 50 mL of liquid DYGS medium (1.5g/L glutamic acid, 2g/L malic acid, 2.0g/L yeast extract, 0.5g/L K2HPO4, 2g/L glucose, 1.5g/L bacteriological peptone, 0.5g/L MgSO47H2O; malic acid was not added in the medium of *G*. *diazotrophicus* PAL5), and kept under agitation overnight at 30°C. Inoculation of the bacterium was performed at a 660nm OD of 0.8. The inoculum was diluted in a 1:1000 ratio with sterile distilled water, in order to maintain a concentration that corresponds to approximately 10⁵ - 10⁶ colony-forming units (CFU).

Seeds of *A. thaliana* wild-type (Columbia), and mutant strain *aip10ko* were sterilized with chlorine gas (97 ml of sodium hypochlorite 4.45% active chlorine and 3 ml of hydrochloric acid) for 2 hours. Subsequently, the seeds were germinated in Petri dishes containing 1/2 strength MS medium (Murashige and Skoog, 1962), 0.05% of 2-(N-morpholino) ethanesulfonic acid, 1% of sucrose and 1% of Agar (Sigma-Aldrich^{®}). The pH was adjusted to 5.7 using 1M solution of KOH, after being maintained for two days at 4 °C in the dark (stratification). Then they were transferred to the *in vitro* cultivation room, cultured at 22°C±2°C and under photoperiod of 12h light/12h dark. Subsequently, seedlings with 3DAG, with emerging rootlets, were inoculated with diazotrophic bacteria by drip method (5µl) on the surface of the radicle. The seedlings were left for 2 hours while the liquid medium containing the bacteria dried, and the non-inoculated seedlings were treated with 5µl of sterile DYGS medium. These seedlings were transferred to new plates with MS 1/2 strength, without sucrose, 1% agar, where they remained for another 13 days for phenotype visualization and collection. Root length measurements and quantification of the number of lateral roots were performed after 15 DAG (days after germination), and/or 7DAI (days after inoculation), using the IMAGE J software (Schindelin et al. 2012). These experiments were carried out in triplicates.

Figure 17 shows the effects of the use of bioinoculants containing diazotrophic bacteria on the *in vitro* growth of genetically modified plants regarding the *AIP10* gene. (A) Plants of *A. thaliana aip10ko* inoculated with diazotrophic bacteria show more shoots and root system compared to wild-type plants. Bioinoculants increase (B) the length of the taproot and (C) the number of lateral roots. Plants 10 DAG, 3 DAI. The bars indicate mean ± standard deviation. The data were analyzed by *two-way* ANOVA and the means were compared by Tukey's test at 5% probability (p<0.05). *, indicates statistical difference between the averages. DAG: days after germination; DAI: days after inoculation. *The percentages in which the quantified value in the mutant is greater than that quantified in wild plants are indicated in the Figure, under the different conditions tested* (*not inoculated or inoculated*)*.* Bioinoculant 1: *A. baldaniorum* Sp245; Bioinoculant 2: *H. seropedicae* HRC54.

*aip10ko* plants showed a taproot length 43% and 45% greater than Col-0 (WT) when inoculated with bioinoculant 1 and bioinoculant 2 respectively. This increase is higher than that observed in *aip10ko* non-inoculated plants that showed an increase of 39% in relation to Col-0 (WT). In addition, it was observed that *aip10ko* plants had 15% and 35% more lateral roots in relation to Col-0 (WT) when inoculated with bioinoculant 1 and bioinoculant 2 respectively. *aip10ko* plants when inoculated with bioinoculant 2 showed an increase in the number of lateral roots higher than observed in non-inoculated *aip10ko* plants, which showed a 25% increase compared to Col-0 (WT).

Figure 18 shows the effects on root architecture in the *in vitro* growth of *aip10ko* plants with the use of different bioinoculants, containing the diazotrophic bacteria *A. baldaniorum* or *H. seropedicae. A. thaliana aip10ko* plants inoculated with two different species of beneficial diazotrophic bacteria have a more developed root system, compared to wild-type plants. Bioinoculants increase (A) root length and (B) the number of lateral roots. NI-Col: Wild control non inoculated; NI-KO: *aip10ko* mutant not inoculated; AZO-Col: wild plant inoculated with *A. baldaniorum;* AZO-KO: mutant plant inoculated with *A. baldaniorum;* HS-Col: wild plant inoculated with *H. seropedicae;* HS-KO: mutant plant inoculated with *H. seropedicae.* The bars indicate mean ± standard deviation. Data were analyzed by *one-way* ANOVA and means were compared by Bonferroni's test at 5% probability (p<0.05).

*aip10ko* plants showed a better root growth response, with longer main root length and higher number of lateral roots, to inoculation with different bioinoculants containing distinct species of diazotrophic bacteria, compared to Col-0 (WT) inoculated plants.

*A. thaliana aip10ko* plants are more responsive to association with bioinoculants, with greater increase in root growth.

For phenotypic analyses at later stages of development, Col-0 (WT) plants and *aip10ko* non-inoculated and inoculated *in vitro,* according to the methodology described above, were kept under these conditions for 15 days and subsequently transferred to pots containing a soil:vermiculite mixture (3:1) and grown at 22°C±2°C with a 16h light/8h dark cycle and covered with plastic wrap for 3 days for acclimatization. After acclimatization, vegetative and reproductive development was monitored for data recording and quantification. For vegetative development analyses, phenotypic parameters such as rosette area and fresh weight were evaluated, while for reproductive development phenotypic parameters such as the number of siliques on the main inflorescence axis and the weighing of the amount of seeds produced by each plant were evaluated.

Figure 19 shows the effects of the use of bioinoculants with diazotrophic bacteria on the growth of *aip10ko* in the soil. Plants of *A. thaliana aip10ko* have (A-B) more developed rosettes (rosette area in cm²) and (C) shoot biomass (fresh rosette weight), compared to wild-type plants. This increased growth in aip10koplants is more pronounced when they are inoculated with bioinoculants containing diazotrophic bacteria. The final yield of the plant in relation to (D) the number of siliques and (E) seeds is higher in inoculated *aip10ko* plants than in inoculated control plants. The bars indicate mean ± standard deviation. The data were analyzed by *one-way* ANOVA and the means were compared by the Bonferroni test at 5% probability (p<0.05). *The percentages in which the quantified value in the mutant is greater than that quantified in wild plants are indicated in the Figure, under the different conditions tested.* Bioinoculant 1: *A. baldaniorum* Sp245; Bioinoculant 2: *H. seropedicae* HRC54.

*aip10ko* plants showed a larger rosette area around 30% larger than Col-0 (WT) when inoculated with the bioinoculant. This increase is higher than that observed in *aip10ko* non-inoculated plants that showed an increase of 25% in relation to Col-0 (WT). In addition, it was observed that aip10koplants had 34% more fresh mass (fresh weight) compared to Col-0 (WT) when inoculated with the bioinoculant. This increase is higher than that observed in *aip10ko* non-inoculated plants that showed an increase of 26% in relation to Col-0 (WT). In addition, *aip10ko* plants showed 60% more siliques per main inflorescence axis compared to Col-0 (WT) when inoculated with bioinoculants 1 and 2. *aip10ko* plants showed a 73% increase in seed yield compared to Col-0 (WT) when inoculated with the bioinoculant.

*aip10ko* plants grown in soil show better growth in response to inoculation with bioinoculants containing diazotrophic bacteria, compared to Col-0 (WT) inoculated plants.

From the analysis performed in Example 5, analyses of photosynthetic pigments, chlorophyll *a* and *b* were performed in Col-0 (WT) plants and *aip10ko* in response to the bioinoculants and the best association observed in the mutant *aip10ko.*

The chlorophyll contents a and b were quantified by the DMSO solvent extraction method from Col-0 (WT) and *aip10ko* plants of 35 DAG, inoculated and non-inoculated, and the reading was performed in a spectrophotometer. Rosette leaves were weighed and immediately submerged in microtubes containing 1ml of DMSO and kept at rest in the dark for 3 days as per (Ni et al., 2009). Subsequently, each sample was analyzed in duplicate technique by spectrophotometry (NanodropTM 2000, Thermo Fisher Scientific). The absorbance readings were taken at different wavelengths: A665nm for chlorophyll a, A649nm for chlorophyll b, and A480nm for carotenoid measurement, as described by Wellburn (1994). The results were presented as a ratio of pigment mass (µg) to ground tissue mass (g).

Figure 20 shows that *aip10ko* plants have higher chlorophyll content when they are associated with bioinoculants composed of diazotrophic bacteria. Plants in *A. thaliana aip10ko* have higher levels of chlorophyll a (mg Chla/g leaf) and chlorophyll b (mg Chlb/g leaf) when these plants are inoculated with the bioinoculant containing diazotrophic bacteria. Increased levels of chlorophyll have been linked to an increased level of photosynthesis. Floor plans 35 DAG, 28 DAI. DAG: days after germination; DAI: days after inoculation. Data were analyzed by *two-way* ANOVA and means were compared by Bonferroni's test at 5% probability (p<0.05). *The percentages in which the quantified value in the mutant is greater than that quantified in wild plants are indicated in the Figure, under the different conditions tested.* Bioinoculant 1: *H. seropedicae* HRC54.

*aip10ko* plants showed higher content of photosynthetic pigments, with chlorophyll a being about 40% higher than Col-0 (WT) when inoculated with the bioinoculant. This increase is higher than that observed in *aip10ko* non-inoculated plants that showed an increase of 35% in relation to Col-0 (WT). In addition, *aip10ko* plants have 40% more chlorophyll *b* when compared to Col-0 (WT) plants.

*aip10ko* mutants associate more efficiently with biofertilizers, maximizing the benefits of chlorophyll content a and b already found in a superior way in non-inoculated *aip10ko* plants.

From the analyses with plants *aip10ko* and the best association with bioinoculants, an analysis of the colonization rate of the bacteria present in the bioinoculants was performed through qRT-PCR.

To verify bacterial colonization in roots of *A.thaliana,* the real-time PCR technique (qRT-PCR) was performed using DNA samples extracted from root tissues, obtained from *in vitro* experiments transplanted to soil. DNA samples were standardized to an initial concentration of 60 ng\µL. DNA concentrations were determined by measuring optical density at 260-280 nm, using the NanoDrop device (NanoDrop Technologies). The qRT-PCR analysis was performed with the SYBR Green PCR Master Mix kit, from the company Applied Biosystems, and the 7500 Fast Real-Time PCR System (Applied Biosystems) equipment. The reactions with a final volume of 10 µL were performed in a 96-well plate (MicroAmp Optical 96-Well Reaction Plate, from the company Applied Biosystems). The reactions were prepared for the final volume of 10 µL, containing: 1 µL of genomic DNA, 5 µL SYBR Green, 2 µL of the mixture of the two oligonucleotides at 10 µM each, and 2 µL of ultrapure H2O to complete the reaction volume. The colonization rate was measured from the amplification of single-copy genes specific for *G*. *diazotrophicus* PAL5, *Azospirillum baldaniorum* Sp245 and *H. seropediceae* HRC54.

Figure 21 shows the colonization rate in absolute single-copy values of specific genes for bioinoculants, in *A. thaliana.* The colonization rate was measured from the amplification of single-copy genes specific for bioinoculant 1 and bioinoculant 2, in mutant plants for the *AIP10* gene. All plants were grown in earth-vermiculite substrate in photoperiod 12 hours light/12 hours dark and collected 37 days after inoculation and 40 days after germination. Bioinoculant 1: *A. baldaniorum* Sp245; Bioinoculant 2: *H. seropedicae* HRC54.

*aip10ko* plants showed a significantly higher number of beneficial diazotrophic bacteria associated with their roots when compared to Col-0 (WT) plants. This higher number of diazotrophic bacteria was maintained for both inoculation with bioinoculant 1 and with bioinoculant 2. Showing that *aip10ko* plants interact better with beneficial bacteria and are more benefited by higher rates of biological nitrogen fixation.

*aip10ko* plants associate better with beneficial diazotrophic bacteria, showing a higher number of bacteria associated with their roots in inoculated plants

From the data of greater colonization of beneficial diazotrophic bacteria in *aip10ko,* the mRNA expression level of the *NifH* gene, which is used as a marker of bacterial nitrogenase activity, was measured by qRT-PCR.

Roots of 30 inoculated and non-inoculated seedlings (Mock) were macerated in liquid nitrogen and submitted to total RNA extraction using the protocol of Logemann et al. (Logemann et al. 1987). For RNA precipitation, 1/10V of 3M ammonium acetate, pH 4.8 and 2V of isopropanol were added. The tubes were homogenized by inversion and incubated at -80°C for 30 minutes and -21°C for two hours. Subsequently, the samples were centrifuged for 20 minutes at 4° C and 12,000g. The supernatant was discarded, and the RNA was desalinated with 70% ethanol treated with DEPC water and resuspended in water treated with DEPC. The amount of RNA was measured using a Thermo Scientific NanoDrop 2000c spectrophotometer and the quality was verified by electrophoresis on 1% agarose gel with ethidium bromide staining. Approximately 5µg of total RNA were treated with DNAse I (Biolabs) according to the manufacturing protocol. The first strand of cDNA was synthesized using the "Taqman First Strand cDNA Synthesis kit", in reactions with a final volume of 25µL. For each 500ng of total RNA, 2.5µL of Taqman RT Buffer 10X, 5.5µL of 25mM MgCl2, 5µL of 10mM deoxyNTPs mixture, 1.0µL of 50 µM random primers, 0.5µL of RNAse Inhibitor, 0.625µL of MultiScribe TM Reverse Transcriptase (50U/µL) were added. The samples were incubated at 25°C for 10 minutes, followed by 48°C for 40 minutes and a final step at 90°C for 5 minutes. The samples were diluted twice with 10mM of Tris-Cl pH 8.0 and frozen at -20°C. To analyze gene expression, qRT-PCR reactions were performed with the SYBR Green PCR Master Mix (Applied Biosystems). To each well, 2.5 µL of the first strand diluted 2X, 5 µL of SYBR Green 124 PCR Master Mix, 1 µL of forward primer (10 µM), and 1 µL of reverse primer (10 µM) were added, along with 0.5 µL of sterile ultrapure water for a final volume of 10 µL. Relative mRNA levels were determined by normalizing amplification cycles of constitutive genes such as Ubiquitin 10 and 28S (ribosomal gene) of *A. thaliana.* The expression level of the *NifH* gene was measured from a universal oligonucleotide capable of evaluating a wide diversity of bacterial genera, being able to evaluate the contribution of *H. seropediceae* HRC54 used in the experiment.

Figure 22 shows the mRNA levels of the *NifH* gene in *of A. thaliana* Col-0 (WT) plants and mutants for the *AIP10 gene.* The graph shows the expression of mRNA levels of the bacterial *NifH* from *H*. *seropedicae* HRC54, in control plants and mutants for the *AIP10* gene in *A. thaliana.* The results suggest that the native microbiota present in *aip10ko* plants may already have a higher biological nitrogen fixation activity due to the greater responsiveness of this mutant to beneficial bacteria. The bars indicate mean ± standard deviation. Statistical analysis with *two-way* ANOVA, Multiple Comparisons. p<0.05%. Plants *aip10* (15 DAG) showed a higher expression of the bacterial NifH gene, compared to the control plant. *The percentages in which the quantified value in the mutant is greater than that quantified in wild plants are indicated in the Figure, under the different conditions tested.*

The inoculated *aip10ko* plants showed a higher expression of the bacterial *NifH* gene, compared to the Col-0 (WT) plant. As the mRNA expression level of the *NifH* gene is used as a marker of bacterial nitrogenase activity, the results suggest that *aip10ko* plants inoculated with beneficial and nitrogen-fixing bacteria favor better colonization and greater biological nitrogen-fixation activity due to the higher responsiveness of this mutant to beneficial bacteria.

*aip10ko* mutants seem to have improved N2 fixation favoring the development of the vegetative and reproductive part, with increased production of siliques when inoculated with bioinoculants of beneficial diazotrophic bacteria.

From the data of greater colonization of beneficial diazotrophic bacteria, higher expression of mRNA of the *NifH* gene, and the results of better association in the mutant *aip10ko,* the inoculation responses under conditions of low and high nitrogen availability were analyzed.

The seeds were germinated for 48 hours in Petri dishes with moist germitest paper (until radicle emergence) in the in vitro growth chamber. Subsequently, seedlings with 1 DAG were inoculated with the diazotrophic bacteria with a concentration of 103 cells per seed. The seeds were left for 3 hours under constant agitation. The non-inoculated Col-0 (WT) and *aip10ko* seeds were subjected to the same treatment, replacing the inoculum with sterile DYGS medium. Next, the seeds were transferred to pots containing a mixture of soil and vermiculite (3:1) and grown in a greenhouse, at 21/22 °C, under a photoperiod of 16/8 hrs. After the 7-day acclimatization period, watering started with two different concentrations of Peters fertilizer: 0.06 g\L and 0.12 g\L, considered NB (low nitrogen dose) and NA (high nitrogen dose) respectively. In the first collection carried out within 7 days after inoculation, samples were obtained for molecular analysis. Subsequently, at 25 days after inoculation, the collection was performed for phenotypic analyses using parameters of fresh weight and dry weight.

Figure 23 shows the effects of the use of bioinoculants containing diazotrophic bacteria on soil growth of *aip10ko* plants at low and high doses of chemical nitrogen fertilization. (A) Plants of *A. thaliana aip10ko* inoculated with bioinoculants show more developed rosette aerials compared to wild-type plants at both high and low nitrogen fertilization doses. (B) Bioinoculant 2 promotes fresh weight gain in mutant *aip10ko* plants with low nitrogen availability. (C) In addition, bioinoculants 1 and 2 do not promote fresh weight gain in mutant *aip10ko* plants at high nitrogen availability. (D) Bioinoculants 1 and 2 promote dry weight increase in mutant *aip10ko* plants with low nitrogen availability. (E) Bioinoculants 1 and 2 do not promote dry weight increase in mutant *aip10ko* plants at high nitrogen availability. It was also observed that at high nitrogen dose, the uninoculated *aip10ko* mutant shows a more pronounced increase in dry weight (biomass) than the non-inoculated WT control plants. The groups were divided into plants under NB (low nitrogen availability) and NA (high nitrogen availability) conditions and inoculated with two distinct bioinoculants, called bioinoculant 1 and bioinoculant 2. The bar chart represents means ± standard deviation and * significantly different from Control with p<0.05. Student's t-test within each treatment. *The percentages in which the quantified value in the mutant is greater than that quantified in wild plants are indicated in the Figure, under the different conditions tested.*

The results showed that *aip10ko* plants are more responsive to bioinoculants under low nitrogen availability, exhibiting greater dry biomass, similar to the phenotype observed in mutant plants not inoculated in environments with high fertilizer availability. This benefit was corroborated by fresh and dry biomass analyses, wherein the *aip10ko* plants showed 20% to 30% higher fresh weight and 18% to 26% higher dry weight when inoculated in environments with low nitrogen availability when compared to Col-0 (WT) plants in the same condition. In addition, fresh and dry biomass were observed with an increase of 30% and 23% respectively in mutant *aip10ko* plants not inoculated under conditions of high fertilizer availability.

*aip10ko* plants showed better association with bioinoculants containing diazotrophic bacteria, presenting accumulation of fresh and dry biomass, compared to Col-0 (WT) plants, and furthermore, these benefits are observed in inoculation with a low dose of nitrogen chemical fertilization. These data indicate that the use of bioinoculants can largely replace the use of chemical nitrogen fertilizers in mutant plants *aip10ko.*

Analyses of the association between plants and plant growth-promoting bacteria were performed evaluating the response in the mutant *aip10ko* in relation to the Col-0 (WT) plant in response to water stress.

The seeds were germinated for three days in the Petri dishes (until radicle emergence) in the growth chamber *in vitro.* Subsequently, seedlings with 3DAG were inoculated with diazotrophic bacteria by drip method (5µl) on the radicle surface. The seedlings were left for 2 hours while the liquid medium containing the bacteria dried, and the non-inoculated seedlings were treated with 5µl of sterile DYGS medium. These seedlings were transferred to new plates with MS 1/2 strength, without sucrose, 1% agar, where they remained for another 13 days for phenotype visualization and collection. A group of plants was transferred to pots containing a mixture of soil:vermiculite (3:1) and grown at 22°C±2°C with a cycle of 16h light/8h dark and covered with plastic wrap for 3 days for acclimatization. The drought experiments were carried out by suspending the watering of the plants at 25 DAG (12 plants per genotype and inoculation condition) for 12 days, next the phenotype of the plants was recorded.

Figure 24 shows that *aip10ko* plants inoculated with bioinoculant containing beneficial diazotrophic bacteria have higher tolerance to water stress. Representative images of wild and *aip10ko* mutant plants of *A. thaliana* grown under normal conditions and under water stress conditions for 15 days. These plants were separated into two groups: non-inoculated (left) and inoculated with bioinoculant containing beneficial diazotrophic bacteria (right). During water stress, the non-inoculated plants showed strong wilt, chlorosis and anthocyanin synthesis in the leaves. On the other hand, *aip10ko* plants inoculated with the bioinoculant showed less wilting, chlorosis, and anthocyanin synthesis in the leaves, when compared to non-inoculated plants. *aip10ko* plants show greater tolerance to water stress when they are inoculated with bioinoculants containing diazotrophic bacteria, compared to non-inoculated or wild-type inoculated plants. Bioinoculant: *H. seropedicae* HRC54.

*aip10ko* plants show greater tolerance to water stress situations when inoculated with bioinoculants containing diazotrophic bacteria, compared to non-inoculated or Col-0 (WT) inoculated plants. The *aip10ko* plants when inoculated under water stress showed fewer phenotypic signs of stress, such as lower turgidity, purplish leaves, and signs of necrosis on the leaves.

*A. thaliana aip10ko* plants show greater association with bioinoculants of beneficial diazotrophic bacteria and greater tolerance to water stress.

### EXAMPLE 12

### ANALYSIS OF RESISTANCE TO ROOT-KNOT NEMATODES MELOIDOGYNE INCOGNITA IN MUTANTS OF A. THALIANA SILENCED IN AIP10 GENE

Analyses of root-knot nematode *(M. incognita)* infection were performed in Col-0 (WT) plants and *aip10ko* mutants under normal conditions and under water deficit.

Infection analyses of *aip10ko* and wild plants with root-knot nematodes *M*. *incognita were performed.* Mutant seeds *aip10ko* and col-0 were placed in pots containing a mixture of soil and sand (2:1) and kept in the refrigerator for 48 h to synchronize germination. The seeds were then transferred to growth chambers with short day photoperiod (8/16 h light:dark at 21°C/18°C) for germination and cultivated for approximately 10 days. Each seedling was then placed in a pot to allow the development of the root system and kept for another 15 days. Subsequently, each seedling was inoculated with 200 second-stage juveniles (J2s) of RKN *M*. *incognita* and transferred to a growth chamber with long day photoperiod (16/8 h light:dark at 21°C/18°C), respectively to obtain galls for the experiments described below. The plants were evaluated according to the objective of each bioassay. For the infection test of *aip10ko* with *M. incognita* under water deficit, the same protocol as above was followed. After inoculation with *M*. *incognita,* watering was suspended for the first time for a period of 24 days, when they were watered again. On this day, irrigation was suspended again for another 24 days when they were collected to carry out the pertinent analyses.

For morphological analysis of the gall tissues, protocols with 0.05 toluidine blue or acid fuchsin staining were used. Nematode-infected roots were collected at 7, 14, 21 and 40 DAI, fixed in 2% glutaraldehyde in 50mM PIPES buffer (0.05M pH 6.9) and then dehydrated and soaked in Technovit 7100 (Heraeus Kulzer, Wehrheim, Germany) as described by the manufacturer. The included tissues were sectioned (3µm) and stained in 0.05% toluidine blue, mounted on Depex (Sigma), and the images were captured using a brightfield optical microscope. Infection tests were also performed, followed by acid fuchsin staining. For the infection tests at 40 DAI, the roots were collected and washed to count the number of galls and egg masses under a stereomicroscope. Three independent biological replications of 20 plants were performed for each lineage to be compared with the wild type. Acid fuchsin staining was performed following standard protocols for visualization of the nematode inside the roots. Juvenile second-stage parasites and pear-shaped females were observed for their development. Microscopic observations were performed using a brightfield optical microscope and the images were acquired with a digital camera (Axiocam; Zeiss).

Figure 25 shows the histological analysis of the formation of galls by the nematode *Meloidogyne incognita* in infected WT control and mutant *aip10ko* plants. Brightfield microscope images of sections of galls stained with toluidine blue at 3, 7, 14, and 21 DAI. Asterisks, giant cells; n, nematode, NC, neighboring cells, DAI, days after inoculation. Bars = 100 µm. These data show that plants of *A. thaliana aip10ko* are less susceptible to infection by root-knot nematode *(M. incognita).* The silencing of AIP10 was able to alter the morphology of root-knot cells, whose formation is dependent on cell cycle modulation and DNA replication. Giant cells are made up of a mixture of cells full of cytoplasm and completely empty cells and are located on the periphery of the gall, unlike control plants, which have large, central giant cells full of cytoplasm. Neighboring cells show greater disorganization than control galls.

The results showed that *A. thaliana aip10ko* plants are less susceptible to infection by root-knot nematode *(M. incognita).* The silencing of *AIP10* was able to alter the morphology of gall cells, whose formation is dependent on cell cycle modulation and DNA replication. In *aip10ko* mutants, giant cells consist of a mixture between cells full of cytoplasm and completely empty cells and are located at the periphery of the gall; unlike control plants, which have large, central giant cells full of cytoplasm.

Figure 26 shows the root-knot nematode *(M. incognita)* infection test in WT control plants and in *aip10ko* mutants under normal conditions and under water deficit. (A) Number of galls per plant. (B) Number of females with egg mass per plant. It is possible to observe that aip10ko plants have fewer galls and fewer females with egg mass than control plants, both in normal situation and under water deficit. The data presented represent means ± standard deviation of three independent biological replications, in which a minimum of 30 seedlings of each line (WT and *aip10ko*) were evaluated for nematode infection in the soil. The data were analyzed by one-way ANOVA and the means were compared by the Scott-Knott test at 5% probability. The results show that plants of *A. thaliana aip10ko* produced fewer galls and had fewer females with egg mass, both under normal conditions, that is, with the watered plant, as well as under water deficit. The data corroborate that the total silencing of AIP10 makes plants less susceptible to infection by root-knot nematode *(M. incognita)* and show that this higher resistance in mutants *aip10ko* occurs even in combination with the water stress condition where nematodes become more infective.

It is possible to observe that *aip10ko*plants have fewer galls and fewer females with egg mass than control plants, both in normal situation and under water deficit. Taken together, the data show that the total silencing of *AIP10* makes plants less susceptible to infection by root-knot nematode *(M. incognita)* and show that this greater resistance in aip10ko mutants occurs even in combination with the water stress condition where nematodes become more infective.

### EXAMPLE 13

### ASSAYS OF A. THALIANA PLANTS SILENCED IN THE ABAP1 GENE, INOCULATED WITH BENEFICIAL BACTERIA

The interaction between ABAP1 and AIP10, *abap1kd,* mutant plants member of the ABAP1/AIP10 complex, bioinoculants of beneficial diazotrophic bacteria, were analyzed, evaluating vegetative and reproductive growth and bacteria count.

Figure 27 shows evidence that AIP10 and ABAP1 act in the same regulatory network in plants. Immunoprecipitation of protein extracts from *A. thaliana* control plants and plants expressing AIP10::YFP was performed. (A) Total protein extracts were immunoprecipitated with anti-ABAP1 and analyzed in protein blot gel with antibodies against YFP and ABAP1. The identification of 50 KDa bands in lines 1, 5 and 9 indicates that AIP10 interacts with ABAP1. (B) The expression pattern of the genes of the AIP10/ABAP1 regulatory network was evaluated in *aip10ko* mutant plants. Relative mRNA expression, by qRT-PCR, of cell cycle marker genes and ABAP1 target genes in Columbia and *aip10ko* plants with 30 DAG. It was shown that *aip10ko* plants showed higher expression of the genes *Cdt1a, Cdt1b* and *CYCB1;1,* while there was a reduction in the expression of *ABAP1* compared to Col-0 (WT). The data were normalized using *UBI10* and *GAPDH* as reference genes. The values presented represent the means obtained from independent amplification reactions (n=3) and biological replicates (n=2). The bars indicate the standard deviation. Statistical analysis was performed using Student's t-test (p<0.05). Asterisks (*) indicate significant differences between the samples.

AIP10 and ABAP1 act in the same regulatory network in plants, wherein the proteins interact, and their expression levels are co-regulated during gene silencing. Mutant plants *aip10ko* have reduced levels of ABAP1. In parallel, mutant plants have higher levels of expression of cell cycle regulatory genes, corroborating the phenotype of greater growth.

Seeds of the Col-0 (WT) ecotype and T-DNA insertion mutant in exon 5'UTR of *A. thaliana* of the ABAP1 gene were obtained from the Cold Spring Harbor Enhancer collection (seed code ET13614), respectively. The strain was selected in 50 mg/L of kanamycin in germination medium and the insertion of the T-DNA confirmed by PCR with specific primers for insertion of the T-DNA in the AtABAP1 gene (primers AtABAP1-RP+AtABAP1-LP) and the primer LB1.3. Heterozygous mutant lines for *abap1* (*abap1kd*) were used for phenotypic investigation. The seeds were sterilized and inoculated according to the methodology described for plants *aip10ko.*

Figure 28 shows the effect of the use of bioinoculants containing diazotrophic bacteria on the growth of mutant with reduced expression of *ABAP1* (*abap1kd*)*.* (A) *abap1kd* plants inoculated with diazotrophic bacteria have more developed shoots and root system, compared to Col-0 (WT) plants. Bioinoculants increase (B) the length of the taproot, (C) the number of lateral roots, and (D) the rosette area. Plants 15 DAG, 7 DAI. DAG: days after germination; DAI: days after inoculation. The bars indicate mean ± standard deviation. Asterisk indicates the differences between genotypes and treatments analyzed with statistical test *two-way* ANOVA; *p*<0.05. DAG: days after germination; DAI: days after inoculation. *The percentages in which the quantified value in the mutant is greater than that quantified in wild plants are indicated in the Figure, under the different conditions tested.* Bioinoculant 1: *A. baldaniorum* Sp245; Bioinoculant 2: *H. seropedicae* HRC54.

*abap1kd* plants show increased biomass in response to the use of bioinoculants containing diazotrophic bacteria, compared to Col-0 (WT) plants. *abap1kd* plants showed greater taproot length, being around 23% and 35% larger than Col-0 (WT) when inoculated with the two bioinoculants. In addition, *abap1kd* plants showed 60% more numbers of lateral roots when inoculated with bioinoculant 2.

*A. thaliana abap1* plants are more responsive to association with bioinoculants, promoting root and shoot growth.

Yield analyses were carried out with Col-0 (WT) and *abap1kd* plants, non-inoculated and inoculated, maintained until the end of development under a 16h/8h light/dark photoperiod at 22°C with NPK 10-10-10 nutritional supplement, in which the number of siliques on the main inflorescence axis produced by each plant was counted.

Figure 29 shows the effects of the use of bioinoculants containing diazotrophic bacteria on the reproductive development of *abap1kd* in the soil. (A) *abap1kd* mutant plants have higher productivity under normal conditions compared to control plants, (D) in addition to having a higher number of siliques and inflorescence length. (B, C) Increased growth in *abap1kd* plants is most pronounced when plants are inoculated with bioinoculants containing diazotrophic bacteria. The bars indicate mean ± standard deviation. The data were analyzed by *one-way* ANOVA and the means were compared by the Bonferroni test at 5% probability (p<0.05). *The percentages in which the quantified value in the mutant is greater than that quantified in wild plants are indicated in the Figure, under the different conditions tested.* Bioinoculant 1: *A. baldaniorum* Sp245; Bioinoculant 2: *H. seropedicae* HRC54.

*abap1kd* plants have a higher number of siliques and inflorescence length when compared to wild-type plants. The use of bioinoculants containing diazotrophic bacteria has an additive effect on the development of the reproductive phase of the plant *abap1kd.*

Plants of *A. thaliana abap1kd* are more responsive to the association with bioinoculants, showing greater development of the reproductive part, with increased pod production (siliques)

From the analyses with *abap1kd* plants and the best association with bioinoculants, the colonization rate of the bacteria present in the bioinoculants was analyzed through qRT-PCR.

To verify bacterial colonization in roots of *abap1kd,* the methodology described for plants *aip10ko* was followed.

Figure 30 shows the colonization rate in absolute single-copy values of specific genes for bioinoculants. The colonization rate was measured from the amplification of specific single-copy genes for bioinoculant 1 and bioinoculant 2, in mutant plants for the *ABAP1 (abap1kd)* gene. All plants were grown in earth-vermiculite substrate in photoperiod 12 hours light/12 hours dark and collected 37 days after inoculation and 40 days after germination. *abap1kdplants* have a higher amplification of single-copy genes, specific to the bacteria that make up the bioinoculants used. Bioinoculant 1: *A. baldaniorum* Sp245; Bioioculant 2: *H. seropedicae* HRC54.

*abap1kd* plants showed a significantly higher number of beneficial diazotrophic bacteria associated with their roots when compared to Col-0 (WT) plants. This higher number of diazotrophic bacteria was maintained for both inoculation with bioinoculant 1 and with bioinoculant 2. Showing that *abap1kd* plants interact better with beneficial bacteria and are more benefited by higher rates of biological nitrogen fixation.

*A. thaliana abap1kd* plants are more responsive to association with bioinoculants, showing a higher colonization rate

To verify the mRNA expression analysis of the bacterial *NifH* gene in *abap1kd,* the methodology described for plants *aip10ko* was followed.

Figure 31 shows the mRNA levels of gene *NifH* in *A. thaliana* wild-type and mutant for the gene *ABAP1 (abap1kd).* The graph shows the expression of the mRNA levels of the bacterial *NifH* of *H*. *seropedicae* HRC54, in control plants and mutants *abap1kd* in *A. thaliana.* The results suggest that *abap1kd* plants may have a higher biological nitrogen fixation activity from the native microbiota present in plants. Plants with 15 DAG. Bars indicate mean ± standard deviation. Statistical analysis was performed using Student's t-test (p<0.05). Asterisk (*) indicates a significant difference between the samples. Plants *abap1kd* (15 DAG) showed a higher expression of the bacterial *NifH* gene, compared to the control plant.

The inoculated *abap1kd* plants showed a higher expression of the bacterial *NifH* gene, compared to the Col-0 (WT) plant. As the mRNA expression level of the *NifH* gene is used as a marker of bacterial nitrogenase activity, the results suggest that *abap1kd* plants inoculated with beneficial and nitrogen-fixing bacteria favor better colonization and higher biological nitrogen fixation activity due to the greater responsiveness of this mutant to beneficial bacteria.

*abap1kd* mutants seem to have improved N2 fixation, favoring the development of the vegetative and reproductive part, with increased production of siliques when inoculated with bioinoculants of beneficial diazotrophic bacteria.

To verify the mRNA expression analysis of the genes *AIP10* and *ABAP1* as biomarkers of better association with bioinoculants of beneficial diazotrophic bacteria, the qRT-PCR analysis methodology described for *aip10ko* plants was followed.

Figure 32 shows the mRNA levels of the *AIP10* and *ABAP1* genes in *A. thaliana* Col-0 (WT) plants associated with the bioinoculant. The graph shows the repression of the mRNA levels of the cell cycle repressor genes (A) *ABAP1* and (B) *AIP10* normalized relative to the non-inoculated control, at 3 and 7 days after inoculation with diazotrophic bacteria. The bars indicate mean ± standard deviation. Statistical analysis with *one-way* ANOVA with Bonferroni's Multiple Comparisons. p<0.05%. Bioinoculant: *H. seropedicae* HRC54.

Plants of *A. thaliana* show repression of the *ABAP1* and *AIP10* genes when they are associated with bioinoculants containing diazotrophic bacteria. Because these are cell cycle repressor genes under normal conditions, these results indicate that the cell cycle is more active when the plant is associated with the bioinoculant.

The repression profile of the *AIP10* and *ABAP1* genes in naturally colonized sugarcane genotypes and plants inoculated with bioinoculants containing diazotrophic bacteria were analyzed.

Figure 33 shows the expression of genes homologous to *ABAP1* and *AIP10* in sugarcane plants under different growing conditions, and in inoculation treatments and abiotic stresses. The graph shows the expression in log2 fold change in sugarcane plants, the values represent the repression of the identified genes, in relation to the control or the contrasting genotype. The mRNA analyzed were: naturally colonized root plants (Stalk Root) and shoot part (Stalk Shoot) of contrasting genotypes in relation to biological nitrogen fixation; sugarcane plants grown in hydroponics and inoculated with *G*. *diazotrophicus* (GD) at low nitrogen concentration (-N); and sugarcane plants inoculated with *G. diazotrophicus* (GD) watered (+WD) and subjected to drought (-WD), root (R), and shoot (S). Bioinoculant: *G*. *diazotrophicus* PAL5.

Naturally associated sugarcane plants and plants inoculated with bioinoculants containing diazotrophic bacteria, in different substrates and growing conditions, show repression of the *ABAP1* and *AIP10* genes during association with the bioinoculant. Because these are cell cycle repressor genes under normal conditions, these results indicate that the cell cycle is more active when the plant is inoculated to such bacteria. The data show that this regulatory network works similarly across different plant species.

### EXAMPLE 14

### ASSAYS OF MAIZE WITH BENEFICIAL DIAZOTROPHIC BACTERIA

Analyses of chlorophyll a, analysis of photosynthetic parameters of *aip10-1 mutants,* count of the number of bacteria associated with the root, mRNA expression level of the gene *NifH and 16s, as well as evaluations of phenotypic parameters during the vegetative stage and* root cross-sections.

*aip10-1,* maize seeds knockout in the *AIP10* gene obtained by gene editing (CRISPR) described in example 9, were inoculated for 12 hours in solution with the bioinoculant and subsequently transferred to pots with soil, being cultivated in a greenhouse. The effect of inoculation on wild and mutant *aip10-1* plants was analyzed during the early stages of plant growth, by counting the number of root-associated bacteria, and evaluating root and shoot fresh and dry weight, time of new leaf appearance, and number of roots. A morphological observation and relative fluorescence of cellulose and lignin were performed under a confocal microscope in cross-sections of maize root. The observation and measurement of the circular area of the vascular cylinder (stele) were performed in transverse root sections, stained with Safranin. The fluorescence intensity of cellulose and lignin were quantified at two different wavelengths, separating each specific spectrum.

Figure 34 shows the quantification of colonization by beneficial bacteria in wild plants and mutant plants knockout in the *AIP10* gene in maize, when inoculated with bioinoculants. (A) The rate of root colonization was measured (absolute values) in DNA samples, from the amplification of bacteria-specific single-copy genes from the bioinoculant. (B) Relative quantification of a broad group of diazotrophic bacteria that was performed from RNA amplification *16S* in RNA samples, by qRT-PCR. All plants were grown in soil-vermiculite substrate and collected 10 days after inoculation with Bioinoculant: *A*. *brasilense* Ab-V6. In yellow: wild control plants (WT); in green: mutant plants *aip10-1.*

Maize plants with knockout in the AIP10 gene (mutant *aip10-*1), obtained by gene editing (CRISPR), were more responsive to the association with bioinoculants with plant growth-promoting bacteria, in different biological experiments. The mutant *aip10-1* had a significantly higher number of beneficial diazotrophic bacteria associated with its roots. It was also remarkable to observe that mutant plants, even when not inoculated, already have a higher number of diazotrophic bacteria in their native microbiota.

Figure 35 shows the mRNA levels of the *NifH* gene in wild maize (WT) plants and knockout mutants in the *AIP10* gene. The expression of the *NifH* gene of diazotrophic bacteria was quantified by qRT-PCR in plants collected 10 DAI. The graph shows a higher expression of bacterial *NifH* mRNA levels in uninoculated *aip10-1* mock mutant plants, and an increased expression of bacterial *NifH* in mutant plants inoculated with the bioinoculant, when compared to wild plants (control- (WT). The bars indicate mean ± standard deviation. The data were analyzed by *two-way* ANOVA and the means were compared by Tukey's test at 5% probability (p<0.05). Bioinoculant: *A. brasilense* Ab-V6. In yellow: wild control plants (WT); in green: mutant plants aip10-1. A: biological replica.

The inoculated *aip10-1* plants showed a higher expression of the bacterial *NifH* gene, compared to the Col-0 (WT) plant. As the mRNA expression level of the *NifH* gene is used as a marker of bacterial nitrogenase activity, the results suggest that *aip10-1,* plants inoculated with beneficial and nitrogen-fixing bacteria favor better colonization and greater biological nitrogen fixation activity due to the higher responsiveness of this mutant to beneficial bacteria.

Phenotypic and photosynthetic analyses were performed to evaluate the growth response to inoculation with bioinoculants containing diazotrophic bacteria

Figure 36 shows the effects of the use of bioinoculant with diazotrophic bacteria on the growth of shoots of mutant plants knockout in the *AIP10* gene in maize, obtained by gene editing (CRISPR). The graphs show phenotypic analyses performed with the edited mutant maize plants, of the *aip10-1,* line and wild plants, 20 days after germination (DAG) and 19 days after inoculation (DAI). Mutant plants knocked out in the *AIP10* gene in maize, obtained by gene editing (CRISPR), showed higher (A) shoot fresh weight; (B) shoot dry weight; (C) length of leaf 7, as a reference for development time, which can be observed through representative images of 20 DAG plants and (D) 19 DAI. The bars indicate mean ± standard deviation. The data were analyzed by *two-way* ANOVA and the means were compared by Tukey's test at 5% probability (p<0.05). Bioinoculant: *A. brasilense* Ab-V6. In yellow: wild control plants (WT); in green: mutant plants *aip10-1. The percentages in which the value quantified in the mutant is greater than that quantified in wild plants are indicated in the Figure under the different conditions tested*

The results show that *aip10-1* plants have a better growth response to inoculation with bioinoculants containing diazotrophic bacteria, compared to inoculated wild-type (WT) plants. The best response of the *aip10-1* mutants is evidenced by the percentage of increase in biomass in dry and fresh weight in the shoot (20 and 27%, respectively), compared to wild plants that did not show any improvement in their growth at this vegetative stage. *aip10-1* mutants also showed an increase in growth velocity, represented herein by the larger size of leaf 7 in the mutant, after inoculation with beneficial diazotrophic bacteria, compared to wild-type (WT) plants.

Figure 37 shows the effects of the use of bioinoculant with diazotrophic bacteria on the growth of roots of mutant knockout plants in the AIP10 gene in maize, obtained by gene editing (CRISPR). The graphs show phenotypic analyses performed with the edited mutant maize plants, of the *aip10-1* lineage, and wild plants, 20 days after germination (DAG) and 19 days after inoculation (DAI). Mutant plants knocked out in the *AIP10* gene in maize, obtained by gene editing (CRISPR), showed higher (A) Fresh root weight; (B) Dry root weight; and (C) Number of roots (*crown*) as a reference to root architecture. The bars indicate mean ± standard deviation. The data were analyzed by *two-way* ANOVA and the means were compared by Tukey's test at 5% probability (p<0.05). Bioinoculant: *A. brasilense* Ab-V6. In yellow: wild control plants(WT); in green *aip10-1* mutant plants. *The percentages in which the quantified value in the mutant is greater than that quantified in wild plants are indicated in the Figure, under the different conditions tested.*

The results show that *aip10-1* plants have a better growth response to inoculation with bioinoculants containing diazotrophic bacteria, compared to inoculated wild-type (WT) plants. The best response of the *aip10-1* mutants is evidenced by the percentage of biomass increase in dry and fresh root weight (18% and 25%, respectively), compared to wild plants. The aip10-1 mutants also showed a higher number of roots, after inoculation with beneficial diazotrophic bacteria, compared to wild-type (WT) plants.

Figure 38 shows the effects of the use of bioinoculant with diazotrophic bacteria on the increase of chlorophyll content a and b, and on the photosynthetic capacity in mutant plants knockout in the *AIP10* gene in maize, obtained by gene editing (CRISPR). The graphs show physiological analyses performed with the edited mutant maize plants, of the *aip10-1* lineage, and wild plants, 45 days after germination (DAG) and 44 days after inoculation (DAI). Knockout mutants in AIP10 gene in maize inoculated with bioinoculant presented: (A) higher chlorophyll content *a*; (B) higher chlorophyll content *b*, chlorophyll readings were performed by the ClorofiLOG ^{®} portable chlorophyll meter equipment; (C) higher net CO assimilation rates ₂; (D) higher water efficiency (WUE) (n=8). The measurements were performed with WT_CRISPR and *aip10_CRISPR* plants with 25 DAG with the LICOR-6400 XT instrument at 25°C in CO₂ ambient at 400 µmol and 500µmol of photons m⁻²s⁻¹. The bar graphs represent means ± standard deviation and * significantly different from WT with p<0.05. Student's t-test. *The percentages in which the quantified value in the mutant is higher than that quantified in wild plants are indicated in the Figure, under the different conditions tested.* Total silencing of the *AIP10* gene promoted a more effective association with biofertilizers in mutant knockout plants for the *AIP10* gene in maize, obtained by gene editing (CRISPR), resulting in a higher content of photosynthetic pigments when compared to WT plants-about 5% higher for chlorophyll a and 6.4% for chlorophyll *b*. For photosynthesis analyses, it was observed that through the better association with biofertilizers, the *aip10-1* mutants showed a higher CO2 uptake as well as more efficient use of water, resulting in an even greater gain than what is observed in these mutants without inoculation. By inoculating the mutants, there was a 36% increase in CO2 uptake and 37% in better water use efficiency compared to the wild type (WT), about 10% more than had already been observed when comparing non-inoculated mutant and wild types.

*aip10-1* maize associate more efficiently with biofertilizers, maximizing the benefits of chlorophyll content a and photosynthesis already found in mutants without inoculation.

Figure 39 shows the morphological analysis and relative fluorescence of cellulose and lignin in root cross-sections of mutant plants knockout in the *AIP10* gene in maize. The graphs show phenotypic analyses performed with the edited mutant maize plants, of the *aip10-1* lineage, and wild plants, 10 days after germination (DAG) and 9 days after inoculation (DAI). (A) Measurements of the circular area of the vascular cylinder (stele) in transverse root sections stained with Safranin; (B) Quantification of cellulose fluorescence intensity at 480 nm; (C) Quantification of lignin fluorescence intensity at 430 nm. The bars indicate mean ± standard deviation. The data were analyzed by *two-way* ANOVA and the means were compared by Tukey's test at 5% probability (p<0.05). Bioinoculant: *A. brasilense* Ab-V6. In yellow: wild control plants (WT); in green: mutant plants *AIP10-1.*

The roots of mutant *aip10-1* plants inoculated with beneficial bacteria bioinoculants have larger areas of the vascular cylinder, compared to wild plants, suggesting greater efficiency in the assimilation of nutrients and water. Cell wall components (cellulose and lignin) are also increased in the roots of mutant plants *aip10-1,* compared to wild plants, in both non-inoculated and inoculated plants. The higher lignin content is described as correlated with increased turgor pressure, in addition to being involved in mechanisms that prevent desiccation (cells with more cell wall lose less water) and that increase resistance to pathogens.

Together, all the data observed in the knockout plants in the gene *AIP10* in maize, obtained by gene editing (CRISPR), corroborate the data demonstrated in the mutants *aip10ko* in the model plant *A*. *thaliana,* validating the use of the technology in other plant species used in agriculture.

### EXAMPLE 15

### HYPOTHETICAL MODEL OF MECHANISMS TRIGGERED BY THE ABSENCE OF AIP10

AIP10 can act by modulating plant development through interaction with ABAP1 and kinases, two key regulators of the cell cycle and primary metabolism, respectively. In the absence of AIP10, the transcriptional levels of ABAP1 decrease, preventing the inhibition of its targets, Cdt1a/b, which allows the formation of the pre-RC complex, licensing DNA replication and the progress of cells throughout the cell cycle. At the same time, the absence of AIP10 triggers transcriptional reprogramming, leading to cell division and modulating plant metabolism. These processes lead to an increase in photosynthetic efficiency and result in (i) greater carbon fixation, enhanced by the increase in the number of cells, and (ii) increased accumulation of metabolites. A combination of all these effects contributes not only to the actual increase in root biomass and seed productivity, but also to the improvement of the nutritional value of the plants and to the increase in energy to sustain higher cell division rates as shown in Figure 40.

AIP10 acts as a central hub that integrates the regulation of cell division rates with the primary metabolism of the plant, becoming an efficient strategy to improve carbon sequestration and fixation in biomass, leading to a real increase in productivity and nutritional value of plants.

### EXAMPLE 16

### PROTEIN AND AIP10 GENE SEQUENCES IN DIFFERENT PLANT SPECIES

The sequence of the AIP10 protein and the *AIP10* gene of *A. thaliana* was used to search through biological sequence databases for homologous sequences in other plant species by means of sequence alignment algorithms, such as BLAST Altschul SF, Gish W, Miller W, Myers EW, Lipman DJ. Basic local alignment search tool. J Mol Biol. 1990 Oct 5; 215(3):403-10. Doi: 10.1016/S0022-2836(05)80360-2. PMID: 2231712).

As a result, homologous sequences of amino acids and nucleotides were identified in 31 species, summarized in Table 1. The sequences mentioned in Table 1 are contained in the sequence list.

As the person skilled in the art will well know, the sequences provided can be used to direct the complete suppression of the *AIP10* gene, as well as to identify homologues of *AIP10* in other unlisted species.

### EXAMPLE 17

### AIP10 CONSENSUS SEQUENCE IDENTIFICATION

The amino acid sequences of AIP10 in several species were aligned using the Jalview 2.11.2.6 software with the Muscle with Defaults parameters, with the objective of identifying conserved regions. The result is shown in Figure 41. The consensus sequence is shown below the alignment.

A conserved region was identified between amino acid positions 215-339. This region has been named here as the "AIP10 domain". SEQ ID NO: 263 corresponds to the amino acid sequence of the AIP10 domain contained in the plant species used in this analysis. This sequence can be used as a reference to determine whether a plant protein is an AIP10 protein and, consequently, whether the gene encoding it is an *AIP10* gene according to the present invention using techniques known to the person skilled in the art.

All products disclosed and claimed herein may be made and performed without undue experimentation in light of this disclosure. Although the products of this invention have been described in terms of the preceding illustrative embodiments, it will be evident to those skilled in the art that variations, changes, modifications, and alterations can be applied to the compositions described in this document without departing from the true concept, essence, and scope of the disclosure. More specifically, it will be apparent that certain agents that are chemically and physiologically related may be substituted for the agents described in this document, provided that the same or comparable results would be achieved. All such substitutes and modifications that are apparent to those skilled in the art are considered to fall within the essence, scope, and concept of the invention as defined by the appended claims.

All publications and patent documents disclosed in the specification are hereby incorporated by reference in their entirety, as if each individual publication or patent application were expressly and individually designated as being incorporated by reference.

## Claims

1. Crop plant comprising a genetic modification,
wherein the genetic modification is (i) a deletion of the native *AIP10* gene; or (ii) a modification to the sequence of the native *AIP10* gene or its promoter, where the modified sequence does not produce detectable mRNA or does not express a non-functional protein,
wherein the native *AIP10* gene encodes a protein with at least 90% sequence identity with an amino acid sequence selected from the group consisting of SEQ ID NO: 2, 3, 4, 5, 9, 12, 13, 15, 17, 21, 22, 23, 25, 26, 28, 30, 32, 34, 36, 39, 40, 45, 46, 47, 48, 51, 52, 57, 58, 59, 60, 63, 64, 66, 69, 70, 72, 75, 76, 78, 81, 82, 84, 85, 86, 89, 90, 92, 94, 96, 98, 99, 101, 104, 105, 107 and 263,
in which the plant is homozygous for the genetic modification and presents an improved agronomic characteristic in relation to an identical plant that has partial expression of the native *AIP10* gene.

2. Plant according to claim 1, wherein the native *AIP10* gene has at least 80% sequence identity with a nucleotide sequence selected from the group consisting of SEQ ID NO: 1, 8, 10, 11, 14, 16, 18, 19, 20, 24, 27, 29, 31, 33, 35, 37, 38, 41, 42, 43, 44, 49, 50, 53, 54, 55, 56, 61, 62, 65, 67, 68, 71, 73, 74, 77, 79, 80, 83, 87, 88, 91, 93, 95, 97, 100, 102, 103 and 106.

3. Crop plant comprising a genetic modification,
wherein such modification is (i) a gene expression silencing cassette; or (ii) a modification in the sequence of the native *ABAP1* gene or its promoter, wherein the modified sequence produces reduced or undetectable amounts of mRNA relative to the native *ABAP1* gene, or expresses a less functional protein relative to the native *ABAP1* gene,
wherein the *ABAP1* native gene encodes a protein with at least 90% sequence identity with an amino acid sequence selected from the group consisting of SEQ ID NO: 7, 109, 111, 114, 115, 117, 121, 122, 123, 125, 127, 129, 132, 133, 136, 137, 140, 141, 142, 146, 147, 148, 155, 156, 157, 158, 159, 160, 161, 164, 165, 171, 172, 173, 174, 175, 180, 181, 182, 183, 188, 189, 190, and 191, 254, 198, 199, 200, 201, 202, 206, 207, 208, 210, 213, 214, 217, 218, 219, 220, 223, 224, 227, 228, 231, 232, 235, 236, 239, 240, 241, 242, 243, 246, 247, 248, 251 and 252,
wherein a plant comprising the referred genetic modification has an improved agronomic trait over an identical plant that has expression of the *ABAP1* native gene.

4. Plant according to claim 3, in which the native *ABAP1* gene has at least 80% sequence identity with a nucleotide sequence selected from the group consisting of SEQ ID NO: 6, 108, 110, 112, 113, 116, 118, 119, 120, 124, 126, 128, 130, 131, 134, 135, 138, 139, 143, 144, 145, 149, 150, 151, 253, 152, 153, 154, 162, 163, 166, 167, 168, 169, 170, 176, 177, 178, 179, 184, 185, 186, 187, 192, 193, 194, 195, 196, 197, 203, 204, 205, 209, 211, 212, 215, 216, 221, 222, 225, 226, 229, 230, 233, 234, 237, 238, 244, 245, 249 and 250.

5. Plant according to any of claims 1 to 4, in which genetic modification is a deletion, insertion, or substitution of one or more nucleotides in the promoter region, in the 5'UTR region, in the coding region, or in the 3'UTR region of the native gene.

6. Plant according to any of claims 1 to 5, wherein the crop is selected from the group consisting of a food crop, a forage crop, a fiber crop, an oilseed crop, an ornamental crop, and an industrial crop.

7. Plant according to any of claims 1 to 5, wherein the crop plant is a monocotyledon, preferably selected from the group consisting of oats, rice, sugar cane, rye, barley, maize, miscanthus, sorghum, wheat, banana, and palm trees.

8. Plant according to any of claims 1 to 5, wherein the crop plant is a dicotyledon, preferably selected from the group consisting of cotton, lettuce, potato, sugar beet, cocoa, coffee, canola, citrus, eucalyptus, vegetables, cassava, castor bean, eucalyptus, pea, bean, papaya, castor bean, mustard, poplar, soybean, tomato and grape.

9. Plant according to any of claims 1 to 5, wherein the enhanced agronomic trait is increased biomass.

10. Plant according to any of claims 1 to 5, where the enhanced agronomic trait is water stress tolerance.

11. Plant according to any of claims 1 to 5, wherein the enhanced agronomic trait is photosynthetic efficiency.

12. Plant according to any of claims 1 to 5, wherein the enhanced agronomic trait is carbon fixation.

13. Plant according to any of claims 1 to 5, wherein the enhanced agronomic trait is nutritional value.

14. Plant according to any of claims 1 to 5, in which the enhanced agronomic trait is resistance to pest nematodes.

15. Plant according to any of claims 1 to 8, in which the improved agronomic trait is responsiveness to beneficial bacteria, preferably a nitrogen-fixing, nutrient-providing, phytohormone-producing, abiotic stress-reducing, and/or pathogen- and pest-controlling bacterium.

16. Plant according to any of claims 1 to 5, in which greater responsiveness to beneficial bacteria leads to increased tolerance to water stress and/or reduced use of nitrogen chemical fertilizers.

17. Plant genome of a crop plant comprising a genetic modification,
wherein the genetic modification is (i) a deletion of the native *AIP10* gene; or (ii) a modification in the sequence of the native gene or its promoter, where the modified sequence does not produce detectable mRNA or expresses a non-functional protein,
wherein the native *AIP10* gene encodes a protein with at least 90% sequence identity with an amino acid sequence selected from the group consisting of SEQ ID NO: 2, 3, 4, 5, 9, 12, 13, 15, 17, 21, 22, 23, 25, 26, 28, 30, 32, 34, 36, 39, 40, 45, 46, 47, 48, 51, 52, 57, 58, 59, 60, 63, 64, 66, 69, 70, 72, 75, 76, 78, 81, 82, 84, 85, 86, 89, 90, 92, 94, 96, 98, 99, 101, 104, 105, 107 and 263,
wherein that genome is homozygous for that genetic modification,
in which a plant comprising the genome has an improved agronomic trait compared to an identical plant that has partial expression of the *AIP10* native gene.

18. Plant genome of a crop plant comprising a genetic modification,
in which the referred modification is (i) a gene expression silencing cassette; or (ii) a modification in the sequence of the native *ABAP1* gene or its promoter, in which the modified sequence produces reduced amounts of mRNA relative to the native *ABAP1* gene, or expresses a less functional protein relative to the native *ABAP1* gene,
wherein the *ABAP1* native gene encodes a protein with at least 90% sequence identity with an amino acid sequence selected from the group consisting of SEQ ID NO: 7, 109, 111, 114, 115, 117, 121, 122, 123, 125, 127, 129, 132, 133, 136, 137, 140, 141, 142, 146, 147, 148, 155, 156, 157, 158, 159, 160, 161, 164, 165, 171, 172, 173, 174, 175, 180, 181, 182, 183, 188, 189, 190, and 191, 254, 198, 199, 200, 201, 202, 206, 207, 208, 210, 213, 214, 217, 218, 219, 220, 223, 224, 227, 228, 231, 232, 235, 236, 239, 240, 241, 242, 243, 246, 247, 248, 251 and 252,
wherein a plant comprising the referred genetic modification has an improved agronomic trait over an identical plant that has expression of the *ABAP1* native gene.

19. Method for the production of a plant with improved agronomic trait, comprising the crossing of a genetically modified plant as defined in any of claims 1 to 16 with a second plant.

20. Method in accordance with claim 19, further including the backcrossing or self-fertilization of the progeny for the production of a new generation of homozygous plants for the aforementioned genetic modification.

21. Method for the production of a crop plant with enhanced agronomic trait comprising the introduction of a modification to the genome of a crop plant,
wherein such modification is a (i) deletion of the *AIP10* native gene; or (ii) a modification to the sequence of the *AIP10* native gene or its promoter, where the modified sequence does not produce detectable mRNA or expresses a non-functional protein,
wherein the native *AIP10* gene encodes a protein with at least 90% sequence identity with an amino acid sequence selected from the group consisting of SEQ ID NO: 2, 3, 4, 5, 9, 12, 13, 15, 17, 21, 22, 23, 25, 26, 28, 30, 32, 34, 36, 39, 40, 45, 46, 47, 48, 51, 52, 57, 58, 59, 60, 63, 64, 66, 69, 70, 72, 75, 76, 78, 81, 82, 84, 85, 86, 89, 90, 92, 94, 96, 98, 99, 101, 104, 105, 107 and 263,
in which the method also includes the backcrossing or self-fertilization of the progeny for the production of a new generation of homozygous plants for said genetic modification,
wherein a plant comprising said genetic modification has an improved agronomic trait over an identical plant that has partial expression of the *AIP10* native gene.

22. Method for the production of a crop plant with an enhanced agronomic trait selected from the group consisting of photosynthetic efficiency, carbon fixation, resistance to pest nematodes, and responsiveness to beneficial bacteria, wherein the method comprises the introduction of a genetic modification into the genome of a crop plant,
in which said modification is (i) an expression silencing cassette; (ii) a deletion of the native *AIP10* gene; or (iii) a modification in the sequence of the native *AIP10* gene or its promoter, in which the modified sequence produces reduced or undetectable amounts of mRNA relative to the native *AIP10* gene or expresses a less functional protein relative to the native *AIP10* gene or a non-functional one,
wherein the native *AIP10* gene encodes a protein with at least 90% sequence identity with an amino acid sequence selected from the group consisting of SEQ ID NO: 2, 3, 4, 5, 9, 12, 13, 15, 17, 21, 22, 23, 25, 26, 28, 30, 32, 34, 36, 39, 40, 45, 46, 47, 48, 51, 52, 57, 58, 59, 60, 63, 64, 66, 69, 70, 72, 75, 76, 78, 81, 82, 84, 85, 86, 89, 90, 92, 94, 96, 98, 99, 101, 104, 105, 107 and 263,
in which a plant comprising the referred genetic modification presents the referred agronomic trait enhanced in relation to an identical plant that has expression of the *AIP10* native gene.

23. Method according to claim 21 or 22, where the native *AIP10* gene has at least 80% sequence identity with a nucleotide sequence selected from the group consisting of SEQ ID NO: 1, 8, 10, 11, 14, 16, 18, 19, 20, 24, 27, 29, 31, 33, 35, 37, 38, 41, 42, 43, 44, 49, 50, 53, 54, 55, 56, 61, 62, 65, 67, 68, 71, 73, 74, 77, 79, 80, 83, 87, 88, 91, 93, 95, 97, 100, 102, 103 and 106.

24. Method for the production of a crop plant with enhanced agronomic trait comprising the introduction of a genetic modification into the genome of a crop plant,
in which the referred modification is (i) a gene expression silencing cassette; or (ii) a modification in the sequence of the native *ABAP1* gene or its promoter, in which the modified sequence produces reduced or undetectable amounts of mRNA relative to the native *ABAP1* gene or expresses a less functional protein relative to the native ABAP1 gene.
wherein the native ABAP1 gene encodes a protein with at least 90% sequence identity with an amino acid sequence selected from the group consisting of SEQ ID NO: 7, 109, 111, 114, 115, 117, 121, 122, 123, 125, 127, 129, 132, 133, 136, 137, 140, 141, 142, 146, 147, 148, 155, 156, 157, 158, 159, 160, 161, 164, 165, 171, 172, 173, 174, 175, 180, 181, 182, 183, 188, 189, 190, E 191, 254, 198, 199, 200, 201, 202, 206,
wherein a plant comprising the referred genetic modification has an improved agronomic trait over an identical plant that has expression of the *ABAP1* native gene.

25. Method according to claim 24, in which the native *ABAP1* gene has at least 80% sequence identity with a nucleotide sequence selected from the group consisting of SEQ ID NO: 6, 108, 110, 112, 113, 116, 118, 119, 120, 124, 126, 128, 130, 131, 134, 135, 138, 139, 143, 144, 145, 149, 150, 151, 253, 152, 153, 154, 162, 163, 166, 167, 168, 169, 170, 176, 177, 178, 179, 184, 185, 186, 187, 192, 193, 194, 195, 196, 197, 203, 204, 205, 209, 211, 212, 215, 216, 221, 222, 225, 226, 229, 230, 233, 234, 237, 238, 244, 245, 249 and 250.

26. Method according to any of claims 21 to 25, in which genetic modification is a deletion, insertion, or substitution of one or more nucleotides in the promoter region, in the 5'UTR region, in the coding region, or in the 3'UTR region of the native gene.

27. Method according to claim 22 or 24, **characterized by** the fact that the expression cassette encodes an anti-sense RNA, RNAi, artificial microRNA.

28. Method in accordance with any of claims 21 to 27, further comprising the backcrossing or self-fertilization of the progeny for the production of a new generation of homozygous plants for the aforementioned genetic modification.

29. Method according to any of claims 21 to 27, in which the crop is selected from the group consisting of a food crop, a forage crop, a fiber crop, an oilseed crop, an ornamental crop, and an industrial crop.

30. Method according to any of claims 21 to 27, in which the crop plant is a monocotyledon, preferably selected from the group consisting of oats, rice, sugar cane, rye, barley, maize, miscanthus, sorghum, wheat, banana and palm trees.

31. Method according to any of claims 21 to 27, in which the crop plant is a dicotyledon, preferably selected from the group consisting of cotton, lettuce, potato, beetroot, cocoa, coffee, canola, citrus, eucalyptus, vegetables, cassava, castor bean, eucalyptus, peas, beans, papaya, castor bean, mustard, poplar, soybean, tomato and grape.

32. Method according to claim 21 or 24, in which the improved agronomic trait is biomass.

33. Method according to claim 21 or 24, in which the improved agronomic trait is tolerance to water stress.

34. Method according to claim 21, 22 or 24, in which the improved agronomic trait is photosynthetic efficiency.

35. Method according to claim 21, 22 or 24, in which the enhanced agronomic trait is carbon fixation.

36. Method according to claim 21, 22 or 24, in which the improved agronomic characteristic is the improvement in nutritional value.

37. Method according to claim 21, 22 or 24, in which the improved agronomic trait is resistance to pest nematodes.

38. Method according to claim 21, 22 or 24, in which the improved agronomic characteristic is responsiveness to beneficial bacteria, preferably a nitrogen-fixing, nutrient-providing, phytohormone-producing, abiotic stress-reducing, and/or pathogen and pest-controlling bacterium.

39. Method according to claim 21, 22, or 24, in which genetic modification is introduced into the plant genome by random or targeted mutagenesis.

40. Method according to claim 39, in which mutagenesis is selected from the group consisting of chemical mutagenesis, radiation mutagenesis, transposon mutagenesis, and T-DNA insertion by transformation with a bacterium capable of transferring DNA to plants.

41. Method according to claim 21, 22 or 24, in which the mutation is introduced into the plant's genome by genome editing.

42. Method according to claim 41, in which genome editing is performed using a technique selected from the group consisting of transcription-activating effector nucleases (TALENs), zinc finger nucleases (ZFNs), RNA-guided Fokl nucleases, homing endonucleases, CRISPR-Cas9, and CRISPR-Cas12a(Cpf1).

43. Nucleic acid construct comprising a polynucleotide sequence from a fragment of *AIP10* or *ABAP1* in antisense orientation operationally linked to a heterologous promoter,
wherein the native *AIP10* gene encodes a protein with at least 90% sequence identity with an amino acid sequence selected from the group consisting of SEQ ID NO: 2, 3, 4, 5, 9, 12, 13, 15, 17, 21, 22, 23, 25, 26, 28, 30, 32, 34, 36, 39, 40, 45, 46, 47, 48, 51, 52, 57, 58, 59, 60, 63, 64, 66, 69, 70, 72, 75, 76, 78, 81, 82, 84, 85, 86, 89, 90, 92, 94, 96, 98, 99, 101, 104, 105, 107 and 263, and
wherein the native *ABAP1* gene encodes a protein with at least 90% sequence identity with an amino acid sequence selected from the group consisting of SEQ ID NO: 7, 109, 111, 114, 115, 117, 121, 122, 123, 125, 127, 129, 132, 133, 136, 137, 140, 141, 142, 146, 147, 148, 155, 156, 157, 158, 159, 160, 161, 164, 165, 171, 172, 173, 174, 175, 180, 181, 182, 183, 188, 189, 190, E 191, 254, 198, 199, 200, 201, 202, 206.

44. Nucleic acid construction according to claim 43, wherein the construction further comprises a polynucleotide sequence of a fragment of *AIP10* or *ABAP1* in sense orientation separated by a spacer sequence of said sequence in antisense orientation.

45. Plant produced by a method as defined in any of claims 21 through 42 or comprising a nucleic acid construct as defined in claim 43 or 44.

46. Use of a plant as defined in any of claims 1 through 16 and 45 or produced by the method as defined in any of claims 21 through 42 for the crossing with a second plant, regeneration of a transgenic plant, planting or growth of a field of genetically modified plants, production of plant products, or production of a plant product.

47. Cultivation method of a crop with an enhanced agronomic trait comprising cultivating one or more plants as defined in any of claims 1 to 16 and 44 or produced by the method as defined in any of claims 21 to 41.

48. Method according to claim 47, in which the improved agronomic trait is biomass.

49. Method according to claim 47, in which the improved agronomic trait is tolerance to water stress.

50. Method according to claim 47, in which the improved agronomic trait is photosynthetic efficiency.

51. Method according to claim 47, in which the improved agronomic trait is carbon fixation.

52. Method according to claim 47, in which the improved agronomic characteristic is nutritional value.

53. Method according to claim 47, in which the improved agronomic trait is resistance to pest nematodes.

54. Method according to claim 47, in which the improved agronomic characteristic is responsiveness to beneficial bacteria, preferably a nitrogen-fixing, nutrient-supplying, phytohormone-producing, abiotic stress-reducing, and/or pathogen and pest-controlling bacterium.

55. Plant product, **characterized by** the fact that it is obtainable from a plant as defined in any of claims 1 to 16 and 44 or produced by the method as defined in any of claims 21 to 42.
